# EUROPEAN PATENT APPLICATION

(11) **EP 3 492 097 A1**
(43) Date of publication of application: **05.06.2019**
(21) Application number: 18214695.1
(22) Date of filing: 04.08.2014
(51) Int. Cl.: A61K 39/02, A61K 39/155, A61K 39/00, A61K 39/10

(54) **COMBINATION IMMUNOGENIC COMPOSITIONS**

(30) Priority: 05.08.2013 GB 201313990; 04.02.2014 GB 201401883
(62) Divisional of application: 14750176.1
(71) Applicant: GlaxoSmithKline Biologicals S.A., 1330 Rixensart (BE)
(72) Inventor: STEFF, Ann-Muriel, Laval, Québec H7V 3S8 (CA); TEMMERMAN, Stéphane T., 1330 Rixensart (BE); TOUSSAINT, Jean-François, 1330 Rixensart (BE)
(74) Representative: Thornley, Rachel Mary

(57) **Abstract**

Combination immunogenic compositions capable of eliciting protection against both RSV and *B. pertussis* infection and disease are disclosed. More specifically, this disclosure concerns such compositions which comprise a recombinant F protein analog of RSV, together with *B. pertussis* acellular (Pa) or whole cell (Pw) antigens and the use of the same, particularly in the context of neonatal and maternal immunization. Also disclosed are vaccine regimens, methods, as well as uses, and kits, of immunogenic compositions for protecting infants against disease caused by RSV and *B. pertussis* by maternal immunization.

## Description

### BACKGROUND

This disclosure concerns the field of immunology. More particularly this disclosure relates to compositions and methods for eliciting immune responses specific for Respiratory Syncytial Virus (RSV) and *Bordetella pertussis.*

Human Respiratory Syncytial Virus (RSV) is the most common worldwide cause of lower respiratory tract infections (LRTI) in infants less than 6 months of age and premature babies less than or equal to 35 weeks of gestation. The RSV disease spectrum includes a wide array of respiratory symptoms from rhinitis and otitis to pneumonia and bronchiolitis, the latter two diseases being associated with considerable morbidity and mortality. Humans are the only known reservoir for RSV. Spread of the virus from contaminated nasal secretions occurs via large respiratory droplets, so close contact with an infected individual or contaminated surface is required for transmission. RSV can persist for several hours on toys or other objects, which explains the high rate of nosocomial RSV infections, particularly in paediatric wards.

The global annual infection and mortality figures for RSV are estimated to be 64 million and 160,000 respectively. In the USA alone RSV is estimated to be responsible for 18,000 to 75,000 hospitalizations and 90 to 1900 deaths annually. In temperate climates, RSV is well documented as a cause of yearly winter epidemics of acute LRTI, including bronchiolitis and pneumonia. In the USA, nearly all children have been infected with RSV by two years of age. The incidence rate of RSV-associated LRTI in otherwise healthy children was calculated as 37 per 1000 child-year in the first two years of life (45 per 1000 child-year in infants less than 6 months old) and the risk of hospitalization as 6 per 1000 child-years. Incidence is higher in children with cardio-pulmonary disease and in those born prematurely, who constitute almost half of RSV-related hospital admissions in the USA. Children who experience a more severe LRTI caused by RSV later have an increased incidence of childhood asthma. These studies demonstrate widespread need for RSV vaccines, as well as use thereof, in industrialized countries, where the costs of caring for patients with severe LRTI and their sequelae are substantial. RSV also is increasingly recognized as an important cause of morbidity from influenza-like illness in the elderly.

The bacterium *Bordetella pertussis* is the causative agent for whooping cough, a respiratory disease that can be severe in infants and young children. WHO estimates suggest that, in 2008, about 16 million cases of whooping cough occurred worldwide, and that 195,000 children died from the disease. Vaccines have been available for decades, and global vaccination is estimated (WHO) to have averted about 687,000 deaths in 2008.

The clinical course of the disease is characterised by paroxysms of rapid coughs followed by inspiratory effort, often associated with a characteristic 'whooping' sound. In serious cases, oxygen deprivation can lead to brain damage; however the most common complication is secondary pneumonia. Although treatment with antibiotics is available, by the time the disease is diagnosed, bacterial toxins have often caused severe damage. Prevention of the disease is therefore of great importance, hence developments in vaccination are of significant interest. The first generation of vaccines against *B. pertussis* were whole cell vaccines, composed of whole bacteria that have been killed by heat treatment, formalin or other means. These were introduced in many countries in the 1950s and 1960s and were successful at reducing the incidence of whooping cough.

A problem with whole cell *B. pertussis* vaccines is the high level of reactogenicity associated with them. This issue was addressed by the development of acellular pertussis vaccines containing highly purified *B. pertussis* proteins - usually at least pertussis toxoid (PT; pertussis toxin chemically treated or genetically modified to eliminate its toxicity) and filamentous haemagglutinin (FHA), often together with the 69kD protein pertactin (PRN), and in some cases further including fimbriae types 2 and 3 (FIMs 2 and 3). These acellular vaccines are generally far less reactogenic than whole cell vaccines, and have been adopted for the vaccination programmes of many countries. However, an overall increasing trend in reported pertussis cases in the US since the early 1980s (with more cases being reported in 2012 than in any year since 1955), and highly publicized outbreaks in many countries in recent years, has led to speculation that the protection elicited by the acellular vaccines is less durable than that conferred by the whole cell vaccines. Such waning of immunity after childhood immunizations means adolescents and adults can act as reservoirs of this highly contagious disease. This puts neonates at particular risk in the first few months of life, before the onset of paediatric vaccination at 2-3 months.

Strategies for protecting vulnerable newborns include 'cocooning', i.e. vaccinating adolescents and adults (including postpartum women) likely to be in contact with newborns, whilst vaccination of pregnant women (maternal immunization) is now recommended in several countries, whereby anti-pertussis antibodies are transferred placentally to provide protection until the infant can be directly vaccinated. At-birth vaccination of neonates has also been evaluated in clinical trials.

Although *B. pertussis* vaccination is well established, despite various attempts to produce a safe and effective RSV vaccine that elicits durable and protective immune responses in healthy and at-risk populations, none of the candidates evaluated to date have been proven safe and effective as a vaccine for the purpose of preventing RSV infection and/or reducing or preventing RSV disease. Accordingly, there is an unmet need for a combination vaccine which confers protection against both RSV and *B. pertussis* infection and associated disease, which pose particular dangers to neonates and young infants.

### BRIEF SUMMARY

This disclosure concerns combination immunogenic compositions, such as vaccines, capable of eliciting protection against both RSV and *B. pertussis* (sometimes referred to as "pertussis" herein) infection and/or disease. More specifically, this disclosure concerns such compositions which comprise a recombinant F protein analog of RSV, together with *B*. *pertussis* acellular (Pa) or whole cell (Pw) antigens and the use of the same, particularly in the context of neonatal and maternal immunization. Also disclosed are vaccine regimens, methods and uses of immunogenic compositions for protecting infants against disease caused by RSV and *B. pertussis* by administering to a pregnant female a recombinant RSV F protein analog and a *B. pertussis* antigen, in at least one immunogenic composition, whereby protection of neonates from birth is effected by trans-placental transfer of maternal antibodies protective against RSV and whooping cough. The at least one immunogenic composition may be a RSV-*B*. *pertussis* combination composition as disclosed herein. Kits useful for such maternal immunization are also disclosed.

### BRIEF DESCRIPTION OF THE DRAWINGS

FIG. 1A is a schematic illustration highlighting structural features of the RSV F protein. FIG. 1B is a schematic illustration of exemplary RSV Prefusion F (PreF) antigens.
FIG. 2 shows the study design for a guinea pig experiment performed in Example 1.
FIG. 3 shows results from Example 1 following challenge of guinea pig progeny with RSV.
FIG. 4 shows the time-course of the neutralizing antibody response in the guinea pig model in Example 1.
FIGS. 5A and 5B are graphs illustrating the neutralizing titres and protection against RSV challenge infection following immunization with an RSV + pertussis combination vaccine (Example 2).
FIGS. 6A and 6B are graphs illustrating serum antibody titers and protection against challenge with *Bordetella pertussis* following immunization with an RSV + pertussis combination vaccine (Example 3).
FIGS. 7A and 7B are graphs illustrating the neutralizing titres in guinea pig dams and pups following maternal immunization of RSV-primed dams with RSV+pertussis combination vaccine (Example 4).
FIG.8 shows protection against RSV challenge infection of guinea pig pups following maternal immunization of RSV-primed dams with an RSV + pertussis combination vaccine (Example 4).

### DETAILED DESCRIPTION

### INTRODUCTION

Development of vaccines to prevent RSV infection has been complicated by the fact that host immune responses appear to play a role in the pathogenesis of the disease. Early studies in the 1960s showed that children vaccinated with a formalin-inactivated RSV vaccine suffered from more severe disease on subsequent exposure to the virus as compared to unvaccinated control subjects. These early trials resulted in the hospitalization of 80% of vaccinees and two deaths. The enhanced severity of disease has been reproduced in animal models and is thought to result from inadequate levels of serum-neutralizing antibodies, lack of local immunity, and excessive induction of a type 2 helper T-cell-like (Th2) immune response with pulmonary eosinophilia and increased production of IL-4 and IL-5 cytokines. In contrast, a successful vaccine that protects against RSV infection induces a Th1 biased immune response, characterized by production of IL-2 and γ-interferon (IFN).

Although immunization against whooping cough is well established, in the erstwhile absence of an acceptable vaccine against RSV there has been no opportunity to investigate a combination vaccine capable of conferring protection against RSV and *B. pertussis* infection and/or disease. As these infectious agents pose the most significant risk to neonates and young infants (who in the case *of B. pertussis* have yet to build the full protection stimulated by the paediatric vaccination schedule), and also both present a danger to the elderly, a combined immunogenic composition allowing delivery of both vaccines in a single injection would be advantageous in terms of comfort for the recipient, compliance with the vaccine schedule, cost-effectiveness, as well as freeing-up space in the immunization schedule for other vaccines.

The present disclosure describes combination immunogenic compositions (e.g. vaccines) that protect against infection, or disease associated, with both RSV and *B. pertussis,* and methods for using the same, especially in the protection of neonates and young infants in which populations are found the highest incidence and severity, with respect to morbidity and mortality, associated with these pathogens. The protection of such a demographic presents challenges. Young infants, and especially those born prematurely, can have an immature immune system. There is also the potential for interference of maternal antibodies with vaccination in very young infants. In the past there has been a problem with enhancement of RSV disease with vaccination of young infants against RSV, as well as challenges arising from waning of immunity elicited by natural infection and immunization. Maternal immunization with *B. pertussis* antigen-containing vaccines has been shown to increase anti-*B*. *pertussis* antibody titres in newborns (relative to newborns from non-maternally-immunised mothers) (for example, Gall et al (2011), Am J Obstet Gynecol, 204:334.e1-5, incorporated herein by reference), and such maternal immunization is now recommended in some countries. In addition to disclosing immunization methods employing the disclosed combination compositions, the present disclosure additionally describes vaccine regimens, methods and uses of immunogenic compositions for protecting neonates and young infants by immunizing pregnant females with combinations of RSV and *B. pertussis* antigens, including by use of the disclosed RSV-B. *pertussis* combination immunogenic compositions. The antigens favourably elicit antibodies which are transferred to the gestational infant via the placenta resulting in passive immunological protection of the infant following birth and lasting through the critical period for infection and severe disease caused by RSV and *B. pertussis.*

One aspect of this disclosure relates to a combination immunogenic composition comprising at least one RSV antigen and at least one *B. pertussis* antigen, wherein the at least one *B. pertussis* antigen comprises at least one acellular pertussis (Pa) antigen or comprises a whole cell (Pw) antigen.

In particular, the disclosure provides such a combination immunogenic composition wherein the at least one RSV antigen is a recombinant soluble F protein analog. F analogs stabilized in the postfusion conformation ("PostF"), or that are labile with respect to conformation, can be employed. Advantageously, the F protein analog is a prefusion F or "PreF" antigen that includes at least one modification that stabilizes the prefusion conformation of the F protein.

In a particular embodiment, the at least one *B. pertussis* antigen of the combination immunogenic composition comprises at least one Pa antigen selected from the group consisting of: pertussis toxoid (PT), filamentous haemagglutinin (FHA), pertactin (PRN), fimbrae type 2 (FIM2), fimbrae type 3 (FIM3). Such Pa vaccines are well known in the art.

In an alternative embodiment, the at least one *B. pertussis* antigen comprises a Pw antigen, Pw vaccines being well known in the art. As used herein, the term "a whole cell (Pw) antigen" means an inactivated *B. pertussis* cell (which of course strictly contains numerous different antigens), and therefore equates to a Pw vaccine.

In certain embodiments, the disclosed combination immunogenic composition comprises a pharmaceutically acceptable carrier or excipient, such as a buffer. Additionally or alternatively, the immunogenic composition may comprise an adjuvant, for example, an adjuvant that includes 3D-MPL, QS21 (e.g., in a detoxified form), an oil-in-water emulsion (e.g., with or without immunostimulatory molecules, such as α-tocopherol), mineral salts such as aluminium salts (including alum, aluminium phosphate, aluminium hydroxide) and calcium phosphate, or combinations thereof.

In certain embodiments, the disclosed combination immunogenic compositions additionally comprise at least one antigen from at least one pathogenic organism other than RSV and *B. pertussis.* In particular, said at least one pathogenic organism may be selected from the group consisting of: *Corynebacterium diphtheriae; Clostridium tetani;* Hepatitis B virus; Polio virus; *Haemophilus influenzae* type b; *N. meningitidis* type C; *N. meningitidis* type Y; *N. meningitidis* type A, *N. meningitidis* type W; and *N. meningitidis* type B.

In another aspect, this disclosure relates to the use of the disclosed combination immunogenic compositions for the prevention and/or treatment of RSV and *B. pertussis* infection/disease. Thus, disclosed herein is a method for eliciting an immune response against RSV and *B. pertussis,* comprising administering to a subject an immunologically effective amount of said combination immunogenic composition.

In a further aspect, the present disclosure concerns the disclosed combination immunogenic compositions for use in medicine, in particular for the prevention or treatment in a subject of infection by, or disease associated with, RSV and *B. pertussis.*

In another aspect, this disclosure concerns a vaccination regimen for protecting an infant against infection or disease caused by RSV and *B. pertussis,* the vaccination regimen comprising:
administering to a pregnant female with a gestational infant at least one immunogenic composition capable of boosting a humoral immune response specific for both RSV and *B. pertussis,* which at least one immunogenic composition comprises a recombinant RSV antigen comprising an F protein analog and at least one *B. pertussis* antigen,
wherein at least one subset of RSV-specific antibodies and at least one subset of *B. pertussis-specific* antibodies elicited or increased in the pregnant female by the at least one immunogenic composition are transferred via the placenta to the gestational infant, thereby protecting the infant against infection or disease caused by RSV and *B. pertussis.*

Also disclosed in an aspect is a method for protecting an infant against infection or disease caused by RSV and *B. pertussis,* the method comprising:
administering to a pregnant female with a gestational infant at least one immunogenic composition capable of boosting a humoral immune response specific for both RSV and *B. pertussis,* which at least one immunogenic composition comprises a recombinant RSV antigen comprising an F protein analog and at least one *B. pertussis* antigen,
wherein at least one subset of RSV-specific antibodies and at least one subset of *B. pertussis*-specific antibodies elicited or increased in the pregnant female by the at least one immunogenic composition are transferred via the placenta to the gestational infant, thereby protecting the infant against infection or disease caused by RSV and *B. pertussis.*

Another aspect of the present disclosure concerns an immunogenic composition or plurality of immunogenic compositions comprising a recombinant RSV antigen comprising an F protein analog and at least one *B. pertussis* antigen for use in protecting an infant against infection or disease caused by RSV and *B. pertussis,* wherein the immunogenic composition(s) is/are formulated for administration to a pregnant female and wherein the immunogenic composition(s) is/are capable of boosting a humoral immune response specific for both RSV and *B. pertussis,* and wherein at least one subset of RSV-specific antibodies and at least one subset of *B. pertussis-specific* antibodies boosted in the pregnant female by the immunogenic composition(s) are transferred via the placenta to the gestational infant, thereby protecting the infant against infection or disease caused by RSV and *B. pertussis.*

In another aspect is disclosed a kit comprising a plurality of immunogenic compositions formulated for administration to a pregnant female, wherein the kit comprises:
(a) a first immunogenic composition comprising an F protein analog capable of inducing, eliciting or boosting a humoral immune response specific for RSV; and
(b) a second immunogenic composition comprising at least one *B. pertussis* antigen capable of inducing, eliciting or boosting a humoral response specific for *B. pertussis,*
wherein upon administration to a pregnant female, the first and second immunogenic compositions induce, elicit or boost at least one subset of RSV-specific antibodies and at least one subset of *B. pertussis-specific* antibodies, which antibodies are transferred via the placenta to a gestating infant of the pregnant female, thereby protecting the infant against infection or disease caused by RSV and *B. pertussis.*

### TERMS

In order to facilitate review of the various embodiments of this disclosure, the following explanations of terms are provided. Additional terms and explanations can be provided in the context of this disclosure.

Unless otherwise explained, all technical and scientific terms used herein have the same meaning as commonly understood by one of ordinary skill in the art to which this disclosure belongs. Definitions of common terms in molecular biology can be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

The singular terms "a," "an," and "the" include plural referents unless context clearly indicates otherwise. Similarly, the word "or" is intended to include "and" unless the context clearly indicates otherwise. The term "plurality" refers to two or more. It is further to be understood that all base sizes or amino acid sizes, and all molecular weight or molecular mass values, given for nucleic acids or polypeptides are approximate, and are provided for description. Additionally, numerical limitations given with respect to concentrations or levels of a substance, such as an antigen, are intended to be approximate. Thus, where a concentration is indicated to be at least (for example) 200 pg, it is intended that the concentration be understood to be at least approximately (or "about" or "∼") 200 pg.

Although methods and materials similar or equivalent to those described herein can be used in the practice or testing of this disclosure, suitable methods and materials are described below. The term "comprises" means "includes." Thus, unless the context requires otherwise, the word "comprises," and variations such as "comprise" and "comprising" will be understood to imply the inclusion of a stated compound or composition (e.g., nucleic acid, polypeptide, antigen) or step, or group of compounds or steps, but not to the exclusion of any other compounds, composition, steps, or groups thereof. The abbreviation, "e.g." is derived from the Latin exempli gratia, and is used herein to indicate a non-limiting example. Thus, the abbreviation "e.g." is synonymous with the term "for example."

The term "F protein" or "Fusion protein" or "F protein polypeptide" or "Fusion protein polypeptide" refers to a polypeptide or protein having all or part of an amino acid sequence of an RSV Fusion protein polypeptide. Numerous RSV Fusion proteins have been described and are known to those of skill in the art. WO2008/114149 sets out exemplary F protein variants (for example, naturally occurring variants).

An "F protein analog" refers to an F protein which includes a modification that alters the structure or function of the F protein but which retains the immunological properties of the F protein such that an immune response generated against an F protein analog will recognize the native F protein. WO2010/149745, incorporated herein in its entirety by reference, sets out exemplary F protein analogs. WO2011/008974, incorporated herein in its entirety by reference, also sets out exemplary F protein analogs. F protein analogs include for example PreF antigens which include at least one modification that stabilizes the prefusion conformation of the F protein and which are generally soluble, i.e., not membrane bound. F protein analogs also include post-fusion F (postF) antigens which are in the post-fusion conformation of the RSV F protein, favorably stabilized in such conformation. F analogs further include F protein in an intermediate conformation, favorably stabilized in such conformation. Such alternatives are also generally soluble.

A "variant" when referring to a nucleic acid or a polypeptide (e.g., an RSV F or G protein nucleic acid or polypeptide, or an F analog nucleic acid or polypeptide) is a nucleic acid or a polypeptide that differs from a reference nucleic acid or polypeptide. Usually, the difference(s) between the variant and the reference nucleic acid or polypeptide constitute a proportionally small number of differences as compared to the referent.

A "domain" of a polypeptide or protein is a structurally defined element within the polypeptide or protein. For example, a "trimerization domain" is an amino acid sequence within a polypeptide that promotes assembly of the polypeptide into trimers. For example, a trimerization domain can promote assembly into trimers via associations with other trimerization domains (of additional polypeptides with the same or a different amino acid sequence). The term is also used to refer to a polynucleotide that encodes such a peptide or polypeptide.

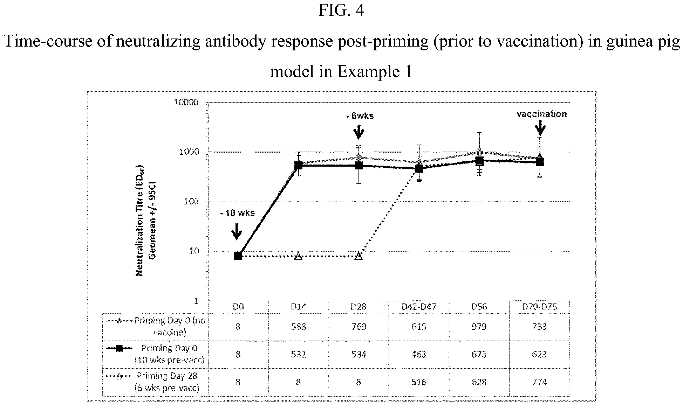

The terms "native" and "naturally occurring" refer to an element, such as a protein, polypeptide or nucleic acid that is present in the same state as it is in nature. That is, the element has not been modified artificially. It will be understood, that in the context of this disclosure, there are numerous native/naturally occurring variants of RSV proteins or polypeptides, e.g., obtained from different naturally occurring strains or isolates of RSV. WO2008114149, incorporated herein by reference in its entirety, contains exemplary RSV strains, proteins and polypeptides, see for example Figure 4.

The term "polypeptide" refers to a polymer in which the monomers are amino acid residues which are joined together through amide bonds. The terms "polypeptide" or "protein" as used herein are intended to encompass any amino acid sequence and include modified sequences such as glycoproteins. The term "polypeptide" is specifically intended to cover naturally occurring proteins, as well as those which are recombinantly or synthetically produced. The term "fragment," in reference to a polypeptide, refers to a portion (that is, a subsequence) of a polypeptide. The term "immunogenic fragment" refers to all fragments of a polypeptide that retain at least one predominant immunogenic epitope of the full-length reference protein or polypeptide. Orientation within a polypeptide is generally recited in an N-terminal to C-terminal direction, defined by the orientation of the amino and carboxy moieties of individual amino acids. Polypeptides are translated from the N or amino-terminus towards the C or carboxy-terminus.

A "signal peptide" is a short amino acid sequence (e.g., approximately 18-25 amino acids in length) that directs newly synthesized secretory or membrane proteins to and through membranes, e.g., of the endoplasmic reticulum. Signal peptides are frequently but not universally located at the N-terminus of a polypeptide, and are frequently cleaved off by signal peptidases after the protein has crossed the membrane. Signal sequences typically contain three common structural features: an N-terminal polar basic region (n-region), a hydrophobic core, and a hydrophilic c-region).

The terms "polynucleotide" and "nucleic acid sequence" refer to a polymeric form of nucleotides at least 10 bases in length. Nucleotides can be ribonucleotides, deoxyribonucleotides, or modified forms of either nucleotide. The term includes single and double-stranded forms of DNA. By "isolated polynucleotide" is meant a polynucleotide that is not immediately contiguous with both of the coding sequences with which it is immediately contiguous (one on the 5' end and one on the 3' end) in the naturally occurring genome of the organism from which it is derived. In one embodiment, a polynucleotide encodes a polypeptide. The 5' and 3' direction of a nucleic acid is defined by reference to the connectivity of individual nucleotide units, and designated in accordance with the carbon positions of the deoxyribose (or ribose) sugar ring. The informational (coding) content of a polynucleotide sequence is read in a 5' to 3' direction.

A "recombinant" nucleic acid is one that has a sequence that is not naturally occurring or has a sequence that is made by an artificial combination of two otherwise separated segments of sequence. This artificial combination can be accomplished by chemical synthesis or, more commonly, by the artificial manipulation of isolated segments of nucleic acids, e.g., by genetic engineering techniques. A "recombinant" protein is one that is encoded by a heterologous (e.g., recombinant) nucleic acid, which has been introduced into a host cell, such as a bacterial or eukaryotic cell. The nucleic acid can be introduced, on an expression vector having signals capable of expressing the protein encoded by the introduced nucleic acid or the nucleic acid can be integrated into the host cell chromosome.

The term "heterologous" with respect to a nucleic acid, a polypeptide or another cellular component, indicates that the component occurs where it is not normally found in nature and/or that it originates from a different source or species.

The term "purification" (e.g., with respect to a pathogen or a composition containing a pathogen) refers to the process of removing components from a composition, the presence of which is not desired. Purification is a relative term, and does not require that all traces of the undesirable component be removed from the composition. In the context of vaccine production, purification includes such processes as centrifugation, dialization, ion-exchange chromatography, and size-exclusion chromatography, affinity-purification or precipitation. Thus, the term "purified" does not require absolute purity; rather, it is intended as a relative term. Thus, for example, a purified nucleic acid or protein preparation is one in which the specified nucleic acid or protein is more enriched than the nucleic acid or protein is in its generative environment, for instance within a cell or in a biochemical reaction chamber. A preparation of substantially pure nucleic acid or protein can be purified such that the desired nucleic acid represents at least 50% of the total nucleic acid content of the preparation. In certain embodiments, a substantially pure nucleic acid or protein will represent at least 60%, at least 70%, at least 80%, at least 85%, at least 90%, or at least 95% or more of the total nucleic acid or protein content of the preparation.

An "isolated" biological component (such as a nucleic acid molecule, protein or organelle) has been substantially separated or purified away from other biological components in the cell of the organism in which the component naturally occurs, such as, other chromosomal and extra-chromosomal DNA and RNA, proteins and organelles. Nucleic acids and proteins that have been "isolated" include nucleic acids and proteins purified by standard purification methods. The term also embraces nucleic acids and proteins prepared by recombinant expression in a host cell as well as chemically synthesized nucleic acids and proteins.

An "antigen" is a compound, composition, or substance that can stimulate the production of antibodies and/or a T cell response in a subject, including compositions that are injected, absorbed or otherwise introduced into a subject. The term "antigen" includes all related antigenic epitopes. The term "epitope" or "antigenic determinant" refers to a site on an antigen to which B and/or T cells respond. The "dominant antigenic epitopes" or "dominant epitope" are those epitopes to which a functionally significant host immune response, e.g., an antibody response or a T-cell response, is made. Thus, with respect to a protective immune response against a pathogen, the dominant antigenic epitopes are those antigenic moieties that when recognized by the host immune system result in protection from disease caused by the pathogen. The term "T-cell epitope" refers to an epitope that when bound to an appropriate MHC molecule is specifically bound by a T cell (via a T cell receptor). A "B-cell epitope" is an epitope that is specifically bound by an antibody (or B cell receptor molecule).

An "adjuvant" is an agent that enhances the production of an immune response in a non-antigen specific manner. Common adjuvants include suspensions of minerals (alum, aluminum hydroxide, aluminum phosphate) onto which antigen is adsorbed; emulsions, including water-in-oil, and oil-in-water (and variants thereof, including double emulsions and reversible emulsions), liposaccharides, lipopolysaccharides, immunostimulatory nucleic acids (such as CpG oligonucleotides), liposomes, Toll-like Receptor agonists (particularly, TLR2, TLR4, TLR7/8 and TLR9 agonists), and various combinations of such components.

An "antibody" or "immunoglobulin" is a plasma protein, made up of four polypeptides that binds specifically to an antigen. An antibody molecule is made up of two heavy chain polypeptides and two light chain polypeptides (or multiples thereof) held together by disulfide bonds. In humans, antibodies are defined into five isotypes or classes: IgG, IgM, IgA, IgD, and IgE. IgG antibodies can be further divided into four sublclasses (IgG1, IgG2, IgG3 and IgG4). A "neutralizing" antibody is an antibody that is capable of inhibiting the infectivity of a virus. For example, neutralizing antibodies specific for RSV are capable of inhibiting or reducing the infectivity of RSV.

An "immunogenic composition" is a composition of matter suitable for administration to a human or animal subject (e.g., in an experimental or clinical setting) that is capable of eliciting a specific immune response, e.g., against a pathogen, such as RSV or *B. pertussis.* As such, an immunogenic composition includes one or more antigens (for example, polypeptide antigens) or antigenic epitopes. An immunogenic composition can also include one or more additional components capable of eliciting or enhancing an immune response, such as an excipient, carrier, and/or adjuvant. In certain instances, immunogenic compositions are administered to elicit an immune response that protects the subject against symptoms or conditions induced by a pathogen. In some cases, symptoms or disease caused by a pathogen is prevented (or reduced or ameliorated) by inhibiting replication of the pathogen (e.g., RSV or *B. pertussis*) following exposure of the subject to the pathogen. In the context of this disclosure, the term immunogenic composition will be understood to encompass compositions that are intended for administration to a subject or population of subjects for the purpose of eliciting a protective or palliative immune response against RSV and/or *B. pertussis* (that is, vaccine compositions or vaccines). Where "combination immunogenic composition" is used herein, this is intended as a reference specifically to immunogenic compositions disclosed herein which comprise both RSV and *B. pertussis* antigens (as opposed to immunogenic compositions comprising RSV but not *B. pertussis* antigens, or vice versa).

An "immune response" is a response of a cell of the immune system, such as a B cell, T cell, or monocyte, to a stimulus, such as a pathogen or antigen (e.g., formulated as an immunogenic composition or vaccine). An immune response can be a B cell response, which results in the production of specific antibodies, such as antigen specific neutralizing antibodies. An immune response can also be a T cell response, such as a CD4+ response or a CD8+ response. B cell and T cell responses are aspects of a "cellular" immune response. An immune response can also be a "humoral" immune response, which is mediated by antibodies. In some cases, the response is specific for a particular antigen (that is, an "antigen-specific response"). If the antigen is derived from a pathogen, the antigen-specific response is a "pathogen-specific response." A "protective immune response" is an immune response that inhibits a detrimental function or activity of a pathogen, reduces infection by a pathogen, or decreases symptoms (including death) that result from infection by the pathogen. A protective immune response can be measured, for example, by the inhibition of viral replication or plaque formation in a plaque reduction assay or ELISA-neutralization assay, or by measuring resistance to pathogen challenge in vivo. Exposure of a subject to an immunogenic stimulus, such as a pathogen or antigen (e.g., formulated as an immunogenic composition or vaccine), elicits a primary immune response specific for the stimulus, that is, the exposure "primes" the immune response. A subsequent exposure, e.g., by immunization, to the stimulus can increase or "boost" the magnitude (or duration, or both) of the specific immune response. Thus, "boosting" a preexisting immune response by administering an immunogenic composition increases the magnitude of an antigen (or pathogen) specific response, (e.g., by increasing antibody titre and/or affinity, by increasing the frequency of antigen specific B or T cells, by inducing maturation effector function, or any combination thereof).

A "Th1" biased immune response is characterized by the presence of CD4+ T helper cells that produce IL-2 and IFN-γ, and thus, by the secretion or presence of IL-2 and IFN-γ. In contrast, a "Th2" biased immune response is characterized by a preponderance of CD4+ helper cells that produce IL-4, IL-5, and IL-13.

An "immunologically effective amount" is a quantity of a composition (typically, an immunogenic composition) used to elicit an immune response in a subject to the composition or to an antigen in the composition. Commonly, the desired result is the production of an antigen (e.g., pathogen)-specific immune response that is capable of or contributes to protecting the subject against the pathogen. However, to obtain a protective immune response against a pathogen can require multiple administrations of the immunogenic composition. Thus, in the context of this disclosure, the term immunologically effective amount encompasses a fractional dose that contributes in combination with previous or subsequent administrations to attaining a protective immune response.

The adjective "pharmaceutically acceptable" indicates that the referent is suitable for administration to a subject (e.g., a human or animal subject). Remington's Pharmaceutical Sciences, by E. W. Martin, Mack Publishing Co., Easton, PA, 15th Edition (1975), describes compositions and formulations (including diluents) suitable for pharmaceutical delivery of therapeutic and/or prophylactic compositions, including immunogenic compositions.

The term "modulate" in reference to a response, such as an immune response, means to alter or vary the onset, magnitude, duration or characteristics of the response. An agent that modulates an immune response alters at least one of the onset, magnitude, duration or characteristics of an immune response following its administration, or that alters at least one of the onset, magnitude, duration or characteristic as compared to a reference agent.

The term "reduces" is a relative term, such that an agent reduces a response or condition if the response or condition is quantitatively diminished following administration of the agent, or if it is diminished following administration of the agent, as compared to a reference agent. Similarly, the term "protects" does not necessarily mean that an agent completely eliminates the risk of an infection or disease caused by infection, so long as at least one characteristic of the response or condition is substantially or significantly reduced or eliminated. Thus, an immunogenic composition that protects against or reduces an infection or a disease, or symptom thereof, can, but does not necessarily prevent or eliminate infection or disease in all subjects, so long as the incidence or severity of infection or incidence or severity of disease is measurably reduced, for example, by at least about 50%, or by at least about 60%, or by at least about 70%, or by at least about 80%, or by at least about 90% of the infection or response in the absence of the agent, or in comparison to a reference agent.

A "subject" is a living multi-cellular vertebrate organism, such as a mammal. In the context of this disclosure, the subject can be an experimental subject, such as a non-human animal, e.g., a mouse, a cotton rat, guinea pig, cow, or a non-human primate. Alternatively, the subject can be a human subject.

### RSV F PROTEIN ANALOGS

In a particular embodiment the F protein analog is a prefusion F or "PreF" antigen that includes at least one modification that stabilizes the prefusion conformation of the F protein. Alternatively, F analogs stabilized in the postfusion conformation ("PostF"), or that are labile with respect to conformation can be employed. Generally the F protein analog, (e.g., PreF, PostF, etc.) antigen lacks a transmembrane domain, and is soluble, i.e., not membrane bound (for example, to facilitate expression and purification of the F protein analog).

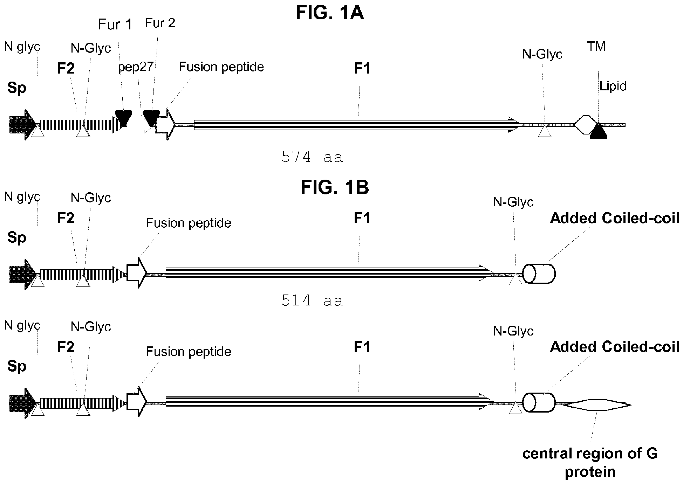

Details of the structure of the RSV F protein are provided herein with reference to terminology and designations widely accepted in the art, and illustrated schematically in FIG. 1A. A schematic illustration of exemplary PreF antigens is provided in FIG. 1B.

In exemplary embodiments, the F protein analog comprises in an N-terminal to C-terminal direction: at least a portion or substantially all of an F₂ domain and an F₁ domain of an RSV F protein polypeptide, optionally with a heterologous trimerization domain. In an embodiment, there is no furin cleavage site between the F₂ domain and the F₁ domain. In certain exemplary embodiments, the F₂ domain comprises at least a portion of an RSV F protein polypeptide corresponding to amino acids 26-105 of the reference F protein precursor polypeptide (F₀) of SEQ ID NO:2 and/or the F₁ domain comprises at least a portion of an RSV F protein polypeptide corresponding to amino acids 137-516 of the reference F protein precursor polypeptide (F₀) of SEQ ID NO:2.

For example, in specific embodiments, the F protein analog is selected from the group of:
(a) a polypeptide comprising a polypeptide selected from the group of SEQ ID NO:6, SEQ ID NO:8, SEQ ID NO:10, SEQ ID NO:18, SEQ ID NO:20 and SEQ ID NO:22;
(b) a polypeptide encoded by a polynucleotide selected from the group of SEQ ID NO:5, SEQ ID NO:7, SEQ ID NO:9, SEQ ID NO: 17, SEQ ID NO: 19 and SEQ ID NO:21, or by a polynucleotide sequence that hybridizes under stringent conditions over substantially its entire length to a polynucleotide selected from the group of SEQ ID NO:5, SEQ ID NO:7, SEQ ID NO:9, SEQ ID NO:17, SEQ ID NO:19 and SEQ ID NO:21, which polypeptide comprises an amino acid sequence corresponding at least in part to a naturally occurring RSV strain;
(c) a polypeptide with at least 95% sequence identity to a polypeptide selected from the group of SEQ ID NO:6, SEQ ID NO:8, SEQ ID NO:10, SEQ ID NO:18, SEQ ID NO:20 and SEQ ID NO:22, which polypeptide comprises an amino acid sequence that does not correspond to a naturally occurring RSV strain.

Optionally, the F protein analog further comprises a signal peptide. Optionally, the F protein analog can further comprise a "tag" or sequence to facilitate purification, e.g., a multi-histidine sequence.

In embodiments comprising a heterologous trimerization domain, such a domain can comprise a coiled-coil domain, such as an isoleucine zipper, or it can comprise an alternative trimerization domain, such as from the bacteriophage T4 fibritin ("foldon"), or influenza HA.

In certain exemplary embodiments, the F protein analog comprises at least one modification selected from:
(i) a modification that alters glycosylation.
(ii) a modification that eliminates at least one non-furin cleavage site;
(iii) a modification that deletes one or more amino acids of the pep27 domain; and
(iv) a modification that substitutes or adds a hydrophilic amino acid in a hydrophobic domain of the F protein extracellular domain.

In certain embodiments, the F protein analog comprises a multimer of polypeptides, for example, a trimer of polypeptides.

As mentioned above, in a particular embodiment the recombinant RSV antigen of the disclosed combination immunogenic composition comprises a Fusion (F) protein analog that includes a soluble F protein polypeptide, which has been modified to stabilize the prefusion conformation of the F protein, that is, the conformation of the mature assembled F protein prior to fusion with the host cell membrane. These F protein analogs are designated "PreF" or "PreF antigens", for purpose of clarity and simplicity. Such antigens, described and exemplified in WO2010/149745, exhibit improved immunogenic characteristics, and are safe and highly protective when administered to a subject *in vivo.* It will be understood by those of skill in the art that any RSV F protein can be modified to stabilize the prefusion conformation according to the teachings provided herein. Therefore, to facilitate understanding of the principles guiding production of PreF antigens, individual structural components will be indicated with reference to an exemplary F protein, the polynucleotide and amino acid sequence of which are provided in SEQ ID NOs:1 and 2, respectively. Similarly, where applicable, G protein antigens are described in reference to an exemplary G protein, the polynucleotide and amino acid sequences of which are provided in SEQ ID NOs:3 and 4, respectively.

With reference to the primary amino acid sequence of the F protein polypeptide (FIG. 1A), the following terms are utilized to describe structural features of the PreF antigens.

The term F₀ refers to a full-length translated F protein precursor. The F₀ polypeptide can be subdivided into an F₂ domain and an F₁ domain separated by an intervening peptide, designated pep27. During maturation, the F₀ polypeptide undergoes proteolytic cleavage at two furin sites situated between F₂ and F₁ and flanking pep27. For purpose of the ensuing discussion, an F₂ domain includes at least a portion, and as much as all, of amino acids 1-109, and a soluble portion of an F₁ domain includes at least a portion, and up to all, of amino acids 137-526 of the F protein. As indicated above, these amino acid positions (and all subsequent amino acid positions designated herein) are given in reference to the exemplary F protein precursor polypeptide (F₀) of SEQ ID NO:2.

The prefusion F (or "PreF") antigen is a soluble (that is, not membrane bound) F protein analog that includes at least one modification that stabilizes the prefusion conformation of the F protein, such that the RSV antigen retains at least one immunodominant epitope of the prefusion conformation of the F protein. The soluble F protein analog includes an F₂ domain and an F₁ domain of the RSV F protein (but does not include a transmembrane domain of the RSV F protein). In exemplary embodiments, the F₂ domain includes amino acids 26-105 and the F₁ domain includes amino acids 137-516 of an F protein. However, smaller portions can also be used, so long as the three-dimensional conformation of the stabilized PreF antigen is maintained. Similarly, polypeptides that include additional structural components (e.g., fusion polypeptides) can also be used in place of the exemplary F₂ and F₁ domains, so long as the additional components do not disrupt the three-dimensional conformation, or otherwise adversely impact stability, production or processing, or decrease immunogenicity of the antigen. The F₂ and F₁ domains are positioned in an N-terminal to C-terminal orientation designed to replicate folding and assembly of the F protein analog into the mature prefusion conformation. To enhance production, the F₂ domain can be preceded by a secretory signal peptide, such as a native F protein signal peptide or a heterologous signal peptide chosen to enhance production and secretion in the host cells in which the recombinant PreF antigen is to be expressed.

The PreF antigens are stabilized (in the trimeric prefusion conformation) by introducing one or more modifications, such as the addition, deletion or substitution, of one or more amino acids. One such stabilizing modification is the addition of an amino acid sequence comprising a heterologous stabilizing domain. In exemplary embodiments, the heterologous stabilizing domain is a protein multimerization domain. One particularly favorable example of such a protein multimerization domain is a coiled-coil domain, such as an isoleucine zipper domain that promotes trimerization of multiple polypeptides having such a domain. An exemplary isoleucine zipper domain is depicted in SEQ ID NO:11. Typically, the heterologous stabilizing domain is positioned C-terminal to the F₁ domain.

Optionally, the multimerization domain is connected to the F₁ domain via a short amino acid linker sequence, such as the sequence GG. The linker can also be a longer linker (for example, including the sequence GG, such as the amino acid sequence: GGSGGSGGS; SEQ ID NO:14). Numerous conformationally neutral linkers are known in the art that can be used in this context without disrupting the conformation of the PreF antigen.

Another stabilizing modification is the elimination of a furin recognition and cleavage site that is located between the F₂ and F₁ domains in the native F₀ protein. One or both furin recognition sites, located at positions 105-109 and at positions 133-136 can be eliminated by deleting or substituting one or more amino acid of the furin recognition sites, such that the protease is incapable of cleaving the PreF polypeptide into its constituent domains. Optionally, the intervening pep27 peptide can also be removed or substituted, e.g., by a linker peptide. Additionally, or optionally, a non-furin cleavage site (e.g., a metalloproteinase site at positions 112-113) in proximity to the fusion peptide can be removed or substituted.

Another example of a stabilizing mutation is the addition or substitution of a hydrophilic amino acid into a hydrophobic domain of the F protein. Typically, a charged amino acid, such as lysine, will be added or substituted for a neutral residue, such as leucine, in the hydrophobic region. For example, a hydrophilic amino acid can be added to, or substituted for, a hydrophobic or neutral amino acid within the HRB coiled-coil domain of the F protein extracellular domain. By way of example, a charged amino acid residue, such as lysine, can be substituted for the leucine present at position 512 of the F protein. Alternatively, or in addition, a hydrophilic amino acid can be added to, or substituted for, a hydrophobic or neutral amino acid within the HRA domain of the F protein. For example, one or more charged amino acids, such as lysine, can be inserted at or near position 105-106 (e.g., following the amino acid corresponding to residue 105 of reference SEQ ID NO:2, such as between amino acids 105 and 106) of the PreF antigen). Optionally, hydrophilic amino acids can be added or substituted in both the HRA and HRB domains. Alternatively, one or more hydrophobic residues can be deleted, so long as the overall conformation of the PreF antigen is not adversely impacted.

Additionally or alternatively, one or more modification may be made which alters the glycosylation state of the PreF antigen. For example, one or more amino acids in a glycosylation site present in a native RSV F protein, e.g., at or around amino acid residue 500 (as compared to SEQ ID NO:2) can be deleted or substituted (or an amino acid can be added such that that the glycosylation site is disrupted) to increase or decrease the glycosylation status of the PreF antigen. For example, the amino acids corresponding to positions 500-502 of SEQ ID NO:2 can be selected from: NGS; NKS; NGT; and NKT. Thus, in certain embodiments, the PreF antigens include a soluble F protein analog comprising an F₂ domain (e.g., corresponding to amino acids 26-105 of SEQ ID NO:2) and an F₁ domain (e.g., corresponding to amino acids 137-516 of SEQ ID NO:2) of an RSV F protein polypeptide, in which at least one modification that alters glycosylation has been introduced. The RSV PreF antigen, typically includes an intact fusion peptide between the F₂ domain and the F₁ domain. Optionally, the PreF antigen includes a signal peptide.

As disclosed above, such F protein analogs can include at least one modification selected from:
(i) an addition of an amino acid sequence comprising a heterologous trimerization domain (such as a isoleucine zipper domain);
(ii) a deletion of at least one furin cleavage site;
(iii) a deletion of at least one non-furin cleavage site;
(iv) a deletion of one or more amino acids of the pep27 domain; and
(v) at least one substitution or addition of a hydrophilic amino acid in a hydrophobic domain of the F protein extracellular domain.

As disclosed above, such glycosylation-modified RSV PreF antigens assemble into multimers, e.g., trimers.

In exemplary embodiments, the glycosylation-modified PreF antigens are selected from the group of:
a) a polypeptide comprising or consisting of SEQ ID NO:22;
b) a polypeptide encoded by SEQ ID NO:21 or by a polynucleotide sequence that hybridizes under stringent conditions over substantially its entire length to SEQ ID NO:21;
c) a polypeptide with at least 95% sequence identity to SEQ ID NO:22.

Any and/or all of the stabilizing modifications can be used individually and/or in combination with any of the other stabilizing modifications disclosed herein to produce a PreF antigen. In exemplary embodiments the PreF protein comprising a polypeptide comprising an F₂ domain and an F₁ domain with no intervening furin cleavage site between the F₂ domain and the F₁ domain, and with a heterologous stabilizing domain (e.g., trimerization domain) positioned C-terminal to the F₁ domain. In certain embodiments, the PreF antigen also includes one or more addition and/or substitution of a hydrophilic residue into a hydrophobic HRA and/or HRB domain. Optionally, the PreF antigen has a modification of at least one non-furin cleavage site, such as a metalloproteinase site.

A PreF antigen can optionally include an additional polypeptide component that includes at least an immunogenic portion of the RSV G protein. That is, in certain embodiments, the PreF antigen is a chimeric protein that includes both an F protein and a G protein component. The F protein component can be any of the PreF antigens described above, and the G protein component is selected to be an immunologically active portion of the RSV G protein (up to and/or including a full-length G protein). In exemplary embodiments, the G protein polypeptide includes amino acids 149-229 of a G protein (where the amino acid positions are designated with reference to the G protein sequence represented in SEQ ID NO:4). One of skill in the art will appreciate that a smaller portion or fragment of the G protein can be used, so long as the selected portion retains the dominant immunologic features of the larger G protein fragment. In particular, the selected fragment retains the immunologically dominant epitope between about amino acid positions 184-198 (e.g., amino acids 180-200), and be sufficiently long to fold and assemble into a stable conformation that exhibits the immunodominant epitope. Longer fragments can also be used, e.g., from about amino acid 128 to about amino acid 229, up to the full-length G protein. So long as the selected fragment folds into a stable conformation in the context of the chimeric protein, and does not interfere with production, processing or stability when produced recombinantly in host cells. Optionally, the G protein component is connected to the F protein component via a short amino acid linker sequence, such as the sequence GG. The linker can also be a longer linker (such as the amino acid sequence: GGSGGSGGS: SEQ ID NO:14). Numerous conformationally neutral linkers are known in the art that can be used in this context without disrupting the conformation of the PreF antigen.

Optionally, the G protein component can include one or more amino acid substitutions that reduce or prevent enhanced viral disease in an animal model of RSV disease. That is, the G protein can include an amino acid substitution, such that when an immunogenic composition including the PreF-G chimeric antigen is administered to a subject selected from an accepted animal model (e.g., mouse model of RSV), the subject exhibits reduced or no symptoms of vaccine enhanced viral disease (e.g., eosinophilia, neutrophilia), as compared to a control animal receiving a vaccine that contains an unmodified G protein. The reduction and/or prevention of vaccine enhanced viral disease can be apparent when the immunogenic compositions are administered in the absence of adjuvant (but not, for example, when the antigens are administered in the presence of a strong Th1 inducing adjuvant). Additionally, the amino acid substitution can reduce or prevent vaccine enhanced viral disease when administered to a human subject. An example of a suitable amino acid substitution is the replacement of asparagine at position 191 by an alanine (Asn → Ala at amino acid 191: N191A).

Optionally, any PreF antigen described above can include an additional sequence that serves as an aid to purification. One example, is a polyhistidine tag. Such a tag can be removed from the final product if desired.

When expressed, the PreF antigens undergo intramolecular folding and assemble into mature protein that includes a multimer of polypeptides. Favorably, the preF antigen polypeptides assemble into a trimer that resembles the prefusion conformation of the mature, processed, RSV F protein.

In some embodiments, the immunogenic composition includes a PreF antigen (such as the exemplary embodiment illustrated by SEQ ID NO:6) and a second polypeptide that includes a G protein component. The G protein component typically includes at least amino acids 149-229 of a G protein. Although smaller portions of the G protein can be used, such fragments should include, at a minimum, the immunological dominant epitope of amino acids 184-198. Alternatively, the G protein can include a larger portion of the G protein, such as amino acids 128-229 or 130-230, optionally as an element of a larger protein, such as a full-length G protein, or a chimeric polypeptide.

In other embodiments, the immunogenic composition includes a PreF antigen that is a chimeric protein that also includes a G protein component (such as the exemplary embodiments illustrated by SEQ ID NOs:8 and 10). The G protein component of such a chimeric PreF (or PreF-G) antigen typically includes at least amino acids 149-229 of a G protein. As indicated above, smaller or larger fragments (such as amino acids 129-229 or 130-230) of the G protein can also be used, so long as the immunodominant epitopes are retained, and conformation of the PreF-G antigen is not adversely impacted.

Additional details regarding PreF antigens, and methods of using them, are presented below, and in the Examples.

The recombinant RSV antigens disclosed herein are F protein analogs derived from, and corresponding immunologically in whole or in part to, the RSV F protein. They can include one or more modifications that alter the structure or function of the F protein but retain the immunological properties of the F protein such that an immune response generated against an F protein analog will recognize the native F protein and thus recognize RSV. F protein analogs described herein are useful as immunogens.

In nature, the RSV F protein is expressed as a single polypeptide precursor 574 amino acids in length, designated F₀. In vivo, F₀ oligomerizes in the endoplasmic reticulum and is proteolytically processed by a furin protease at two conserved furin consensus sequences (furin cleavage sites), RARR109 (SEQ ID NO:15) and RKRR136 (SEQ ID NO:16) to generate an oligomer consisting of two disulfide-linked fragments. The smaller of these fragments is termed F₂ and originates from the N-terminal portion of the F₀ precursor. The larger, C-terminal F₁ fragment anchors the F protein in the membrane via a sequence of hydrophobic amino acids, which are adjacent to a 24 amino acid cytoplasmic tail. Three F₂-F₁ dimers associate to form a mature F protein, which adopts a metastable prefusogenic ("prefusion") conformation that is triggered to undergo a conformational change upon contact with a target cell membrane. This conformational change exposes a hydrophobic sequence, known as the fusion peptide, which associates with the host cell membrane and promotes fusion of the membrane of the virus, or an infected cell, with the target cell membrane.

The F₁ fragment contains at least two heptad repeat domains, designated HRA and HRB, and situated in proximity to the fusion peptide and transmembrane anchor domains, respectively. In the prefusion conformation, the F₂-F₁ dimer forms a globular head and stalk structure, in which the HRA domains are in a segmented (extended) conformation in the globular head. In contrast, the HRB domains form a three-stranded coiled coil stalk extending from the head region. During transition from the prefusion to the postfusion conformations, the HRA domains collapse and are brought into proximity to the HRB domains to form an anti-parallel six helix bundle. In the postfusion state the fusion peptide and transmembrane domains are juxtaposed to facilitate membrane fusion.

Although the conformational description provided above is based on molecular modeling of crystallographic data, the structural distinctions between the prefusion and postfusion conformations can be monitored without resort to crystallography. For example, electron micrography can be used to distinguish between the prefusion and postfusion (alternatively designated prefusogenic and fusogenic) conformations, as demonstrated by Calder et al., Virology, 271:122-131 (2000) and Morton et al., Virology, 311:275-288, which are incorporated herein by reference for the purpose of their technological teachings. The prefusion conformation can also be distinguished from the fusogenic (postfusion) conformation by liposome association assays as described by Connolly et al., Proc. Natl. Acad. Sci. USA, 103:17903-17908 (2006), which is also incorporated herein by reference for the purpose of its technological teachings. Additionally, prefusion and fusogenic conformations can be distinguished using antibodies that specifically recognize conformation epitopes present on one or the other of the prefusion or fusogenic form of the RSV F protein, but not on the other form. Such conformation epitopes can be due to preferential exposure of an antigenic determinant on the surface of the molecule. Alternatively, conformational epitopes can arise from the juxtaposition of amino acids that are non-contiguous in the linear polypeptide.

Typically, the F protein analogs (PreF, PostF, etc.) analogs lack a transmembrane domain and cytoplasmic tail, and can also be referred to as an F protein ectodomain or soluble F protein ectodomain.

F protein analogs include an F protein polypeptide, which has been modified to stabilize the prefusion conformation of the F protein, that is, the conformation of the mature assembled F protein prior to fusion with the host cell membrane. These F protein analogs are designated "PreF analogs", "PreF" or "PreF antigens", for purpose of clarity and simplicity, and are generally soluble. The PreF analogs disclosed herein are predicated on the discovery that soluble F protein analogs that have been modified by the incorporation of a heterologous trimerization domain exhibit improved immunogenic characteristics, and are safe and highly protective when administered to a subject in vivo. Exemplary PreF antigens are described in WO2010/149745, herein incorporated by reference in its entirety for the purpose of providing examples of PreF antigens.

F protein analogs also include an F protein polypeptide which has the conformation of the postfusion F protein and which may be referred to as a PostF antigen or postfusion antigen. PostF analogs are described in WO2011/008974, incorporated herein by reference. The PostF antigen contains at least one modification to alter the structure or function of the native postfusion F protein.

The PreF analogs disclosed herein are designed to stabilize and maintain the prefusion conformation of the RSV F protein, such that in a population of expressed protein, a substantial portion of the population of expressed protein is in the prefusogenic (prefusion) conformation (e.g., as predicted by structural and/or thermodynamic modeling or as assessed by one or more of the methods disclosed above). Stabilizing modifications are introduced into a native (or synthetic) F protein, such as the exemplary F protein of SEQ ID NO:2, such that the major immunogenic epitopes of the prefusion conformation of the F protein are maintained following introduction of the PreF analog into a cellular or extracellular environment (for example, in vivo, e.g., following administration to a subject).

First, a heterologous stabilizing domain can be placed at the C-terminal end of the construct in order to replace the membrane anchoring domain of the F₀ polypeptide. This stabilizing domain is predicted to compensate for the HRB instability, helping to stabilize the -prefusion conformer. In exemplary embodiments, the heterologous stabilizing domain is a protein multimerization domain. One particularly favorable example of such a protein multimerization domain is a trimerization domain. Exemplary trimerization domains fold into a coiled-coil that promotes assembly into trimers of multiple polypeptides having such coiled-coil domains. Examples of trimerization domains include trimerization domains from influenza hemagglutinin, SARS spike, HIV gp41, modified GCN4, bacteriophage T4 fibritin and ATCase. One favorable example of a trimerization domain is an isoleucine zipper. An exemplary isoleucine zipper domain is the engineered yeast GCN4 isoleucine variant described by Harbury et al. Science 262:1401-1407 (1993). The sequence of one suitable isoleucine zipper domain is represented by SEQ ID NO:11, although variants of this sequence that retain the ability to form a coiled-coil stabilizing domain are equally suitable. Alternative stabilizing coiled coil trimerization domains include: TRAF2 (GENBANK® Accession No. Q12933 [gi:23503103]; amino acids 299-348); Thrombospondin 1 (Accession No. PO7996 [gi:135717]; amino acids 291-314); Matrilin-4 (Accession No. O95460 [gi:14548117]; amino acids 594-618; CMP (matrilin-1) (Accession No. NP_002370 [gi:4505111]; amino acids 463-496; HSF1 (Accession No. AAX42211 [gi:61362386]; amino acids 165-191; and Cubilin (Accession No. NP_001072 [gi:4557503]; amino acids 104-138. It is expected that a suitable trimerization domain results in the assembly of a substantial portion of the expressed protein into trimers. For example, at least 50% of a recombinant PreF polypeptide having a trimerization domain will assemble into a trimer (e.g., as assessed by AFF-MALS). Typically, at least 60%, more favorably at least 70%, and most desirably at least about 75% or more of the expressed polypeptide exists as a trimer.

Another example of a stabilizing mutation is the addition or substitution of a hydrophilic amino acid into a hydrophobic domain of the F protein. Typically, a charged amino acid, such as lysine, will be added or substituted for a neutral residue, such as leucine, in the hydrophobic region. For example, a hydrophilic amino acid can be added to, or substituted for, a hydrophobic or neutral amino acid within the HRB coiled-coil domain of the F protein extracellular domain. By way of example, a charged amino acid residue, such as lysine, can be substituted for the leucine present at position 512 the F protein (relative to the native F₀ polypeptide; L482K of the exemplary PreF analog polypeptide of SEQ ID NO:6). Alternatively, or in addition, a hydrophilic amino acid can be added to, or substituted for, a hydrophobic or neutral amino acid within the HRA domain of the F protein. For example, one or more charged amino acids, such as lysine, can be inserted at or near position 105-106 (e.g., following the amino acid corresponding to residue 105 of reference SEQ ID NO:2, such as between amino acids 105 and 106) of the PreF analog). Optionally, hydrophilic amino acids can be added or substituted in both the HRA and HRB domains. Alternatively, one or more hydrophobic residues can be deleted, so long as the overall conformation of the PreF analog is not adversely impacted.

Secondly, pep27 can be removed. Analysis of a structural model of the RSV F protein in the prefusion state suggests that pep27 creates a large unconstrained loop between F₁ and F₂. This loop does not contribute to stabilization of the prefusion state, and is removed following cleavage of the native protein by furin. Thus, pep27 can also be removed from embodiments that involve a postfusion (or other) conformational analog.

Third, one or both furin cleavage motifs can be deleted (from between the F₂ and F₁ domains in the native F₀ protein). One or both furin recognition sites, located at positions 105-109 or 106-109 and at positions 133-136 can be eliminated by deleting or substituting one or more amino acid of the furin recognition sites, for example deleting one or more amino acids or substituting one or more amino acids or a combination of one or more substitutions or deletions, or modifying such that the protease is incapable of cleaving the PreF (or other F protein analog) polypeptide into its constituent domains. Optionally, the intervening pep27 peptide can also be removed or substituted, e.g., by a linker peptide. Additionally, or optionally, a non-furin cleavage site (e.g., a metalloproteinase site at positions 112-113) in proximity to the fusion peptide can be removed or substituted.

Thus, an F protein analog for use in the methods and uses according to the disclosure can be obtained which is an uncleaved ectodomain having one or more altered furin cleavage sites. Such F protein analog polypeptides are produced recombinantly in a host cell which secretes them uncleaved at position from amino acid 101 to 161, e.g. not cleaved at the furin cleavage sites at positions 105-109 and 131-136. In particular embodiments, the substitution K131Q, the deletion of the amino acids at positions 131-134, or the substitutions K131Q or R133Q or R135Q or R136Q, are used to inhibit cleavage at 136/137.

In an exemplary design, the fusion peptide is not cleaved from F₂, preventing release from the globular head of the prefusion conformer and accessibility to nearby membranes. Interaction between the fusion peptide and the membrane interface is predicted to be a major issue in the prefusion state instability. During the fusion process, interaction between the fusion peptide and the target membrane results in the exposure of the fusion peptide from within the globular head structure, enhancing instability of the prefusion state and folding into post-fusion conformer. This conformation change enables the process of membrane fusion. Removal of one or both of the furin cleavage sites is predicted to prevent membrane accessibility to the N-terminal part of the fusion peptide, stabilizing the prefusion state. Thus, in exemplary embodiments disclosed herein, removal of the furin cleavage motifs results in a PreF analog that comprises an intact fusion peptide, which is not cleaved by furin during or following processing and assembly.

Optionally, at least one non-furin cleavage site can also be removed, for example by substitution of one or more amino acids. For example, experimental evidence suggests that under conditions conducive to cleavage by certain metalloproteinases, the F protein analog can be cleaved in the vicinity of amino acids 110-118 (for example, with cleavage occurring between amino acids 112 and 113 of the F protein analog; between a leucine at position 142 and glycine at position 143 of the reference F protein polypeptide of SEQ ID NO:2). Accordingly, modification of one or more amino acids within this region can reduce cleavage of the F protein analog. For example, the leucine at position 112 can be substituted with a different amino acid, such as isoleucine, glutamine or tryptophan (as shown in the exemplary embodiment of SEQ ID NO:20). Alternatively or additionally, the glycine at position 113 can be substituted by a serine or alanine. In further embodiments the F prtein analogs further contain altered trypsin cleavage sites, and F protein analogs are not cleaved by trypsin at a site between amino acid 101 and 161.

Optionally, a F protein analog can include one or more modifications that alters the glycosylation pattern or status (e.g., by increasing or decreasing the proportion of molecules glycosylated at one or more of the glycosylation sites present in a native F protein polypeptide). For example, the native F protein polypeptide of SEQ ID NO:2 is predicted to be glycosylated at amino acid positions 27, 70 and 500 (corresponding to positions 27, 70 and 470 of the exemplary PreF analog of SEQ ID NO:6). In an embodiment, a modification is introduced in the vicinity of the glycosylation site at amino acid position 500 (designated N470). For example, the glycosylation site can be removed by substituting an amino acid, such as glutamine (Q) in place of the asparagine at position 500 (of the reference sequence, which corresponds by alignment to position 470 of the exemplary PreF analog). Favorably, a modification that increases glycosylation efficiency at this glycosylation site is introduced. Examples of suitable modifications include at positions 500-502, the following amino acid sequences: NGS; NKS; NGT; NKT. Interestingly, it has been found that modifications of this glycosylation site that result in increased glycosylation also result in substantially increased PreF production. Thus, in certain embodiments, the PreF analogs have a modified glycosylation site at the position corresponding to amino acid 500 of the reference PreF sequence (SEQ ID NO:2), e.g., at position 470 of the PreF analog exemplified by SEQ ID NO:6). Suitable, modifications include the sequences: NGS; NKS; NGT; NKT at amino acids corresponding to positions 500-502 of the reference F protein polypeptide sequence. The amino acid of an exemplary embodiment that includes an "NGT" modification is provided in SEQ ID NO:18. One of skill in the art can easily determine similar modifications for corresponding NGS, NKS, and NKT modifications. Such modifications are favorably combined with any of the stabilizing mutations disclosed herein (e.g., a heterologous coiled-coil, such as an isoleucine zipper, domain and/or a modification in a hydrophobic region, and/or removal of pep27, and/or removal of a furin cleavage site, and/or removal of a non-furin cleavage site, and/or removal of a non-furin cleavage site). For example, in one specific embodiment, the F protein analog includes a substitution that eliminates a non-furin cleavage site and a modification that increases glycosylation. An exemplary PreF analog sequence is provided in SEQ ID NO:22 (which exemplary embodiment includes an "NGT" modification and the substitution of glutamine in the place of leucine at position 112). For example, in certain exemplary embodiments, the glycosylation modified PreF analogs are selected from the group of:
a) a polypeptide comprising or consisting of SEQ ID NO:22;
b) a polypeptide encoded by SEQ ID NO:21 or by a polynucleotide sequence that hybridizes under stringent conditions over substantially its entire length to SEQ ID NO:21;
c) a polypeptide with at least 95% sequence identity to SEQ ID NO:22.

More generally, any one of the stabilizing modifications disclosed herein, e.g., addition of a heterologous stabilizing domain, such as a coiled-coil (for example, an isoleucine zipper domain), preferably situated at the C-terminal end of the soluble F protein analog; modification of a residue, such as leucine to lysine, in the hydrophobic HRB domain; removal of pep27; removal of one or both furin cleavage motifs; removal of a non-furin cleavage site such as a trypsin cleavage site; and/or modification of a glycosylation site can be employed in combination with any one or more (or up to all-in any desired combination) of the other stabilizing modifications. For example, a heterologous coiled-coil (or other heterologous stabilizing domain) can be utilized alone or in combination with any of: a modification in a hydrophobic region, and/or removal of pep27, and/or removal of a furin cleavage site, and/or removal of a non-furin cleavage site, and/or removal of a non-furin cleavage site. In certain specific embodiments, the F protein analog, such as the PreF analog, includes a C-terminal coiled-coil (isoleucine zipper) domain, a stabilizing substitution in the HRB hydrophobic domain, and removal of one or both furin cleavage sites. Such an embodiment includes an intact fusion peptide that is not removed by furin cleavage. In one specific embodiment, the F protein analog also includes a modified glycosylation site at amino acid position 500.

The F protein analog for the compositions and methods described herein can be produced by a method which comprises providing a biological material containing the F protein analog (e.g., PreF analog, PostF analog or uncleaved F protein ectodomain, etc.) and purifying the analog polypeptide monomers or multimers (e.g., trimers) or a mixture thereof from the biological material.

The F protein analog can be in the form of polypeptide monomers or trimers, or a mixture of monomers and trimers which may exist in equilibrium. The presence of a single form may provide advantages such as a more predictable immune response and better stability.

Thus, in an embodiment, the F protein analog for use according to the disclosure is a purified F protein analog, which may be in the form of monomers or trimers or a mixture of monomers and trimers, substantially free of lipids and lipoproteins.

The F protein polypeptide can be selected from any F protein of an RSV A or RSV B strain, or from variants thereof (as defined above). In certain exemplary embodiments, the F protein polypeptide is the F protein represented by SEQ ID NO:2. To facilitate understanding of this disclosure, all amino acid residue positions, regardless of strain, are given with respect to (that is, the amino acid residue position corresponds to) the amino acid position of the exemplary F protein. Comparable amino acid positions of any other RSV A or B strain can be determined easily by those of ordinary skill in the art by aligning the amino acid sequences of the selected RSV strain with that of the exemplary sequence using readily available and well-known alignment algorithms (such as BLAST, e.g., using default parameters). Numerous additional examples of F protein polypeptides from different RSV strains are disclosed in WO2008/114149 (which is incorporated herein by reference for the purpose of providing additional examples of RSV F and G protein sequences). Additional variants can arise through genetic drift, or can be produced artificially using site directed or random mutagenesis, or by recombination of two or more preexisting variants. Such additional variants are also suitable in the context of the F protein analogs utilized in the context of the immunogenic compositions disclosed herein.

In alternative embodiments useful in the compositions and methods described herein the recombinant RSV protein is an F protein analog as described in WO2011/008974, incorporated herein by reference for the purpose of describing additional F protein analogs, see for example F protein analogs in Figure 1 of WO2011/008974 and also described in Example 1 of WO2011/008974.

In selecting F₂ and F₁ domains of the F protein, one of skill in the art will recognize that it is not strictly necessary to include the entire F₂ and/or F₁ domain. Typically, conformational considerations are of importance when selecting a subsequence (or fragment) of the F₂ domain. Thus, the F₂ domain typically includes a portion of the F₂ domain that facilitates assembly and stability of the polypeptide. In certain exemplary variants, the F₂ domain includes amino acids 26-105. However, variants having minor modifications in length (by addition, or deletion of one or more amino acids) are also possible.

Typically, at least a subsequence (or fragment) of the F₁ domain is selected and designed to maintain a stable conformation that includes immunodominant epitopes of the F protein. For example, it is generally desirable to select a subsequence of the F₁ polypeptide domain that includes epitopes recognized by neutralizing antibodies in the regions of amino acids 262-275 (palivizumab neutralization) and 423-436 (Centocor's ch101F MAb). Additionally, it may be desirable to include T cell epitopes, e.g., in the region of animo acids 328-355, most commonly, as a single contiguous portion of the F₁ subunit (e.g., spanning amino acids 262-436) but epitopes could be retained in a synthetic sequence that includes these immunodominant epitopes as discontinuous elements assembled in a stable conformation. Thus, an F₁ domain polypeptide comprises at least about amino acids 262-436 of an RSV F protein polypeptide. In one non-limiting example provided herein, the F₁ domain comprises amino acids 137 to 516 of a native F protein polypeptide. One of skill in the art will recognize that additional shorter subsequences can be used at the discretion of the practitioner.

When selecting a subsequence of the F₂ or F₁ domain (e.g., as discussed below with respect to the G protein component of certain PreF-G analogs), in addition to conformational consideration, it can be desirable to choose sequences (e.g., variants, subsequences, and the like) based on the inclusion of additional immunogenic epitopes. For example, additional T cell epitopes can be identified using anchor motifs or other methods, such as neural net or polynomial determinations, known in the art, see, e.g., RANKPEP (available on the world wide web at: mif.dfci.harvard.edu/Tools/rankpep.html); ProPredI (available on the world wide web at: imtech.res.in/raghava/propredI/index.html); Bimas (available on the world wide web at: www-bimas.dcrt.nih.gov/molbi/hla_bind/index.html); and SYFPEITH (available on the world wide web at: syfpeithi.bmi-heidelberg.com/scripts/MHCServer.dll/home.htm). For example, algorithms are used to determine the "binding threshold" of peptides, and to select those with scores that give them a high probability of MHC or antibody binding at a certain affinity. The algorithms are based either on the effects on MHC binding of a particular amino acid at a particular position, the effects on antibody binding of a particular amino acid at a particular position, or the effects on binding of a particular substitution in a motif-containing peptide. Within the context of an immunogenic peptide, a "conserved residue" is one which appears in a significantly higher frequency than would be expected by random distribution at a particular position in a peptide. Anchor residues are conserved residues that provide a contact point with the MHC molecule. T cell epitopes identified by such predictive methods can be confirmed by measuring their binding to a specific MHC protein and by their ability to stimulate T cells when presented in the context of the MHC protein.

Favorably, the F protein analog, for example a PreF analog (including PreF-G analogs as discussed below), a Post F analog, or other conformational analog, includes a signal peptide corresponding to the expression system, for example, a mammalian or viral signal peptide, such as an RSV F0 native signal sequence (e.g., amino acids 1-25 of SEQ ID NO:2 or amino acids 1-25 of SEQ ID NO:6). Typically, the signal peptide is selected to be compatible with the cells selected for recombinant expression. For example, a signal peptide (such as a baculovirus signal peptide, or the melittin signal peptide, can be substituted for expression, in insect cells. Suitable plant signal peptides are known in the art, if a plant expression system is preferred. Numerous exemplary signal peptides are known in the art, (see, e.g., see Zhang & Henzel, Protein Sci., 13:2819-2824 (2004), which describes numerous human signal peptides) and are catalogued, e.g., in the SPdb signal peptide database, which includes signal sequences of archaea, prokaryotes and eukaryotes (http://proline.bic.nus.edu.sg/spdb/). Optionally, any of the preceding antigens can include an additional sequence or tag, such as a His-tag to facilitate purification.

Optionally, the F protein analog (for example, the PreF or Post F or other analog) can include additional immunogenic components. In certain particularly favorable embodiments, the F protein analog includes an RSV G protein antigenic component. Exemplary chimeric proteins having a PreF and G component include the following PreF_V1 (represented by SEQ ID NOs:7 and 8) and PreF_V2 (represented by SEQ ID NOs:9 and 10).

In the PreF-G analogs , an antigenic portion of the G protein (e.g., a truncated G protein, such as amino acid residues 149-229) is added at the C-terminal end of the construct. Typically, the G protein component is joined to the F protein component via a flexible linker sequence. For example, in the exemplary PreF_V1 design, the G protein is joined to the PreF component by a -GGSGGSGGS- linker (SEQ ID NO:14). In the PreF_V2 design, the linker is shorter. Instead of having the -GGSGGSGGS- linker (SEQ ID NO:14), PreF_V2 has 2 glycines (-GG-) for linker.

Where present, the G protein polypeptide domain can include all or part of a G protein selected from any RSV A or RSV B strain. In certain exemplary embodiments, the G protein is (or is 95% identical to) the G protein represented by SEQ ID NO:4. Additional examples of suitable G protein sequences can be found in WO2008/114149 (which is incorporated herein by reference).

The G protein polypeptide component is selected to include at least a subsequence (or fragment) of the G protein that retains the immunodominant T cell epitope(s), e.g., in the region of amino acids 183-197, such as fragments of the G protein that include amino acids 151-229, 149-229, or 128-229 of a native G protein. In one exemplary embodiment, the G protein polypeptide is a subsequence (or fragment) of a native G protein polypeptide that includes all or part of amino acid residues 149 to 229 of a native G protein polypeptide. One of skill in the art will readily appreciate that longer or shorter portions of the G protein can also be used, so long as the portion selected does not conformationally destabilize or disrupt expression, folding or processing of the F protein analog. Optionally, the G protein domain includes an amino acid substitution at position 191, which has previously been shown to be involved in reducing and/or preventing enhanced disease characterized by eosinophilia associated with formalin inactivated RSV vaccines. A thorough description of the attributes of naturally occurring and substituted (N191A) G proteins can be found, e.g., in US Patent Publication No. 2005/0042230, which is incorporated herein by reference.

Alternatively, the F protein analog can be formulated in an immunogenic composition that also contains a second polypeptide that includes a G protein component. The G protein component typically includes at least amino acids 149-229 of a G protein. Although smaller portions of the G protein can be used, such fragments should include, at a minimum, the immunological dominant epitope of amino acids 184-198. Alternatively, the G protein can include a larger portion of the G protein, such as amino acids 128-229 or 130-230, optionally as an element of a larger protein, such as a full-length G protein, or a chimeric polypeptide.

For example, with respect to selection of sequences corresponding to naturally occurring strains, one or more of the domains can correspond in sequence to an RSV A or B strain, such as the common laboratory isolates designated A2 or Long, or any other naturally occurring strain or isolate. Numerous strains of RSV have been isolated to date. Exemplary strains indicated by GenBank and/or EMBL Accession number can be found in WO2008114149, which is incorporated herein by reference for the purpose of disclosing the nucleic acid and polypeptide sequences of RSV F suitable for use in F protein analogs disclosed herein. Additional strains of RSV are likely to be isolated, and are encompassed within the genus of RSV. Similarly, the genus of RSV encompasses variants arising from naturally occurring (e.g., previously or subsequently identified strains) by genetic drift, and/or recombination.

In addition to such naturally occurring and isolated variants, engineered variants that share sequence similarity with the aforementioned sequences can also be employed in the context of F protein analogs, including PreF, PostF or other analogs (including F-G) analogs. It will be understood by those of skill in the art, that the similarity between F protein analog polypeptide (and polynucleotide sequences as described below), as for polypeptide (and nucleotide sequences in general), can be expressed in terms of the similarity between the sequences, otherwise referred to as sequence identity. Sequence identity is frequently measured in terms of percentage identity (or similarity); the higher the percentage, the more similar are the primary structures of the two sequences. In general, the more similar the primary structures of two amino acid (or polynucleotide) sequences, the more similar are the higher order structures resulting from folding and assembly. Variants of an F protein, polypeptide (and polynucleotide) sequences typically have one or a small number of amino acid deletions, additions or substitutions but will nonetheless share a very high percentage of their amino acid, and generally their polynucleotide sequence. More importantly, the variants retain the structural and, thus, conformational attributes of the reference sequences disclosed herein.

Methods of determining sequence identity are well known in the art, and are applicable to F protein analog polypeptides, as well as the nucleic acids that encode them (e.g., as decribed below). Various programs and alignment algorithms are described in: Smith and Waterman, Adv. Appl. Math. 2:482, 1981; Needleman and Wunsch, J. Mol. Biol. 48:443, 1970; Higgins and Sharp, Gene 73:237, 1988; Higgins and Sharp, CABIOS 5:151, 1989; Corpet et al., Nucleic Acids Research 16:10881, 1988; and Pearson and Lipman, Proc. Natl. Acad. Sci. USA 85:2444, 1988. Altschul et al., Nature Genet. 6:119, 1994, presents a detailed consideration of sequence alignment methods and homology calculations. The NCBI Basic Local Alignment Search Tool (BLAST) (Altschul et al., J. Mol. Biol. 215:403, 1990) is available from several sources, including the National Center for Biotechnology Information (NCBI, Bethesda, MD) and on the internet, for use in connection with the sequence analysis programs blastp, blastn, blastx, tblastn and tblastx. A description of how to determine sequence identity using this program is available on the NCBI website on the internet.

In some instances, the F protein analog has one or more amino acid modifications relative to the amino acid sequence of the naturally occurring strain from which it is derived (e.g., in addition to the aforementioned stabilizing modifications). Such differences can be an addition, deletion or substitution of one or more amino acids. A variant typically differs by no more than about 1%, or 2%, or 5%, or 10%, or 15%, or 20% of the amino acid residues. For example, a variant F protein analog, e.g., PreF or PostF or other analog polypeptide sequence can include 1, or 2, or up to 5, or up to about 10, or up to about 15, or up to about 50, or up to about 100 amino acid differences as compared to the relevant portion of a reference F protein sequence (for example, the PreF analog polypeptide sequences of SEQ ID NOs:6, 8, 10, 18, 20 and/or 22. Thus, a variant in the context of an RSV F or G protein, or F protein analog, typically shares at least 80%, or 85%, more commonly, at least about 90% or more, such as 95%, or even 98% or 99% sequence identity with a reference protein, e.g., in the case of a PreF analog: the reference sequences illustrated in SEQ ID NO:2, 4, 6, 8, 10, 18, 20 and/or 22, or any of the exemplary PreF analogs disclosed herein. Additional variants included as a feature of this disclosure are F protein analogs that include all or part of a nucleotide or amino acid sequence selected from the naturally occurring variants disclosed in WO2008/114149. Additional variants can arise through genetic drift, or can be produced artificially using site directed or random mutagenesis, or by recombination of two or more preexisting variants. Such additional variants are also suitable in the context of the F protein analog antigens disclosed herein. For example, the modification can be a substitution of one or more amino acids (such as two amino acids, three amino acids, four amino acids, five amino acids, up to about ten amino acids, or more) that do not alter the conformation or immunogenic epitopes of the resulting F protein analog.

Alternatively or additionally, the modification can include a deletion of one or more amino acids and/or an addition of one or more amino acids. Indeed, if desired, one or more of the polypeptide domains can be a synthetic polypeptide that does not correspond to any single strain, but includes component subsequences from multiple strains, or even from a consensus sequence deduced by aligning multiple strains of RSV virus polypeptides. In certain embodiments, one or more of the polypeptide domains is modified by the addition of an amino acid sequence that constitutes a tag, which facilitates subsequent processing or purification. Such a tag can be an antigenic or epitope tag, an enzymatic tag or a polyhistidine tag. Typically the tag is situated at one or the other end of the protein, such as at the C-terminus or N-terminus of the antigen or fusion protein.

The F protein analogs (and also where applicable, G antigens) disclosed herein can be produced using well established procedures for the expression and purification of recombinant proteins.

In brief, recombinant nucleic acids that encode the F protein analogs are introduced into host cells by any of a variety of well-known procedures, such as electroporation, liposome mediated transfection, Calcium phosphate precipitation, infection, transfection and the like, depending on the selection of vectors and host cells. Favorable host cells include prokaryotic (i.e., bacterial) host cells, such as E. coli, as well as numerous eukaryotic host cells, including fungal (e.g., yeast, such as Saccharomyces cerevisiae and Picchia pastoris) cells, insect cells, plant cells, and mammalian cells (such as 3T3, COS, CHO, BHK, HEK 293) or Bowes melanoma cells. Following expression in a selected host cell, the recombinant F protein analogs can be isolated and/or purified according to procedures well-known in the art. Exemplary expression methods, as well as nucleic acids that encode PreF analogs (including PreF-G analogs) are provided in WO2010/149745, which is incorporated herein for the purpose of providing suitable methods for the expression and purification of F protein analogs.

### B. PERTUSSIS ANTIGENS

In a particular embodiment of the disclosed combination immunogenic compositions, the at least one *B. pertussis* antigen comprises at least one Pa antigen selected from the group consisting of: pertussis toxoid (PT), filamentous haemagglutinin (FHA), pertactin (PRN), fimbrae type 2 (FIM2), and fimbrae type 3 (FIM3). The antigens are partially or highly purified.

PT may be produced in a variety of ways, for instance by purification of the toxin from a culture *of B. pertussis* followed by chemical detoxification (for example as described in WO91/12020, incorporated herein by reference), or alternatively by purification of a genetically-detoxified analog of PT (for example, as described in the following, incorporated herein by reference for the purpose of disclosing contemplated genetic modifications of PT: EP306318, EP322533, EP396964, EP322115, EP275689). In a particular embodiment, the PT is genetically detoxified. More particularly, the genetically-detoxified PT carries one or both of the following substitutions: R9K and E129G.

The disclosed combination immunogenic composition may comprise any 1, 2, 3, 4 or 5 of the acellular pertussis antigens PT, FHA, PRN, FIM2 and FIM3. More particularly, said composition may comprise the combinations: PT and FHA; PT, FHA and PRN; PT, FHA, PRN and FIM2; PT, FHA, PRN and FIM3; and PT, FHA, PRN, FIM2 and FIM3.

In a particular embodiment, PT is used at an amount of 2-50µg (for example exactly or approximately 2.5 or 3.2µg per dose), 5-40µg (for example exactly or approximately 5 or 8µg per dose) or 10-30µg (for example exactly or approximately 20 or 25µg per dose).

In a particular embodiment, FHA is used at an amount of 2-50µg (for example exactly or approximately 2.5 or 34.4µg per dose), 5-40µg (for example exactly or approximately 5 or 8µg per dose) or 10-30µg (for example exactly or approximately 20 or 25µg per dose).

In a particular embodiment, PRN is used at an amount of 0.5-20µg, 0.8-15µg (for example exactly or approximately 0.8 or 1.6µg per dose) or 2-10µg (for example exactly or approximately 2.5 or 3 or 8µg per dose).

In a particular embodiment, FIM2 and/or FIM3 are used at a total amount of 0.5-10µg (for example exactly or approximately 0.8 or 5µg per dose).

In a particular embodiment, the combination immunogenic composition comprises PT and FHA at equivalent amounts per dose, being either exactly or approximately 8 or 20 or 25µg. Alternatively, the combination immunogenic composition comprises PT and FHA at exactly or approximately 5 and 2.5µg respectively, or exactly or approximately 3.2 and 34.4µg. In a further embodiment, the immunogenic composition comprises PT, FHA and PRN at the respective exact or approximate amounts per dose: 25:5:8µg; 8:8:2.5µg; 20:20:3µg; 2.5:53µg; 5:2.5:2.5µg; or 3.2:34.4:1.6µg.

Alternatively, or in combination with any of the above-discussed Pa antigens, the disclosed combination immunogenic composition may comprise an antigen derived from the *B. pertussis* 'BrkA' protein (as disclosed in WO2005/032584, and Marr et al (2008), Vaccine, 26(34):4306-4311, incorporated herein by reference).

In a further embodiment, the at least one Pa antigen comprises an outer membrane vesicle (OMV) obtained from *B. pertussis,* as disclosed in Roberts et al (2008), Vaccine, 26:4639-4646, incorporated herein by reference. In particular, such OMV may be derived from a recombinant *B. pertussis* strain expressing a lipid A-modifying enzyme, such as a 3-O-deacylase, for example PagL (Asensio et al (2011), Vaccine, 29:1649-1656, incorporated herein by reference).

In an alternative embodiment, the at least one *B. pertussis* antigen comprises a Pw antigen. Pw may be inactivated by several known methods, including mercury-free methods. Such methods may include heat (e.g. 55-65°C or 56-60°C, for 5-60 minutes or for 10-30 minutes, e.g. 60°C for 30 minutes), formaldehyde (e.g. 0.1% at 37°, 24 hours), glutaraldehyde (e.g. 0.05% at room temperature, 10 minutes), acetone-I (e.g. three treatments at room temperature) or acetone-II (e.g. three treatments at room temperature and fourth treatment at 37°C) inactivation (see for example Gupta et al., 1987, J. Biol. Stand. 15:87; Gupta et al., 1986, Vaccine, 4:185). Methods of preparing killed Pw antigen suitable for use in the combination immunogenic composition are disclosed in WO93/24148.

More particularly, the combination immunogenic composition comprises Pw at a perdose amount of (in International Opacity Units, "IOU"): 5-50, 7-40, 9-35, 11-30, 13-25, 15-21, or approximately or exactly 20.

In a particular embodiment of a Pw-comprising combination immunogenic composition according to the disclosure, the Pw component of the composition elicits reduced reactogenicity. Reactogenicity (pain, fever, swelling etc) of Pw vaccines is primarily caused by lipo-oligosaccharide ('LOS', which is synonymous with lipo-polysaccharide ('LPS') in the context of *B. pertussis;* 'LOS' will be used herein), which is the endotoxin from the bacterial outer membrane. The lipid A part of LOS is mainly responsible. In order to produce a less reactogenic Pw-containing vaccine (relative to 'traditional' Pw vaccines such as produced by the above-discussed inactivation procedures), the endotoxin can be genetically or chemically detoxified and/or extracted from the outer membrane. However, this must be done in a way which does not substantially impair the immunogenicity of the Pw antigen, as LOS is a potent adjuvant of the immune system.

In one embodiment, the at least one *B. pertussis* antigen of the disclosed combination immunogenic composition comprises a 'low reactogenicity' Pw antigen in which the LOS has been genetically or chemically detoxified and/or extracted. For example, the Pw antigen may be subjected to treatment with a mixture of an organic solvent, such as butanol, and water, as described in WO2006/002502 and Dias et al (2012), Human Vaccines & Immunotherapeutics, 9(2):339-348 which are incorporated herein by reference for the purpose of disclosing chemical extraction of LOS.

In an alternative embodiment, 'low reactogenicity' is achieved by deriving the Pw antigen from a *B. pertussis* strain genetically engineered to produce a less toxic LOS. WO2006/065139 (incorporated herein by reference) discloses genetic 3-*O*-deacylation and detoxification *of B. pertussis* LOS, resulting in strains comprising at least partially 3-*O-*deacylated LOS. The at least one *B. pertussis* antigen of the combination immunogenic composition may therefore be a Pw antigen derived from a strain of *B. pertussis* which has been engineered to express a lipid A-modifying enzyme, such as a de-O-acylase. In particular, such a strain may express PagL as described in WO2006/065139, as well as in Geurtsen et al (2006), Infection and Immunity, 74(10):5574-5585 and Geurtsen et al (2007), Microbes and Infection, 9:1096-1103, all incorporated herein by reference. Alternatively or additionally, the strain from which the Pw antigen is derived may naturally, or as a result of engineering: lack the ability to modify its lipid A phosphate groups with glucosamine; have a lipid A diglucosamine backbone substituted with at the C-3' position with C10-OH or C12-OH; and/or express molecular LOS species that lack a terminal heptose. Such a strain, 18-323, is disclosed in Marr et al (2010), The Journal of Infectious Diseases, 202(12):1897-1906 (incorporated herein by reference).

### IMMUNOGENIC COMPOSITION

The combination immunogenic compositions disclosed herein typically contain a pharmaceutically acceptable carrier or excipients, and optionally contain additional antigens.

Pharmaceutically acceptable carriers and excipients are well known and can be selected by those of skill in the art. For example, the carrier or excipient can favorably include a buffer. Optionally, the carrier or excipient also contains at least one component that stabilizes solubility and/or stability. Examples of solubilizing/stabilizing agents include detergents, for example, laurel sarcosine and/or tween. Alternative solubilizing/stabilizing agents include arginine, and glass forming polyols (such as sucrose, trehalose and the like). Numerous pharmaceutically acceptable carriers and/or pharmaceutically acceptable excipients are known in the art and are described, e.g., in Remington's Pharmaceutical Sciences, by E. W. Martin, Mack Publishing Co., Easton, PA, 5th Edition (975).

Accordingly, suitable excipients and carriers can be selected by those of skill in the art to produce a formulation suitable for delivery to a subject by a selected route of administration.

Suitable excipients include, without limitation: glycerol, Polyethylene glycol (PEG), Sorbitol, Trehalose, N-lauroylsarcosine sodium salt, L -proline, Non detergent sulfobetaine, Guanidine hydrochloride, Urea, Trimethylamine oxide, KCl, Ca2+, Mg2+ , Mn2+ , Zn2+ and other divalent cation related salts, Dithiothreitol, Dithioerytrol, and ß-mercaptoethanol. Other excipients can be detergents (including: Tween80, Tween20, Triton X-00, NP-40, Empigen BB, Octylglucoside, Lauroyl maltoside, Zwittergent 3-08, Zwittergent 3-0, Zwittergent 3-2, Zwittergent 3-4, Zwittergent 3-6, CHAPS, Sodium deoxycholate, Sodium dodecyl sulphate, Cetyltrimethylammonium bromide).

Optionally, the disclosed combination immunogenic composition also includes an adjuvant, which adjuvant also may be used with the disclosed vaccine regimens, methods, uses and kits. When the combination immunogenic composition is to be administered to a subject of a particular age group susceptible to (or at increased risk of) RSV and/or *B*. *pertussis* infection, the adjuvant is selected to be safe and effective in the subject or population of subjects. Thus, when formulating a combination immunogenic composition for administration in an elderly subject (such as a subject greater than 65 years of age), the adjuvant is selected to be safe and effective in elderly subjects. Similarly, when the combination immunogenic composition is intended for administration in neonatal or infant subjects (such as subjects between birth and the age of two years), the adjuvant is selected to be safe and effective in neonates and infants. In the case of an adjuvant selected for safety and efficacy in neonates and infants, an adjuvant dose can be selected that is a dilution (e.g., a fractional dose) of a dose typically administered to an adult subject.

Additionally, the adjuvant is typically selected to enhance the desired aspect of the immune response when administered via a route of administration, by which the combination immunogenic composition is administered. For example, when formulating a combination immunogenic composition for nasal administration, proteosome and protollin are favorable adjuvants. In contrast, when the combination immunogenic composition is formulated for intramuscular administration, adjuvants including one or more of 3D-MPL, squalene (e.g., QS21), liposomes, and/or oil and water emulsions are favorably selected.

One suitable adjuvant for use in combination with RSV F protein analog antigens is a non-toxic bacterial lipopolysaccharide derivative. An example of a suitable non-toxic derivative of lipid A, is monophosphoryl lipid A or more particularly 3-Deacylated monophoshoryl lipid A (3D-MPL). 3D-MPL is sold under the name MPL by GlaxoSmithKline Biologicals N.A., and is referred throughout the document as MPL or 3D-MPL. See, for example, US Patent Nos. 4,436,727; 4,877,611; 4,866,034 and 4,912,094. 3D-MPL primarily promotes CD4+ T cell responses with an IFN-γ (Th1) phenotype. 3D-MPL can be produced according to the methods disclosed in GB2220211 A. Chemically it is a mixture of 3-deacylated monophosphoryl lipid A with 3, 4, 5 or 6 acylated chains. In the compositions of the present disclosure small particle 3D-MPL can be used. Small particle 3D-MPL has a particle size such that it can be sterile-filtered through a 0.22µm filter. Such preparations are described in WO94/21292.

A lipopolysaccharide, such as 3D-MPL, can be used at amounts between 1 and 50µg, per human dose of the immunogenic composition. Such 3D-MPL can be used at a level of about 25µg, for example between 20-30µg, suitably between 21-29µg or between 22 and 28µg or between 23 and 27µg or between 24 and 26µg, or 25µg. In another embodiment, the human dose of the immunogenic composition comprises 3D-MPL at a level of about 10µg, for example between 5 and 15µg, suitably between 6 and 14µg, for example between 7 and 13µg or between 8 and 12µg or between 9 and 11µg, or 10µg. In a further embodiment, the human dose of the immunogenic composition comprises 3D-MPL at a level of about 5µg, for example between 1 and 9µg, or between 2 and 8µg or suitably between 3 and 7µg or 4 and µg, or 5µg.

In other embodiments, the lipopolysaccharide can be a β(1-6) glucosamine disaccharide, as described in US Patent No. 6,005,099 and EP Patent No. 0 729 473 B1. One of skill in the art would be readily able to produce various lipopolysaccharides, such as 3D-MPL, based on the teachings of these references. Nonetheless, each of these references is incorporated herein by reference. In addition to the aforementioned immunostimulants (that are similar in structure to that of LPS or MPL or 3D-MPL), acylated monosaccharide and disaccharide derivatives that are a sub-portion to the above structure of MPL are also suitable adjuvants. In other embodiments, the adjuvant is a synthetic derivative of lipid A, some of which are described as TLR-4 agonists, and include, but are not limited to: OM174 (2-deoxy-6-o-[2-deoxy-2-[(R)-3-dodecanoyloxytetra-decanoylamino]-4-o-phosphono-β-D-glucopyranosyl]-2-[(R)-3-hydroxytetradecanoylamino]-α-D-glucopyranosyldihydrogenphosphate), (WO 95/14026); OM 294 DP (3S, 9R)-3--[(R)-dodecanoyloxytetradecanoylamino]-4-oxo-5-aza-9(R)-[(R)-3-hydroxytetradecanoylamino]decan-1,10-diol,1,10-bis(dihydrogenophosphate) (WO 99/64301 and WO 00/0462); and OM 197 MP-Ac DP (3S-, 9R) -3-[(R)-dodecanoyloxytetradecanoylamino]-4-oxo-5-aza-9-[(R)-3-hydroxytetradecanoylamino]decan-1,10-diol,1 -dihydrogenophosphate 10-(6-aminohexanoate) (WO 01/46127).

Other TLR4 ligands which can be used are alkyl Glucosaminide phosphates (AGPs) such as those disclosed in WO 98/50399 or US Patent No. 6,303,347 (processes for preparation of AGPs are also disclosed), suitably RC527 or RC529 or pharmaceutically acceptable salts of AGPs as disclosed in US Patent No. 6,764,840. Some AGPs are TLR4 agonists, and some are TLR4 antagonists. Both are thought to be useful as adjuvants.

Other suitable TLR-4 ligands, capable of causing a signaling response through TLR-4 (Sabroe et al, JI 2003 p1630-5) are, for example, lipopolysaccharide from gram-negative bacteria and its derivatives, or fragments thereof, in particular a non-toxic derivative of LPS (such as 3D-MPL). Other suitable TLR agonists are: heat shock protein (HSP) 10, 60, 65, 70, 75 or 90; surfactant Protein A, hyaluronan oligosaccharides, heparan sulphate fragments, fibronectin fragments, fibrinogen peptides and b-defensin-2, and muramyl dipeptide (MDP). In one embodiment the TLR agonist is HSP 60, 70 or 90. Other suitable TLR-4 ligands are as described in WO 2003/011223 and in WO 2003/099195, such as compound I, compound II and compound III disclosed on pages 4-5 of WO2003/011223 or on pages 3-4 of WO2003/099195 and in particular those compounds disclosed in WO2003/011223 as ER803022, ER803058, ER803732, ER804053, ER804057, ER804058, ER804059, ER804442, ER804680, and ER804764. For example, one suitable TLR-4 ligand is ER804057.

Additional TLR agonists are also useful as adjuvants. The term "TLR agonist" refers to an agent that is capable of causing a signaling response through a TLR signaling pathway, either as a direct ligand or indirectly through generation of endogenous or exogenous ligand. Such natural or synthetic TLR agonists can be used as alternative or additional adjuvants. A brief review of the role of TLRs as adjuvant receptors is provided in Kaisho & Akira, Biochimica et Biophysica Acta 1589:1-13, 2002. These potential adjuvants include, but are not limited to agonists for TLR2, TLR3, TLR7, TLR8 and TLR9. Accordingly, in one embodiment, the adjuvant and combination immunogenic composition further comprises an adjuvant which is selected from the group consisting of: a TLR-1 agonist, a TLR-2 agonist, TLR-3 agonist, a TLR-4 agonist, TLR-5 agonist, a TLR-6 agonist, TLR-7 agonist, a TLR-8 agonist, TLR-9 agonist, or a combination thereof.

In one embodiment of the present disclosure, a TLR agonist is used that is capable of causing a signaling response through TLR-1. Suitably, the TLR agonist capable of causing a signaling response through TLR-1 is selected from: Tri-acylated lipopeptides (LPs); phenol-soluble modulin; Mycobacterium tuberculosis LP; S-(2,3-bis(palmitoyloxy)-(2-RS)-propyl)-N-palmitoyl-(R)-Cys-(S)-Ser-(S)-Lys(4)-OH, trihydrochloride (Pam3Cys) LP which mimics the acetylated amino terminus of a bacterial lipoprotein and OspA LP from *Borrelia burgdorferi.*

In an alternative embodiment, a TLR agonist is used that is capable of causing a signaling response through TLR-2. Suitably, the TLR agonist capable of causing a signaling response through TLR-2 is one or more of a lipoprotein, a peptidoglycan, a bacterial lipopeptide from *M tuberculosis, B burgdorferi* or *T pallidum*; peptidoglycans from species including *Staphylococcus aureus;* lipoteichoic acids, mannuronic acids, *Neisseria* porins, bacterial fimbriae, Yersina virulence factors, CMV virions, measles haemagglutinin, and zymosan from yeast.

In an alternative embodiment, a TLR agonist is used that is capable of causing a signaling response through TLR-3. Suitably, the TLR agonist capable of causing a signaling response through TLR-3 is double stranded RNA (dsRNA), or polyinosinic-polycytidylic acid (Poly IC), a molecular nucleic acid pattern associated with viral infection.

In an alternative embodiment, a TLR agonist is used that is capable of causing a signaling response through TLR-5. Suitably, the TLR agonist capable of causing a signaling response through TLR-5 is bacterial flagellin.

In an alternative embodiment, a TLR agonist is used that is capable of causing a signaling response through TLR-6. Suitably, the TLR agonist capable of causing a signaling response through TLR-6 is mycobacterial lipoprotein, di-acylated LP, and phenol-soluble modulin. Additional TLR6 agonists are described in WO 2003/043572.

In an alternative embodiment, a TLR agonist is used that is capable of causing a signaling response through TLR-7. Suitably, the TLR agonist capable of causing a signaling response through TLR-7 is a single stranded RNA (ssRNA), loxoribine, a guanosine analogue at positions N7 and C8, or an imidazoquinoline compound, or derivative thereof. In one embodiment, the TLR agonist is imiquimod. Further TLR7 agonists are described in WO 2002/085905.

In an alternative embodiment, a TLR agonist is used that is capable of causing a signaling response through TLR-8. Suitably, the TLR agonist capable of causing a signaling response through TLR-8 is a single stranded RNA (ssRNA), an imidazoquinoline molecule with anti-viral activity, for example resiquimod (R848); resiquimod is also capable of recognition by TLR-7. Other TLR-8 agonists which can be used include those described in WO 2004/071459.

In an alternative embodiment, a TLR agonist is used that is capable of causing a signaling response through TLR-9. In one embodiment, the TLR agonist capable of causing a signaling response through TLR-9 is HSP90. Alternatively, the TLR agonist capable of causing a signaling response through TLR-9 is bacterial or viral DNA, DNA containing unmethylated CpG nucleotides, in particular sequence contexts known as CpG motifs. CpG-containing oligonucleotides induce a predominantly Th1 response. Such oligonucleotides are well known and are described, for example, in WO 96/02555, WO 99/33488 and U.S. Patent Nos. 6,008,200 and 5,856,462. Suitably, CpG nucleotides are CpG oligonucleotides. Suitable oligonucleotides for use in the combination immunogenic composition are CpG containing oligonucleotides, optionally containing two or more dinucleotide CpG motifs separated by at least three, suitably at least six or more nucleotides. A CpG motif is a Cytosine nucleotide followed by a Guanine nucleotide. The CpG oligonucleotides are typically deoxynucleotides. In a specific embodiment the internucleotide in the oligonucleotide is phosphorodithioate, or suitably a phosphorothioate bond, although phosphodiester and other internucleotide bonds are possible. Also possible are oligonucleotides with mixed internucleotide linkages. Methods for producing phosphorothioate oligonucleotides or phosphorodithioate are described in US Patent Nos. 5,666,153, 5,278,302 and WO 95/26204.

Other adjuvants that can be used in the disclosed combination immunogenic composition, and with the disclosed vaccination regimens, methods, uses and kits comprising an F protein analog, such as a PreF analog, e.g., on their own or in combination with 3D-MPL, or another adjuvant described herein, are saponins, such as QS21. Such adjuvants are typically not employed (but could be if so desired) with a *B. pertussis* antigen.

Saponins are taught in: Lacaille-Dubois, M and Wagner H. (1996. A review of the biological and pharmacological activities of saponins. Phytomedicine vol 2 pp 363-386). Saponins are steroid or triterpene glycosides widely distributed in the plant and marine animal kingdoms. Saponins are noted for forming colloidal solutions in water which foam on shaking, and for precipitating cholesterol. When saponins are near cell membranes they create pore-like structures in the membrane which cause the membrane to burst. Haemolysis of erythrocytes is an example of this phenomenon, which is a property of certain, but not all, saponins.

Saponins are known as adjuvants in vaccines for systemic administration. The adjuvant and haemolytic activity of individual saponins has been extensively studied in the art (Lacaille-Dubois and Wagner, supra). For example, Quil A (derived from the bark of the South American tree Quillaja Saponaria Molina), and fractions thereof, are described in US 5,057,540 and "Saponins as vaccine adjuvants", Kensil, C. R., Crit Rev Ther Drug Carrier Syst, 1996, 12 (1-2):1-55; and EP 0 362 279 B1. Particulate structures, termed Immune Stimulating Complexes (ISCOMS), comprising fractions of Quil A are haemolytic and have been used in the manufacture of vaccines (Morein, B., EP 0 109 942 B1; WO 96/11711; WO 96/33739). The haemolytic saponins QS21 and QS17 (HPLC purified fractions of Quil A) have been described as potent systemic adjuvants, and the method of their production is disclosed in US Patent No.5,057,540 and EP 0 362 279 B1, which are incorporated herein by reference. Other saponins which have been used in systemic vaccination studies include those derived from other plant species such as Gypsophila and Saponaria (Bomford et al., Vaccine, 10(9):572-577, 1992).

QS21 is an Hplc purified non-toxic fraction derived from the bark of Quillaja Saponaria Molina. A method for producing QS21 is disclosed in US Patent No. 5,057,540. Non-reactogenic adjuvant formulations containing QS21 are described in WO 96/33739. The aforementioned references are incorporated by reference herein. Said immunologically active saponin, such as QS21, can be used in amounts of between 1 and 50µg, per human dose of the combination immunogenic composition. Advantageously QS21 is used at a level of about 25µg, for example between 20-30µg, suitably between 21-29µg or between 22 - 28µg or between 23 -27µg or between 24 -26µg, or 25µg. In another embodiment, the human dose of the combination immunogenic composition comprises QS21 at a level of about 10µg, for example between 5 and 15µg, suitably between 6 -14µg, for example between 7 -13µg or between 8 -12µg or between 9 -11µg, or 10µg. In a further embodiment, the human dose of the combination immunogenic composition comprises QS21 at a level of about 5µg, for example between 1-9µg, or between 2 -8µg or suitably between 3-7µg or 4 -6µg, or 5µg. Such formulations comprising QS21 and cholesterol have been shown to be successful adjuvants when formulated together with an antigen. Thus, for example, RSV F protein analog polypeptides can favorably be employed in the combination immunogenic composition with an adjuvant comprising a combination of QS21 and cholesterol.

Optionally, the adjuvant can also include mineral salts such as an aluminium salt, in particular aluminium hydroxide or aluminium phosphate, or calcium phosphate. For example, an adjuvant containing 3D-MPL in combination with an aluminium salt (e.g., aluminium hydroxide or "alum") is suitable for formulation in a combination immunogenic composition containing a RSV F protein analog antigen for administration to a human subject. Alternatively, such mineral salt adjuvants may be used other than in combination with non-mineral-salt adjuvants, i.e. the combination immunogenic composition may be adjuvanted only with one, or more than one, mineral salt adjuvant such as aluminium hydroxide, aluminium phosphate and calcium phosphate.

Another class of suitable adjuvants for use in formulations with RSV F protein analog antigens (and optionally, if desired, with pertussis antigens, such as purified acellular *B. pertussis* proteins) includes OMP-based immunostimulatory compositions. OMP-based immunostimulatory compositions are particularly suitable as mucosal adjuvants, e.g., for intranasal administration. OMP-based immunostimulatory compositions are a genus of preparations of outer membrane proteins (OMPs, including some porins) from Gram-negative bacteria, such as, but not limited to, *Neisseria* species (see, e.g., Lowell et al., J. Exp. Med. 167:658, 1988; Lowell et al., Science 240:800, 1988; Lynch et al., Biophys. J. 45:104, 1984; Lowell, in "New Generation Vaccines" 2nd ed., Marcel Dekker, Inc., New York, Basil, Hong Kong, page 193, 1997; U.S. Pat. No. 5,726,292; U.S. Pat. No. 4,707,543), which are useful as a carrier or in compositions for immunogens, such as bacterial or viral antigens. Some OMP-based immunostimulatory compositions can be referred to as "Proteosomes," which are hydrophobic and safe for human use. Proteosomes have the capability to auto-assemble into vesicle or vesicle-like OMP clusters of about 20 nm to about 800 nm, and to noncovalently incorporate, coordinate, associate (e.g., electrostatically or hydrophobically), or otherwise cooperate with protein antigens (Ags), particularly antigens that have a hydrophobic moiety. Any preparation method that results in the outer membrane protein component in vesicular or vesicle-like form, including multi-molecular membranous structures or molten globular-like OMP compositions of one or more OMPs, is included within the definition of Proteosome. Proteosomes can be prepared, for example, as described in the art (see, e.g., U.S. Pat. No. 5,726,292 or U.S. Pat. No. 5,985,284). Proteosomes can also contain an endogenous lipopolysaccharide or lipooligosaccharide (LPS or LOS, respectively) originating from the bacteria used to produce the OMP porins (e.g., *Neisseria* species), which generally will be less than 2% of the total OMP preparation.

Proteosomes are composed primarily of chemically extracted outer membrane proteins (OMPs) from *Neisseria menigitidis* (mostly porins A and B as well as class 4 OMP), maintained in solution by detergent (Lowell GH. Proteosomes for Improved Nasal, Oral, or Injectable Vaccines. In: Levine MM, Woodrow GC, Kaper JB, Cobon GS, eds, New Generation Vaccines. New York: Marcel Dekker, Inc. 1997; 193-206). Proteosomes can be formulated with a variety of antigens such as purified or recombinant proteins derived from viral sources, including the RSV F protein polypeptides disclosed herein, e.g., by diafiltration or traditional dialysis processes or with purified *B. pertussis* antigenic proteins. The gradual removal of detergent allows the formation of particulate hydrophobic complexes of approximately 100-200nm in diameter (Lowell GH. Proteosomes for Improved Nasal, Oral, or Injectable Vaccines. In: Levine MM, Woodrow GC, Kaper JB, Cobon GS, eds, New Generation Vaccines. New York: Marcel Dekker, Inc. 1997; 193-206).

"Proteosome: LPS or Protollin" as used herein refers to preparations of proteosomes admixed, e.g., by the exogenous addition, with at least one kind of lipo-polysaccharide to provide an OMP-LPS composition (which can function as an immunostimulatory composition). Thus, the OMP-LPS composition can be comprised of two of the basic components of Protollin, which include (1) an outer membrane protein preparation of Proteosomes (e.g., Projuvant) prepared from Gram-negative bacteria, such as Neisseria meningitidis, and (2) a preparation of one or more liposaccharides. A lipo-oligosaccharide can be endogenous (e.g., naturally contained with the OMP Proteosome preparation), can be admixed or combined with an OMP preparation from an exogenously prepared lipo-oligosaccharide (e.g., prepared from a different culture or microorganism than the OMP preparation), or can be a combination thereof. Such exogenously added LPS can be from the same Gram-negative bacterium from which the OMP preparation was made or from a different Gram-negative bacterium. Protollin should also be understood to optionally include lipids, glycolipids, glycoproteins, small molecules, or the like, and combinations thereof. The Protollin can be prepared, for example, as described in U.S. Patent Application Publication No. 2003/0044425.

Combinations of different adjuvants, such as those mentioned hereinabove, can also be used in compositions with F protein analogs such as PreF analogs (and optionally also with *B. pertussis* antigens if so desired). For example, as already noted, QS21 can be formulated together with 3D-MPL. The ratio of QS21 : 3D-MPL will typically be in the order of 1 : 10 to 10 : 1; such as 1:5 to 5 : 1, and often substantially 1 : 1. Typically, the ratio is in the range of 2.5 : 1 to 1 : 1 3D-MPL: QS21. Another combination adjuvant formulation includes 3D-MPL and an aluminium salt, such as aluminium hydroxide.

In some instances, the adjuvant formulation includes a mineral salt, such as an aluminium (alum) salt for example aluminium phosphate or aluminium hydroxide, or calcium phosphate. Where alum is present, e.g., in combination with 3D-MPL, the amount is typically between about 100µg and 1mg, such as from about 100µg, or about 200µg to about 750µg, such as about 500µg per dose.

In some embodiments, the adjuvant includes an oil and water emulsion, e.g., an oil-in-water emulsion. One example of an oil-in-water emulsion comprises a metabolisable oil, such as squalene, a tocol such as a tocopherol, e.g., alpha-tocopherol, and a surfactant, such as sorbitan trioleate (Span 85™) or polyoxyethylene sorbitan monooleate (Tween 80™), in an aqueous carrier. In certain embodiments, the oil-in-water emulsion does not contain any additional immunostimulants(s), (in particular it does not contain a non-toxic lipid A derivative, such as 3D-MPL, or a saponin, such as QS21). The aqueous carrier can be, for example, phosphate buffered saline. Additionally the oil-in-water emulsion can contain span 85 and/or lecithin and/or tricaprylin.

In another embodiment the combination immunogenic composition comprises an oil-in-water emulsion and optionally one or more further immunostimulants, wherein said oil-in-water emulsion comprises 0.5-10 mg metabolisable oil (suitably squalene), 0.5-11 mg tocol (suitably a tocopherol, such as alpha-tocopherol) and 0.4-4 mg emulsifying agent.

In one specific embodiment, the adjuvant formulation includes 3D-MPL prepared in the form of an emulsion, such as an oil-in-water emulsion. In some cases, the emulsion has a small particle size of less than 0.2µm in diameter, as disclosed in WO 94/21292. For example, the particles of 3D-MPL can be small enough to be sterile filtered through a 0.22micron membrane (as described in European Patent number 0 689 454). Alternatively, the 3D-MPL can be prepared in a liposomal formulation. Optionally, the adjuvant containing 3D-MPL (or a derivative thereof) also includes an additional immunostimulatory component.

The adjuvant is selected to be safe and effective in the population to which the immunogenic composition is administered. For adult and elderly populations, the formulations typically include more of an adjuvant component than is typically found in an infant formulation. In particular formulations using an oil-in-water emulsion, such an emulsion can include additional components, for example, such as cholesterol, squalene, alpha tocopherol, and/or a detergent, such as tween 80 or span85. In exemplary formulations, such components can be present in the following amounts: from about 1-50mg cholesterol, from 2 to 10% squalene, from 2 to 10% alpha tocopherol and from 0.3 to 3% tween 80. Typically, the ratio of squalene: alpha tocopherol is equal to or less than 1 as this provides a more stable emulsion. In some cases, the formulation can also contain a stabilizer.

When a combination immunogenic composition with a RSV F protein polypeptide antigen is formulated for administration to an infant, the dosage of adjuvant is determined to be effective and relatively non-reactogenic in an infant subject. Generally, the dosage of adjuvant in an infant formulation is lower (for example, the dose may be a fraction of the dose provided in a formulation to be administered to adults) than that used in formulations designed for administration to adult (e.g., adults aged 65 or older). For example, the amount of 3D-MPL is typically in the range of 1µg-200µg, such as 10-100µg, or 10µg-50µg per dose. An infant dose is typically at the lower end of this range, e.g., from about 1µg to about 50µg, such as from about 2µg, or about 5µg, or about 10µg, to about 25µg, or to about 50µg. Typically, where QS21 is used in the formulation, the ranges are comparable (and according to the ratios indicated above). In the case of an oil and water emulsion (e.g., an oil-in-water emulsion), the dose of adjuvant provided to a child or infant can be a fraction of the dose administered to an adult subject. A demonstration of the efficacy of immunogenic compositions containing a PreF antigen in combination various doses of an exemplary oil-in-water adjuvant is provided in WO2010/149745.

In compositions (including the disclosed combination immunogenic composition) containing an RSV F protein analog and a *B. pertussis* antigen for maternal immunisation, the composition is designed to induce a strong neutralizing antibody response. Mothers have already been exposed to RSV and *B. pertussis* and therefore will have an existing primed response, so the goal for providing protection for the infant is to boost this primed response as effectively as possible and in respect of the antibody subclasses such as IgG₁ that can cross the placenta with high efficiency and provide protection to the infant. This can be achieved without including an adjuvant, or by including an adjuvant that includes only mineral salts, in particular aluminium hydroxide (alum), aluminium phosphate or calcium phosphate. Alternatively, this can be achieved by formulating with an oil and water emulsion adjuvant, or another adjuvant that enhances the production of antibodies of the IgG₁ subclass. Thus the F protein analog for use in maternal immunisation is favorably formulated with a mineral salt, favorably alum, or with an oil and water emulsion adjuvant.

In this context the adjuvant is selected to be safe and well tolerated in pregnant women. Optionally, the immunogenic compositions also include an adjuvant other than alum. For example, adjuvants including one or more of 3D-MPL, squalene (e.g., QS21), liposomes, and/or oil and water emulsions are favorably selected, provided that the final formulation enhances the production in the primed female of RSV- and/or *B.pertussis-*specific antibodies with the desired characteristics (e.g., of subclass and neutralizing function).

It should be noted that regardless of the adjuvant selected, the concentration in the final formulation is calculated to be safe and effective in the target population. For example, in the context of maternal immunization, regardless of the adjuvant selected the concentration in the final formulation is calculated to be safe and effective in the target population of pregnant females.

An immunogenic composition as disclosed herein (i.e. combination), or for use in the disclosed vaccination regimens, methods, uses and kits, typically contains an immunoprotective quantity (or a fractional dose thereof) of the antigen and can be prepared by conventional techniques. In the case of maternal immunization, the required quantity is that which provides passive transfer of antibodies so as to be immunoprotective in infants of immunized pregnant females. Preparation of immunogenic compositions, including those for administration to human subjects, is generally described in Pharmaceutical Biotechnology, Vol.61 Vaccine Design-the subunit and adjuvant approach, edited by Powell and Newman, Plenum Press, 1995. New Trends and Developments in Vaccines, edited by Voller et al., University Park Press, Baltimore, Maryland, U.S.A. 1978. Encapsulation within liposomes is described, for example, by Fullerton, U.S. Patent 4,235,877. Conjugation of proteins to macromolecules is disclosed, for example, by Likhite, U.S. Patent 4,372,945 and by Armor et al., U.S. Patent 4,474,757.

Typically, the amount of antigen (e.g. protein) in each dose of the immunogenic composition is selected as an amount which induces an immunoprotective response without significant, adverse side effects in the typical subject. Immunoprotective in this context does not necessarily mean completely protective against infection; it means protection against symptoms or disease, especially severe disease associated with the pathogens. The amount of antigen can vary depending upon which specific immunogen is employed. Generally, it is expected that each human dose will comprise 1-1000µg of each protein or antigen, such as from about 1µg to about 100µg, for example, from about 1µg to about 50µg, such as about 1µg, about 2µg, about 5µg, about 10µg, about 15µg, about 20µg, about 25µg, about 30µg, about 40µg, or about 50µg, or about 60µg. Generally a human dose will be in a volume of 0.5ml. Thus the composition for the uses and methods described herein can be for example 10µg or 30µg or 60µg in a 0.5 ml human dose. The amount utilized in an immunogenic composition is selected based on the subject population (e.g., infant or elderly or pregnant female). An optimal amount for a particular composition can be ascertained by standard studies involving observation of antibody titres and other responses in subjects. Following an initial vaccination, subjects can receive a boost in about 4-12 weeks (or, for maternal immunization, at any time prior to delivery of the infant, favorably at least two or at least four weeks prior to the expected delivery date). For example, when administering an immunogenic composition to an infant subject, the initial and subsequent inoculations can be administered to coincide with other vaccines administered during this period.

Additional formulation details can be found in WO2010/149745, which is incorporated herein by reference for the purpose of providing additional details concerning formulation of immunogenic compositions comprising RSV F protein analogs such as PreF analogs.

In certain embodiments, the disclosed combination immunogenic compositions additionally comprise at least one antigen from at least one pathogenic organism other than RSV and *B. pertussis.* More particularly, said at least one antigen may be selected from the group consisting of: diphtheria toxoid (D); tetanus toxoid (T); Hepatitis B surface antigen (HBsAg); inactivated polio virus (IPV); capsular saccharide of *H. influenzae* type b (Hib) conjugated to a carrier protein; capsular saccharide of *N. meningitidis* type C conjugated to a carrier protein (MenC); capsular saccharide of *N. meningitidis* type Y conjugated to a carrier protein (MenY); capsular saccharide of *N. meningitidis* type A conjugated to a carrier protein (MenA); capsular saccharide of *N. meningitidis* type W conjugated to a carrier protein (MenW); and an antigen from *N. meningitidis* type B (MenB).

Combination vaccines containing *B. pertussis* antigens (Pa or Pw) with D and T and various combinations of other antigens such as selected from IPV, HBsAg, Hib and conjugated *N. meningitidis* capsular saccharides are well known in the art, for example as Infanrix™ (such as DTPa-IPV-HBsAg-Hib) and Boostrix™ (such as dTpa) products. In this regard, WO93/024148, WO97/000697 and WO98/019702 are incorporated by reference, as is WO02/00249 which discloses a DTPw-HepB-MenAC-Hib composition. Suitable carrier proteins for the capsular saccharide antigens are known in the art, and include T, D and the D derivative CRM197.

Particular combination immunogenic compositions comprise, in addition to at least one RSV antigen and at least one *B. pertussis* antigen: D and T; D, T and IPV; D, T and HBsAg; D, T and Hib; D, T, IPV and HBsAg; D, T, IPV and Hib; D, T, HBsAg and Hib; or D, T, IPV, HBsAg and Hib.

In a particular embodiment, D is used at the amount per dose of 1-10 International Units (IU) (for example exactly or approximately 2IU) or 10-40IU (for example exactly or approximately 20 or 30IU) or 1-10 Limit of flocculation (Lf) units (for example exactly or approximately 2 or 2.5 or 9Lf) or 10-30Lf (for example exactly or approximately 15 or 25Lf).

In a particular embodiment, T is used at the amount per dose of 10-30 IU (for example exactly or approximately 20IU) or 30-50IU (for example exactly or approximately 40IU) or 1-15Lf (for example exactly or approximately 5 or 10Lf).

In exemplary embodiments the combination immunogenic compositions comprise, in addition to the at least one RSV antigen and at least one *B. pertussis* antigen, D and T at the respective exact or approximate amounts per dose: 30:40IU; 25:10Lf; 20:40IU; 15:5Lf; 2:20IU; 2.5:5Lf; 2:5Lf; 25:10Lf; 9:5Lf. For example, such a composition may comprise (in addition to the at least one RSV antigen):
(i) 20-30ug, for example exactly or approximately 25ug of PT;
(ii) 20-30ug, for example exactly or approximately 25ug of FHA;
(iii) 1-10ug, for example exactly or approximately 3 or 8ug of PRN;
(iv) 10-30Lf, for example exactly or approximately 15 or 25Lf of D; and
(v) 1-15Lf, for example exactly or approximately 5 of 10Lf of T.

By way of another example, such a composition may comprise (in addition to the at least one RSV antigen):
(i) 2-10ug, for example exactly or approximately 2.5 or 8ug of PT;
(ii) 2-10ug, for example exactly or approximately 5 or 8ug of FHA;
(iii) 0.5-4ug, for example 2-3ug such as exactly or approximately 2.5 or 3ug of PRN;
(iv) 1-10Lf, for example exactly or approximately 2 or 2.5 or 9Lf of D; and
(v) 1-15Lf, for example exactly or approximately 5 of 10Lf of T.

The immunogenic composition may further comprise additional antigens, such as another RSV antigen (e.g., a G protein antigen as described in WO2010/149745) or a human metapneumovirus (hMPV) antigen, or an influenza antigen, or an antigen from Streptococcus or Pneumococcus. WO2010/149743 describes examples of hMPV antigens that can be combined with RSV antigens, and is incorporated herein by reference for the purpose of providing examples of hMPV antigens.

The maternal immunization embodiment described herein is carried out via a suitable route for administration for an RSV and a *B. pertussis* vaccine, including intramuscular, intranasal, or cutaneous administration. Favorably, maternal immunization as described herein is carried out cutaneously, which means that the antigen is introduced into the dermis and/or epidermis of the skin (e.g., intradermally). In a particular embodiment a recombinant RSV antigen comprising an F protein analog such as a PreF antigen or a PostF antigen and/or a *B. pertussis* antigen comprising acellular *B. pertussis* proteins or whole cell *B. pertussis* is delivered to the pregnant female cutaneously or intradermally. In a particular embodiment the immunogenic composition is formulated with an adjuvant described herein for example a saponin such as QS21, with or without 3D-MPL, for cutaneous or intradermal delivery. In another embodiment the immunogenic composition is formulated with a mineral salt such as aluminium hydroxide or aluminium phosphate or calcium phosphate, with or without an immunostimulant such as QS21 or 3D-MPL, for cutaneous or intradermal delivery. *B*. *pertussis* antigen is typically formulated in combination with an aluminium salt and can optionally be administered by a cutaneous or intradermal route. Optionally, the F protein analog and *B. pertussis* antigen are coformulated in a combination immunogenic composition as disclosed herein, e.g., in the presence of a mineral salt such as aluminium hydroxide or aluminium phosphate or calcium phosphate, with or without an immunostimulant such as QS21 or 3D-MPL, for cutaneous or intradermal delivery.

Delivery via the cutaneous route including the intradermal route may require or allow a lower dose of antigen than other routes such as intramuscular delivery. Therefore also provided is a combination immunogenic composition for cutaneous or intradermal delivery comprising at least one RSV antigen and at least one *B.pertussis* antigen in a low dose e.g. less than the normal intramuscular dose, e.g. 50% or less of the normal intramuscular dose, for example 50 µg or less, or 20 µg or less, or 10 µg or less or 5 µg or less per human dose of an F protein analog and, for example, between 1-10 µg PT, between 1-10 µg FHA, and between 0.5-4 µg PRN (with or without additional antigenic components). Optionally the immunogenic composition for cutaneous or intradermal delivery also comprises an adjuvant e.g. an aluminium salt or QS21 or 3D-MPL or a combination thereof.

Devices for cutaneous administration include short needle devices (which have a needle between about 1 and about 2 mm in length) such as those described in US 4,886,499, US5,190,521, US5,328,483, US 5,527,288, US 4,270,537, US 5,015,235, US 5,141,496, US 5,417,662 and EP1092444. Cutaneous vaccines may also be administered by devices which limit the effective penetration length of a needle into the skin, such as those described in WO99/34850, incorporated herein by reference, and functional equivalents thereof. Also suitable are jet injection devices which deliver liquid vaccines to the dermis via a liquid jet injector or via a needle which pierces the stratum corneum and produces a jet which reaches the dermis. Jet injection devices are described for example in US 5,480,381, US 5,599,302, US 5,334,144, US 5,993,412, US 5,649,912, US 5,569,189,US 5,704,911, US 5,383,851, US 5,893,397, US 5,466,220, US 5,339,163, US5,312,335, US 5,503,627, US 5,064,413, US 5,520, 639, US 4,596,556US 5 4,790,824,US 4,941,880, US 4,940,460, WO 97/37705 and WO 97/13537.

Devices for cutaneous administration also include ballistic powder/particle delivery devices which use compressed gas to accelerate vaccine in powder form through the outer layers of the skin to the dermis. Additionally, conventional syringes may be used in the classical mantoux method of cutaneous administration. However, the use of conventional syringes requires highly skilled operators and thus devices which are capable of accurate delivery without a highly skilled user are preferred. Additional devices for cutaneous administration include patches comprising immunogenic compositions as described herein. A cutaneous delivery patch will generally comprise a backing plate which includes a solid substrate (e.g. occlusive or nonocclusive surgical dressing). Such patches deliver the immunogenic composition to the dermis or epidermis via microprojections which pierce the stratum corneum. Microprojections are generally between 10Dm and 2mm, for example 20Dm to 500Dm, 30Dm to 1mm, 100 to 200, 200 to 300, 300 to 400, 400 to 500, 500 to 600, 600 to 700, 700, 800, 800 to 900, 100Dm to 400Dm, in particular between about 200Dm and 300Dm or between about 150Dm and 250Dm. Cutaneous delivery patches generally comprise a plurality of microprojections for example between 2 and 5000 microneedles for example between 1000 and 2000 microneedles. The microprojections may be of any shape suitable for piercing the stratum corneum,epidermis and/or dermis Microprojections may be shaped as disclosed in WO2000/074765 and WO2000/074766 for example. The microprojections may have an aspect ratio of at least 3:1 (height to diameter at base), at least about 2:1, or at least about 1:1. One suitable shape for the microproj ections is a cone with a polygonal bottom, for example hexagonal or rhombus-shaped. Other possible microprojection shapes are shown, for example, in U.S. Published Patent App. 2004/0087992. In a particular embodiment, microprojections have a shape which becomes thicker towards the base. The number of microprotrusions in the array is typically at least about 100, at least about 500, at least about 1000, at least about 1400, at least about 1600, or at least about 2000. The area density of microprotrusions, given their small size, may not be particularly high, but for example the number of microprotrusions per cm2 may be at least about 50, at least about 250, at least about 500, at least about 750, at least about 1000, or at least about 1500. In one embodiment of the disclosure the combination immunogenic composition is delivered to the subject within 5 hours of placing the patch on the skin of the host, for example, within 4 hours, 3 hours, 2 hours, 1 hour or 30 minutes. In a particular embodiment, the combination immunogenic composition is delivered within 20 minutes of placing the patch on the skin, for example within 30 seconds 1, 2, 3, 4, 5, 6, 7, 8, 9, 10, 11, 12, 13, 14, 15, 16, 17, 18 or 19 minutes.

The microprojections can be made of any suitable material known to the skilled person. In a particular embodiment at least part of the microprojections are biodegradable, in particular the tip of the microprojection or the outer most layer of the microprojection. In a particular embodiment substantially all the microprojection is biodegradable. The term "biodegradable" as used herein means degradable under expected conditions of in vivo use (e.g. insertion into skin), irrespective of the mechanism of biodegradation. Exemplary mechanisms of biodegradation include disintegration, dispersion, dissolution, erosion, hydrolysis, and enzymatic degradation.

Examples of microprojections comprising antigens are disclosed in WO2008/130587 and WO2009/048607. Methods of manufacture of metabolisable microneedles are disclosed in WO2008/130587 and WO2010/124255. Coating of microprojections with antigen can be performed by any method known to the skilled person for example by the methods disclosed in WO06/055844, WO06/055799.

Suitable delivery devices for cutaneous delivery including intradermal delivery, in the methods and uses described herein include the BD Soluvia™ device which is a microneedle device for intradermal administration, the Corium MicroCor™ patch delivery system, the Georgia Tech microneedle vaccine patch, the Nanopass microneedle delivery device and the Debiotech Nanoject™ microneedle device. Also provided is a cutaneous or intradermal delivery device containing a combination immunogenic composition as described herein, optionally formulated with an adjuvant such as a mineral salt e.g. alum, or QS21, or 3D-MPL or a combination thereof.

In connection with the disclosed method for eliciting an immune response against RSV and *B. pertussis,* comprising administering to a subject an immunologically effective amount of the combination immunogenic composition, the elicited immune response against RSV and *B. pertussis* advantageously comprises a protective immune response that reduces or prevents incidence, or reduces severity, of infection with RSV and *B. pertussis* and/or reduces or prevents incidence, or reduces severity, of a pathological response following infection with a RSV and *B. pertussis.* Said elicited immune response may be a booster response. Furthermore, the disclosed combination immunogenic composition achieves this without enhancing viral disease following contact with RSV.

The combination immunogenic composition can be administered via a variety of routes, including routes, such as intranasal, that directly place the antigens in contact with the mucosa of the upper respiratory tract. Alternatively, more traditional administration routes can be employed, such an intramuscular route of administration.

Thus, the combination immunogenic composition is herein contemplated for use in medicine, and in particular for the prevention or treatment in a human subject of infection by, or disease associated with, RSV and *B. pertussis.* In certain embodiments containing antigens from other pathogens, such prevention or treatment will extend to said other pathogens.

In a particular embodiment of such methods and uses, the subject is a human subject. Said human subject may be selected from the group of: a neonate; an infant; a child; an adolescent; an adult; and an elderly adult. The subject may be a pregnant female with a gestational infant. Alternatively, the subject may not be a pregnant female. Where the subject is a neonate, administration of the combination immunogenic composition may take place within 1 day, or within 1 week, or within 1 month of birth.

In a preferred embodiment, the (preferably human) subject is administered the combination immunogenic composition as a single-dose regimen, i.e. as a stand-alone dose which is not part of a pre-determined series of doses. The dose may be given at the same time as other vaccines, for example as part of an immunization schedule such as a paediatric immunization schedule. Although in such a single-dose embodiment the subject may receive more than one dose of the composition throughout the subject's lifetime, each of these doses is stand-alone and "single" in the sense of being administered in the absence of any planned further doses being deemed necessary in order to achieve the desired level of protection. In one embodiment the combination immunogenic composition is administered to a subject as a single-dose regimen only once within a 10, favourably a 5, year period. In one embodiment the subject is a human adolescent between 10 and 18 years of age and the combination immunogenic composition is administered only once, i.e. as a single-dose regimen. In another embodiment the subject is a pregnant human female and the combination immunogenic composition is administered only once per gestation, i.e. the pregnant female receives only a single dose of the composition during one episode of pregnancy.

### MATERNAL IMMUNISATION

A particular challenge in the development of a safe and effective vaccine that protects infants against disease caused by RSV and *B. pertussis* is that the highest incidence and greatest morbidity and mortality is in very young infants. Young infants, especially those born prematurely, can have an immature immune system. Thus, protecting young infants from RSV and *B. pertussis* (whooping cough) disease is important. There is also the potential for interference of antibodies transferred via the placenta to the infant ("maternal antibodies") with vaccination of the infant, such that vaccination in early infancy may not be sufficiently effective, e.g., to elicit a fully protective neutralizing antibody response.

In one aspect the present disclosure concerns vaccination regimens, methods and uses of immunogenic compositions and kits suitable for protecting young infants from disease caused by RSV and *B. pertussis* by actively immunizing pregnant women with a safe and effective immunogenic composition(s) comprising an analog of the RSV F protein and an acellular or whole cell *B. pertussis* antigen(s). The F protein analog favorably elicits antibodies (e.g., neutralizing antibodies) by boosting or increasing the magnitude of the humoral response previously primed by natural exposure to (or prior vaccination against) RSV. Similarly, the *B. pertussis* antigen elicits antibodies, by boosting or increasing the magnitude of the humoral response previously primed by natural exposure to, or prior vaccination against, *B. pertussis.* The antibodies produced in response to the F protein analog and *B. pertussis* antigen are transferred to the gestational infant via the placenta, resulting in passive immunological protection of the infant following birth and lasting through the critical period for infection and severe disease caused by RSV and *B. pertussis* (e.g., before infant vaccination is fully protective). Typically, the passive immunological protection conferred by this method lasts between birth and at least two months of age, for example up to about 6 months of age, or even longer.

All such compositions are designed to induce a strong antibody response (e.g., neutralizing antibodies). Since pregnant mothers have typically been exposed to RSV one or more times during their lives, they have an existing primed response to RSV. The proportion of the population exposed to RSV infection by adulthood is essentially 100%. Pediatric immunization programs designed to protect against and prevent whooping cough are widespread. However, despite widespread immunization, natural infection with *B. pertussis* is also common. Thus, priming to *B. pertussis* is also widespread. Therefore, the provision of protection from RSV and *B. pertussis* for the infant immediately after birth and for the crucial first few months that follow, can be achieved by boosting these primed responses as effectively as possible to increase serum antibody responses (levels) against RSV and *B. pertussis* in the mother, and favorably in respect of particular antibody subclasses (subtypes) such as IgG₁ that can cross the placenta and provide protection to the infant. In one embodiment, immunogenic compositions for use herein do not include an adjuvant, or include an adjuvant which favors a strong IgG₁ response such as a mineral salt such as an aluminium salt, in particular aluminium hydroxide, aluminium phosphate, or calcium phosphate. Thus in a particular embodiment an immunogenic composition for use in maternal immunization is favorably formulated with a mineral salt, favorably alum. In alternative embodiments, the adjuvant that favors a strong IgG₁ response is an oil-in-water emulsion, or a saponin, such as QS21 (or a detoxified version thereof).

A pregnant female can be a human female, and accordingly, the infant or gestational infant can be a human infant. For a pregnant human female the gestational age of the developing fetus is measured from the start of the last menstrual period. The number of weeks post-conception is measured from 14 days after the start of the last menstrual period. Thus, when a pregnant human female is said to be 24 weeks post conception this will be equal to 26 weeks after the start of her last menstrual period, or 26 weeks of gestation. When a pregnancy has been achieved by assisted reproductive technology, gestational stage of the developing fetus is calculated from two weeks before the date of conception.

The term "gestational infant" as used herein means the fetus or developing fetus of a pregnant female. The term "gestational age" is used to mean the number of weeks of gestation i.e. the number of weeks since the start of the last menstrual period. Human gestation is typically about 40 weeks from the start of the last menstrual period, and may conveniently be divided into trimesters, with the first trimester extending from the first day of the last menstrual period through the 13th week of gestation; the second trimester spanning from the 14th through the 27th weeks of gestation, and the third trimester starting in the 28th week and extending until birth. Thus, the third trimester starts at 26 weeks post-conception and continues through to birth of the infant.

The term "infant" when referring to a human is between 0 and two years of age It will be understood that the protection provided by the methods and uses described herein can potentially provide protection for an infant into childhood, from aged 2 to 11, or early childhood for example from ages 2 to 5, or even into adolescence, from aged 12 to 18. However it is during infancy, especially from birth to about 6 months of age, that an individual is most vulnerable to severe RSV and complications of whooping cough.

A human infant can be immunologically immature in the first few months of life, especially when born prematurely, e.g., before 35 weeks gestation, when the immune system may not be sufficiently well developed to mount an immune response capable of preventing infection or disease caused by a pathogen in the way that a developed immune system would be capable of doing in response to the same pathogen. An immunologically immature infant is more likely to succumb to infection and disease caused by a pathogen than an infant with a more developed or mature immune system. A human infant can also have an increased vulnerability to LRTIs (including pneumonia) during the first few months of life for physiological and developmental reasons, for example, airways are small and less developed or mature than in children and adults. For these reasons, when we refer herein to the first six months of infancy this may be extended for premature or pre-term infants according to the amount of time lost in gestational age below 40 weeks or below 38 weeks or below 35 weeks.

In an alternative embodiment, the pregnant female and its infant are from any species such as those described above under "subjects". For a pregnant animal, such as a pregnant guinea pig or cow, the time post-conception is measured as the time since mating. In humans and in some animals, for example guinea pigs, antibodies pass from the mother to the fetus via the placenta. Some antibody isotypes may be preferentially transferred through the placenta, for example in humans IgG₁ antibodies are the isotype most efficiently transferred across the placenta. Although subclasses exist in experimental animals, such as guinea pigs and mice, the various subclasses do not necessarily serve the same function, and a direct correlation between subclasses of humans and animals cannot easily be made.

Favorably, protecting the infant by inhibiting infection and reducing the incidence or severity of RSV and *B. pertussis* disease covers at least the neonatal period and very young infancy, for example at least the first several weeks of life following birth, such as the first month from birth, or the first two months, or the first three months, or the first four months, or the first five months, or the first six months from birth, or longer, e.g., when the infant is a full-term infant delivered at about 40 weeks of gestation or later. After the first few months, when the infant is less vulnerable to the effects of severe RSV and whooping cough, protection against RSV and *B. pertussis* infection may wane. Thus vital protection is provided during the period when it is most needed. In the case of a pre-term infant, favorably protection is provided for a longer period from birth for example an additional time period at least equaling the time interval between birth of the infant and what would have been 35 weeks gestation (i.e., by about 5 extra weeks), or 38 weeks gestation (by about 2 extra weeks), or longer depending on the gestational age of the infant at birth.

It will be evident that protecting the infant does not necessarily mean 100% protection against infection by RSV or by *B. pertussis.* Provided that that there is a reduction in incidence or severity of infection or disease it will be recognized that protection is provided. Protecting the infant favorably includes protecting the infant from severe disease and hospitalization caused by RSV and *B. pertussis.* As such, the compositions and methods disclosed herein reduce the incidence or severity of disease caused by RSV, such as lower respiratory tract infection (LRTI), pneumonia or other symptoms or disease, and *B. pertussis.* For example, as regards RSV, administration of an immunogenic composition as disclosed herein can reduce the incidence (in a cohort of infants of vaccinated mothers) of LRTI by at least about 50%, or at least about 60%, or by 60 to 70%, or by at least about 70%, or by at least about 80%, or by at least about 90% compared to infants of unvaccinated mothers. Favorably, such administration reduces the severity of LRTI by at least about 50%, or at least about 60%, or by 60 to 70%, or by at least about 70%, or by at least about 80%, or by at least about 90% compared to infected infants of unvaccinated mothers. Favorably, such administration reduces the need for hospitalization due to severe RSV disease in such a cohort by at least about 50%, or at least about 60%, or by 60 to 70%, or by at least about 70%, or by at least about 80%, or by at least about 90% compared to infected infants of unvaccinated mothers. Whether there is considered to be a need for hospitalization due to severe LRTI, or whether a particular case of LRTI is hospitalized, may vary from country to country and therefore severe LRTI as judged according to defined clinical symptoms well known in the art may be a better measure than the need for hospitalization. With respect to *B. pertussis*, administration of an immunogenic composition containing an acellular or whole cell pertussis antigen as disclosed herein can reduce the incidence (in a cohort of infants of vaccinated mothers) of severe disease (e.g., pneumonia and/or respiratory distress and failure) by at least about 50%, or at least about 60%, or by 60 to 70%, or by at least about 70%, or by at least about 80%, or by at least about 90% compared to infants of unvaccinated mothers. Favorably, such administration reduces the severity of pneumonia by at least about 50%, or at least about 60%, or by 60 to 70%, or by at least about 70%, or by at least about 80%, or by at least about 90% compared to infected infants of unvaccinated mothers. Favorably, such administration reduces the need for hospitalization due to severe complications of pertussis in such a cohort by at least about 50%, or at least about 60%, or by 60 to 70%, or by at least about 70%, or by at least about 80%, or by at least about 90% compared to infected infants of unvaccinated mothers.

Typically, according to the vaccination regimens, methods, uses and kits, the F protein analog and *B. pertussis* antigen are administered to the pregnant female during the third trimester of pregnancy (gestation), although a beneficial effect (especially pregnancies at increased risk of preterm delivery) can be obtained prior to the beginning of the third trimester. The timing of maternal immunization is designed to allow generation of maternal antibodies and transfer of the maternal antibodies to the fetus. Thus, favorably, sufficient time elapses between immunization and birth to allow optimum transfer of maternal antibodies across the placenta. Antibody transfer starts in humans generally at about 25 weeks of gestation, increasing up 28 weeks and becoming and remaining optimal from about 30 weeks of gestation. A minimum of about two to four weeks is believed to be needed between maternal immunization as described herein and birth to allow effective transfer of maternal antibodies against RSV F protein and *B. pertussis* antigens (e.g., comprising one or more of pertussis toxoid (PT), filamentous haemagglutinin (FHA), pertactin (PRN), fimbrae type 2 (FIM2), fimbrae type 3 (FIM3) and BrkA, or whole cell pertussis antigen) to the fetus. Thus maternal immunization can take place any time after 25 weeks of gestation (measured from the start of the last menstrual period), for example at or after 25, 26, 27, 28, 29, 30, 31, 32, 33 or 34 weeks of gestation (23, 24, 25, 26, 27, 28, 29, 30, 31, 32, 33, 34, 35 or 36 weeks post-conception), or at or before 38, 37, 36, 35, 34, 33, 32, 31 or 30 weeks of gestation (36, 35, 34, 33, 32, 31, 29 or 28 weeks post-conception). Favorably maternal immunization is carried out between 26 and 38 weeks, such as between 28 and 34 weeks of gestation.

Favorably maternal immunization is carried out at least two or at least three or at least four or at least five or at least six weeks prior to the expected date of delivery of the infant. Timing of administration may need to be adjusted in the case of a pregnant female who is at risk of an early delivery, in order to provide sufficient time for generation of antibodies and transfer to the fetus.

Favorably, a single dose (or respectively single doses) of the RSV F protein analog and *B. pertussis* antigen(s) or formulation thereof is administered to the pregnant female, during the period described. Maternal immunization against RSV and *B. pertussis* described herein can be considered as a "booster" for existing maternal immunity against RSV and *B. pertussis* that increases the immune response against RSV and *B. pertussis* that has previously been primed, e.g., by natural exposure or vaccination. Thus, it is expected that only a single dose is required. Therefore, in a preferred embodiment of the regimens, methods and uses disclosed herein, especially when the RSV antigenic component (e.g., recombinant protein, such as a F protein analog) and the *B. pertussis* antigenic component are co-formulated into a single (i.e. combination) immunogenic composition, the RSV F protein analog and *B. pertussis* antigens are administered as a single-dose (or respectively single doses) regimen In other words, during a single gestation (episode of pregnancy) the pregnant female is administered each of the RSV F protein analog and *B. pertussis* antigens only once, meaning that when co-formulated into a combination immunogenic composition said composition is given only once during the gestation. If a second dose is administered, this is favorably also within the time period for administration for the first dose, favorably with a time gap between the first and second doses of for example one to eight weeks or two to six weeks, for example two weeks or four weeks or six weeks.

Administration of an RSV F protein analog and a *B. pertussis* antigen to a pregnant female results in boosting maternal antibody titres, for example, increasing titres of serum (e.g., neutralizing) antibodies, preferably of the IgG₁ subclass. The increased antibody titre in the mother results in the passive transfer of RSV-specific and *B. pertussis-*specific antibodies with neutralizing effector function to the gestating infant across the placenta via an active transport mechanism mediated by Fc receptors, e.g., in the syncytiotrophoblast of the chorionic villi. Transport across the placenta of RSV-specific and *B. pertussis-*specific IgG₁ antibodies resulting from the immunization methods disclosed herein is expected to be efficient and result in titres, which in infants born at or near term, approach, equal or exceed the titres in maternal circulation. For example, titres of RSV-specific antibodies are favorably at levels of at least 30 µg/mL at birth. Typically, the titres can be at or above this level, such as at 40 µg/mL, 50 µg/mL, 60 µg/mL, or even higher, such as 75 µg/mL, 80 µg/mL, 90 µg/mL, 100 µg/mL, or even up to 120 µg/mL or higher in healthy infants born at full term gestation. These values can be on an individual basis or on a population mean basis. Favorably, the level of antibodies observed at birth is above the stated thresholds and persists for several months following birth.

Titres of pertussis- (e.g., PT-) specific antibodies are typically measured by ELISA in terms of ELISA units/ml (EU), as described, e.g., in Meade et al., "Description and evaluation of serologic assays used in a multicenter trial of acellular pertussis vaccines", Pediatrics (1995) 96:570-5, incorporated by reference. Briefly, for example, microtiter plates (e.g., Immulon 2 , VWR International, West Chester, PA, USA) are coated with standard quantities of PT, FHA, FIM or PRN. Serial dilutions of serum is incubated for approximately 2 h at 28 °C and an appropriate dilution of alkaline phosphatase-conjugated goat anti-human IgG is added. The reaction is developed and read at 405 nm. The lower limit of detection of each specific antibody is determined by multiple measurements of serially diluted reference material for each antigen and is set at 1 ELISA unit (EU) for PT, FHA and PRN, and 2 EU for FIM. Favorably, following administration of an immunogenic composition comprising a pertussis antigen as according to the vaccination regimen, method, use or as contained in the kits disclosed herein, pertussis-specific antibody titres are at levels of at least 10 EU at birth. Typically the titres can be at or above this level, such as at 20 EU, 30 EU, 40 EU, 50 EU, 60 EU, 70 EU, 80 EU, 90 EU or at or above 100 EU. These values can be on an individual basis or on a population mean basis. Favorably, the level of antibodies observed at birth is above the stated thresholds and persists for several months following birth.

Effector function, e.g., neutralizing capacity (neutralization titre) of the transferred anti-RSV antibodies can also be assessed, and provides a measure of functional attribute of the antibodies correlated with protection. For example, in the case of RSV, a specific quantity of a replication capable RSV virus and a defined dilution of serum are mixed and incubated. The virus-serum reaction mixture is then transferred onto host cells (e.g., HEp-2 cells) permissive for viral replication and incubated under conditions and for a period of time suitable for cell growth and viral replication. Non-neutralized virus is able to infect and replicate in the host cells. This leads to the formation of a given number of plaque forming units (PFU) on the cell monolayer that can be detected using a fluorochrome-tagged anti-RSV antibody. The neutralising titre is determined by calculating the serum dilution inducing a specified level of inhibition (e.g., 50% inhibition or 60% inhibition) in PFUs compared to a cell monolayer infected with virus alone, without serum. For example, the Palivizumab antibody has been shown to have a neutralization titer (50% effective concentration [EC50]) expressed as the antibody concentration required to reduce detection of RSV antigen by 50% compared with untreated virus-infected cells of 0.65 µg per mL (mean 0.75 ± 0.53 µg per mL; n=69, range 0.07-2.89 µg per mL) and 0.28 µg per mL (mean 0.35 ± 0.23 µg per mL; n=35, range 0.03-0.88 µg per mL) against clinical RSV A and RSV B isolates, respectively. Thus, in certain embodiments, the neutralization titre of antibodies transferred via the placenta to the gestational infant can be measured in the infant following birth and has (on a population median basis) an EC50 of at least about 0.50 µg/mL (for example, at least about 0.65 µg/mL), or greater for an RSV A strain and an EC50 of at least about 0.3 µg/mL (for example, at least about 0.35 µg/mL), or greater for an RSV B strain. Favorably, the neutralizing antibody titre remains above the stated threshold for several weeks to months following birth.

Toxin-neutralizing effector function of antibodies specific for pertussis toxin can also be measured if desired, e.g., in a Chinese Hamster Ovary (CHO) cell neutralization assay, for example as described in Gillenius et al., "The standardization of an assay for pertussis toxin and antitoxin in microplate culture of Chinese hamster ovary cells". J. Biol. Stand. (1985) 13:61-66, incorporated by reference. However, neutralizing activity in this assay is less well correlated with protection.

Optionally, according to the vaccination regimens, methods, uses and kits disclosed herein, in order to extend protection against RSV and *B. pertussis* beyond the early months of life during which the passively transferred maternal antibodies provide protection, the infant can be actively immunized to elicit an adaptive immune response specific for RSV and/or *B*. *pertussis.* Such active immunization of the infant can be accomplished by administering one or more than one composition that contains an RSV antigen and/or a *B. pertussis* antigen. For example, the (one or more) composition(s) can comprise an RSV F protein analog, optionally formulated with an adjuvant to enhance the immune response elicited by the antigen. For administration to an infant that has not been previously exposed to RSV, the F protein analog can be formulated with an adjuvant that elicits immune response that is characterized by the production of T cells that exhibit a Th1 cytokine profile (or that is characterized by a balance of T cells that exhibit Th1 and Th2 cytokine profiles). Analogously, the infant can be actively immunized with a *B. pertussis* vaccine, which optionally may be administered as a combination vaccine also conferring protection against other pathogens.

Alternatively, rather than administering an RSV F protein analog or other protein subunit vaccine to the infant, the composition that elicits an adaptive immune response to protect against RSV can include a live attenuated virus vaccine, or a nucleic acid that encodes one or more RSV antigens (such as an F antigen, a G antigen, an N antigen, or a M2 antigen, or portions thereof). For example, the nucleic acid may be in a vector, such as a recombinant viral vector, for example, an adenovirus vector, an adeno-associated virus vector, an MVA vector, a measles vector, or the like. Exemplary viral vectors are disclosed in WO2012/089231, which is incorporated herein for the purpose of illustrating immunogenic compositions that contain a viral vector that encodes one or more RSV antigens. Alternatively, the nucleic acid can be a self replicating nucleic acid, such as a self-replicating RNA, e.g., in the form of a viral replicon, such as an alphavirus replicon (e.g., in the form of a virus replicon particle packaged with virus structural proteins). Examples of such self-replicating RNA replicons are described in WO2012/103361, which is incorporated herein for the purpose of disclosing RNA replicons that encode RSV proteins and their formulation as immunogenic compositions.

Additionally or alternatively, one or more composition(s) that contain a *B. pertussis* antigen can be administered to the infant. For example, the composition can include an acellular pertussis antigen selected from the group consisting of: pertussis toxoid (PT), filamentous haemagglutinin (FHA), pertactin (PRN), fimbrae type 2 (FIM2), fimbrae type 3 (FIM3) and BrkA, or a combination thereof (e.g., PT and FHA; PT, FHA and PRN; or PT, FHA, PRN and either or both of FIM2 and FIM3), for example where the PT is chemically or is genetically toxoided as described herein. Alternatively, the composition can include a whole cell pertussis antigen as described herein.

In the context of the vaccination regimens, methods and uses disclosed herein, the RSV antigenic component (e.g., recombinant protein, such as a F protein analog) and the *B. pertussis* antigenic component may be co-formulated into a single (i.e. combination) immunogenic composition, as disclosed herein. Alternatively, the RSV antigenic component and *B. pertussis* antigenic component are formulated in two (or more) different immunogenic compositions, which can be administered at the same or different times, e.g., according to the various approved and recommended pediatric immunization schedules, and which may be presented in kits (as disclosed herein).

When a composition(s) that elicits an adaptive RSV immune response and/or an adaptive *B. pertussis* immune response is administered to an infant born to a mother that received RSV and *B. pertussis* immunization as disclosed herein during pregnancy, the composition can be administered one or more times. The first administration can be at or near the time of birth (e.g., on the day of or the day following birth), or within 1 week of birth or within about 2 weeks of birth. Alternatively, the first administration can be at about 4 weeks after birth, about 6 weeks after birth, about 2 months after birth, about 3 months after birth, about 4 months after birth, or later, such as about 6 months after birth, about 9 months after birth, or about 12 months after birth. For example, in the case of a composition containing a *B. pertussis* antigen (e.g., Pa or Pw), it is common to administer the vaccine at about 2, 4 and 6 months after birth (followed by additional doses at 12-18 months and optionally, between 4-7 yrs of age). Thus, in an embodiment, this disclosure provides methods for protecting an infant from disease caused by RSV and *B. pertussis*, by administering one or more compositions that elicits an immune response specific for RSV and/or *B. pertussis* to an infant born to a female to whom an immunogenic composition(s) comprising an F protein analog and a *B. pertussis* antigen was administered during the time that she was pregnant with the infant. Favourably, the maternally-derived RSV- and *B. pertussis-*specific antibodies do not mediate inhibition or "blunting" of the infant's immune response to the respective antigens in such infant-administered compositions.

As mentioned above, the immunogenic compositions for use in the disclosed vaccination regimens, methods and uses may be RSV-*B*. *pertussis* combination (coformulated) compositions as described herein, or may be different compositions which separately provide an F protein analog and a *B. pertussis* antigen. Such 'separate' compositions may be provided as kits. They may be administered on the same day (co-administered) or on different days.

In the disclosed vaccination regimens, methods and uses, the infant may be immunologically immature. The infant may be less than six months of age, such as less than two months of age, for example less than one month of age, for example a newborn.

The at least one immunogenic composition administered to a pregnant female in the context of the disclosed vaccination regimens, methods and uses may, in an embodiment, be administered at 26 weeks of gestation or later, such as between 26 and 38 weeks of gestation, for example between 28 and 34 weeks of gestation.

In an embodiment of such vaccination regimens, methods and uses, the at least one subset of RSV-specific antibodies is detectable at a level at or greater than 30µg/mL in the infant's serum at birth and/or the at least one subset of pertussis-specific antibodies is detectable at a level at or greater than 10 ELISA Units/ml (EU) in the infant's serum at birth.

In certain embodiments, such vaccination regimens, methods and uses further comprise administering to the infant at least one composition that primes or induces an active immune response against RSV and/or *B. pertussis* in the infant. Where an active immune response is primed or induced against both RSV and *B. pertussis*, the at least one composition that primes or induces an active immune response against RSV and the at least one composition that primes or induces an active immune response against *B. pertussis* may be the same composition or alternatively may be different compositions. In the latter case, the different compositions may be administered on the same or different days. Favourably, the active immune response primed or induced in the infant by said at least one immunogenic composition is not quantitatively different, to a clinically meaningful extent, from the active immune response generated in response to the same composition(s) in infants of mothers who had not been immunized during pregnancy according to the disclosed vaccine regimens, methods and uses.

In such embodiments of the disclosed vaccination regimens, methods and uses wherein at least one composition that primes or induces an active immune response against RSV and/or *B. pertussis* in the infant is administered to the infant, the at least one composition administered to the infant may comprise a nucleic acid, a recombinant viral vector or a viral replicon particle, which nucleic acid, recombinant viral vector or viral replicon particle encodes at least one RSV protein antigen or antigen analog. Said at least one composition may comprise an RSV antigen comprising an F protein analog.

Kits are disclosed herein, which comprise a plurality of immunogenic compositions formulated for administration to a pregnant female, wherein the kit comprises:
(a) a first immunogenic composition comprising an F protein analog capable of inducing, eliciting or boosting a humoral immune response specific for RSV; and
(b) a second immunogenic composition comprising at least one *B. pertussis* antigen capable of inducing, eliciting or boosting a humoral response specific for *B. pertussis*,
wherein upon administration to a pregnant female, the first and second immunogenic compositions induce, elicit or boost at least one subset of RSV-specific antibodies and at least one subset of *B. pertussis-*specific antibodies, which antibodies are transferred via the placenta to a gestating infant of the pregnant female, thereby protecting the infant against infection or disease caused by RSV and *B. pertussis.* Preferably the respective compositions of the kit are administered to a pregnant female only once per gestation. Put another way, during one episode of pregnancy the pregnant female preferably is administered only a single dose of each of the kit compositions.

In such a kit, the F protein analog of the first immunogenic composition and/or the at least one *B. pertussis* antigen of the second immunogenic composition may be as described herein, including in disclosures made in the context of describing the disclosed combination immunogenic compositions. In one embodiment, the first immunogenic composition and/or the second immunogenic composition are in at least one pre-filled syringe. Such a syringe may be a dual-chamber syringe.
The following numbered paragraphs further define the present disclosure:
1. A combination immunogenic composition comprising at least one Respiratory Syncytial Virus (RSV) antigen and at least one *Bordetella pertussis* antigen, wherein said at least one RSV antigen is a recombinant soluble F protein analog and the at least one *B. pertussis* antigen comprises at least one acellular pertussis (Pa) antigen or comprises a whole cell (Pw) antigen.
2. The combination immunogenic composition of paragraph 1, wherein said F protein analog is a PreF antigen that comprises at least one modification that stabilizes the prefusion conformation of the F protein.
3. The combination immunogenic composition of paragraph 1 or 2, wherein the F protein analog comprises in an N-terminal to C-terminal direction: an F₂ domain and an F₁ domain of an RSV F protein polypeptide, and a heterologous trimerization domain, wherein there is no furin cleavage site between the F₂ domain and the F₁ domain.
4. The combination immunogenic composition of any one of paragraphs 1 to 3, wherein the F protein analog comprises at least one modification selected from:
   (i) a modification that alters glycosylation;
   (ii) a modification that eliminates at least one non-furin cleavage site;
   (iii) a modification that deletes one or more amino acids of the pep27 domain; and
   (iv) a modification that substitutes or adds a hydrophilic amino acid in a hydrophobic domain of the F protein extracellular domain.
5. The combination immunogenic composition of paragraph 3 or 4, wherein the F₂ domain comprises an RSV F protein polypeptide corresponding to amino acids 26-105 and/or wherein the F₁ domain comprises an RSV F protein polypeptide corresponding to amino acids 137-516 of the reference F protein precursor polypeptide (F₀) of SEQ ID NO:2.
6. The combination immunogenic composition of paragraph 1, wherein the F protein analog is selected from the group of:
   i. a polypeptide comprising a polypeptide selected from the group of SEQ ID NO:6, SEQ ID NO:8, SEQ ID NO:10, SEQ ID NO:18, SEQ ID NO:20 and SEQ ID NO:22;
   ii. a polypeptide encoded by a polynucleotide selected from the group of SEQ ID NO:5, SEQ ID NO:7, SEQ ID NO:9, SEQ ID NO:17, SEQ ID NO:19 and SEQ ID NO:21, or by a polynucleotide sequence that hybridizes under stringent conditions over substantially its entire length to a polynucleotide selected from the group of SEQ ID NO:5, SEQ ID NO:7, SEQ ID NO:9, SEQ ID NO:17, SEQ ID NO:19 and SEQ ID NO:21, which polypeptide comprises an amino acid sequence corresponding at least in part to a naturally occurring RSV strain;
   iii. a polypeptide with at least 95% sequence identity to a polypeptide selected from the group of SEQ ID NO:6, SEQ ID NO:8, SEQ ID NO:10, SEQ ID NO:18, SEQ ID NO:20 and SEQ ID NO:22, which polypeptide comprises an amino acid sequence that does not correspond to a naturally occurring RSV strain.
7. The combination immunogenic composition of paragraph 1, wherein said F protein analog comprises an F₂ domain and an F₁ domain of an RSV F protein polypeptide, wherein the F protein polypeptide comprises at least one modification that alters glycosylation.
8. The combination immunogenic composition of paragraph 7, wherein said F protein analog comprises at least one modification selected from:
   (i) an addition of an amino acid sequence comprising a heterologous trimerization domain;
   (ii) a deletion of at least one furin cleavage site;
   (iii) a deletion of at least one non-furin cleavage site;
   (iv) a deletion of one or more amino acids of the pep27 domain; and
   (v) at least one substitution or addition of a hydrophilic amino acid in a hydrophobic domain of the F protein extracellular domain.
9. The combination immunogenic composition of paragraph 7 or 8, wherein the at least one modification that alters glycosylation comprises a substitution of one or more amino acids comprising and/or adjacent to the amino acid corresponding position 500 of SEQ ID NO:2.
10. The combination immunogenic composition of any one of paragraphs 7-9, wherein amino acids corresponding to positions 500-502 of SEQ ID NO:2 are selected from: NGS; NKS; NGT; NKT.
11. The combination immunogenic composition of any one of paragraphs 7-10, wherein the modification that alters glycosylation comprises a substitution of glutamine at the amino acid corresponding to position 500 of SEQ ID NO:2.
12. The combination immunogenic composition of any one of paragraphs 7-11, wherein the F protein analog comprises an intact fusion peptide between the F₂ domain and the F₁ domain.
13. The combination immunogenic composition of any one of paragraphs 7-12, wherein the at least one modification comprises the addition of an amino acid sequence comprising a heterologous trimerization domain.
14. The combination immunogenic composition of paragraph 13, wherein the heterologous trimerization domain is positioned C-terminal to the F₁ domain.
15. The combination immunogenic composition of any one of paragraphs 7-14, comprising an F₂ domain and an F₁ domain with no intervening furin cleavage site.
16. The combination immunogenic composition of any one of paragraphs 7-15, wherein the F protein analog assembles into a multimer, such as a trimer.
17. The combination immunogenic composition of any one of paragraphs 7-16, wherein the F₂ domain comprises at least a portion of an RSV F protein polypeptide corresponding to amino acids 26-105 of the reference F protein precursor polypeptide (F₀) of SEQ ID NO:2.
18. The combination immunogenic composition of any one of paragraphs 7-17, wherein the F₁ domain comprises at least a portion of an RSV F protein polypeptide corresponding to amino acids 137-516 of the reference F protein precursor polypeptide (F₀) of SEQ ID NO:2.
19. The combination immunogenic composition of any one of paragraphs 7-18, wherein the F₂ domain comprises an RSV F protein polypeptide corresponding to amino acids 26-105 and/or wherein the F₁ domain comprises an RSV F protein polypeptide corresponding to amino acids 137-516 of the reference F protein precursor polypeptide (F₀) of SEQ ID NO:2.
20. The combination immunogenic composition of any one of paragraphs 7-19, wherein the F protein analog is selected from the group of:
   i. a polypeptide comprising SEQ ID NO:22;
   ii. a polypeptide encoded by SEQ ID NO:21 or by a polynucleotide sequence that hybridizes under stringent conditions over substantially its entire length to SEQ ID NO:21;
   iii. a polypeptide with at least 95% sequence identity to SEQ ID NO:22.
21. The combination immunogenic composition of any one of paragraphs 7-20, wherein the F₂ domain comprises amino acids 1-105 of the RSV F protein polypeptide.
22. The combination immunogenic composition of any one of paragraphs 7-21, wherein the F₂ domain and the F₁ domain are positioned with an intact fusion peptide and without an intervening pep27 domain.
23. The combination immunogenic composition of any one of paragraphs 8-22, wherein the heterologous trimerization domain comprises a coiled-coil domain or comprises an isoleucine zipper.
24. The combination immunogenic composition of paragraph 23, wherein the isoleucine zipper domain comprises the amino acid sequence of SEQ ID NO:11.
25. The combination immunogenic composition of any one of paragraphs 7-24, wherein the F protein analog comprises at least one substitution or addition of a hydrophilic amino acid in a hydrophobic domain of the F protein extracellular domain.
26. The combination immunogenic composition of paragraph 25, wherein the hydrophobic domain is the HRB coiled-coil domain of the F protein extracellular domain.
27. The combination immunogenic composition of paragraph 26, wherein the HRB coiled-coil domain comprises the substitution of a charged residue in place of a neutral residue at the position corresponding to amino acid 512 of the reference F protein precursor (F₀) of SEQ ID NO:2.
28. The combination immunogenic composition of paragraph 27, wherein the HRB coiled-coil domain comprises a substitution of lysine or glutamine for leucine at the position corresponding to amino acid 512 of the reference F protein precursor (F₀) of SEQ ID NO:2.
29. The combination immunogenic composition of paragraph 25, wherein the hydrophobic domain is the HRA domain of the F protein extracellular domain.
30. The combination immunogenic composition of paragraph 29, wherein the HRA domain comprises the addition of a charged residue following the position corresponding to amino acid 105 of the reference F protein precursor (F₀) of SEQ ID NO:2.
31. The combination immunogenic composition of paragraph 30, wherein the HRA domain comprises the addition of a lysine following the position corresponding to amino acid 105 of the reference F protein precursor (F0) of SEQ ID NO:2.
32. The combination immunogenic composition of any one of paragraphs 25-31, wherein the F protein analog comprises at least a first substitution or addition of a hydrophilic amino acid in the HRA domain and at least a second substitution or addition of a hydrophilic amino acid in the HRB domain of the F protein extracellular domain.
33. The combination immunogenic composition of any one of paragraphs 7-32, wherein the F protein analog comprises at least one amino acid addition, deletion or substitution that eliminates a furin cleavage site present in a naturally occurring F protein precursor (F₀).
34. The combination immunogenic composition of paragraph 33, wherein the F protein analog comprises an amino acid addition, deletion or substitution that eliminates a furin cleavage site at a position corresponding to amino acids 105-109, a position corresponding to amino acids 133-136, or at both positions corresponding to amino acids 105-109 and 133-136 of the reference F protein precursor (F₀) of SEQ ID NO:2.
35. The combination immunogenic composition of any one of paragraphs 7-34, wherein the F₁ and F₂ polypeptide domains correspond in sequence to the RSV A Long strain.
36. The combination immunogenic composition of any one of paragraphs 7-35, wherein the at least one RSV antigen comprises a multimer, such as a trimer, of polypeptides.
37. The combination immunogenic composition of any one of paragraphs 1 to 36, wherein said at least one Pa antigen is selected from the group consisting of: pertussis toxoid (PT), filamentous haemagglutinin (FHA), pertactin (PRN), fimbrae type 2 (FIM2), fimbrae type 3 (FIM3) and BrkA.
38. The combination immunogenic composition of paragraph 37, wherein the PT is chemically toxoided, or is genetically toxoided for example by one or both of the mutations: R9K and E129G.
39. The combination immunogenic composition of paragraph 37 or 38, wherein said at least one Pa antigen comprises: PT and FHA; PT, FHA and PRN; or PT, FHA, PRN and either or both of FIM2 and FIM3.
40. The combination immunogenic composition of any one of paragraphs 37 to 39, comprising:
   i. 10-30µg, for example exactly or approximately 25ug of PT;
   ii. 10-30µg, for example exactly or approximately 25µg of FHA.
41. The combination immunogenic composition of paragraph 40, further comprising:
   2-10µg, for example exactly or approximately 8µg of PRN.
42. The combination immunogenic composition of any one of paragraphs 37 to 39, comprising:
   i. 10-30µg, for example exactly or approximately 20µg of PT;
   ii. 10-30µg, for example exactly or approximately 20µg of FHA;
   iii. 2-10µg, for example exactly or approximately 3µg of PRN; and
   iv. 1-10µg, for example exactly or approximately 5µg total of FIM2 and FIM3.
43. The combination immunogenic composition of any one of paragraphs 37 to 39, comprising:
   i. 2-10µg, for example exactly or approximately 8µg of PT;
   ii. 2-10µg, for example exactly or approximately 8µg of FHA; and
   iii. 0.5-4µg, for example exactly or approximately 25µg of PRN.
44. The combination immunogenic composition of any one of paragraphs 37 to 39, comprising
   i. 2-10µg, for example exactly or approximately 25µg of PT;
   ii. 2-10µg, for example exactly or approximately 5µg of FHA;
   iii. 0.5-4µg, for example exactly or approximately 3µg of PRN; and
   iv. 1-10µg, for example exactly or approximately 5µg total of FIM2 and FIM3.
45. The combination immunogenic composition of any one of paragraphs 37 to 39, comprising
   i. 2-5 µg, for example exactly or approximately 3.2µg of PT;
   ii. 25-40µg, for example exactly or approximately 34.4µg of FHA;
   iii. 0.5-3µg, for example exactly or approximately 1.6µg of PRN; and
   iv. 0.5-1µg, for example exactly or approximately 0.8µg of FIM2.
46. The combination immunogenic composition of any one of paragraphs 37 to 39, comprising:
   i. 2-10µg, for example exactly or approximately 8µg of PT;
   ii. 1-4µg, for example exactly or approximately 25µg of FHA; and
   iii. 1-4µg, for example exactly or approximately 25µg of PRN.
47. The combination immunogenic composition of any one of paragraphs 1 to 36, wherein said at least one *B. pertussis* antigen comprises a Pw antigen.
48. The combination immunogenic composition of paragraph 47, wherein said Pw antigen has reduced endotoxin content.
49. The combination immunogenic composition of paragraph 48, wherein said reduced endotoxin content is achieved by chemical extraction of lipo-oligosaccharide (LOS), or by genetic manipulation of endotoxin production, for example to induce overexpression or heterologous expression of a 3-*O*-deacylase.
50. The combination immunogenic composition of any one of paragraphs 47 to 49, wherein said Pw antigen comprises *B. pertussis* cells comprising at least partially 3-*O-*deacylated LOS.
51. The combination immunogenic composition of any one of paragraphs 1 to 50, further comprising a pharmaceutically acceptable carrier or excipient.
52. The combination immunogenic composition of paragraph 51, wherein the carrier or excipient comprises a buffer.
53. The combination immunogenic composition of any one of paragraphs 1 to 52, further comprising at least one adjuvant.
54. The combination immunogenic composition of paragraph 53, wherein the at least one adjuvant comprises at least one adjuvant selected from the group of: an aluminium salt such as aluminium hydroxide or aluminium phosphate; calcium phosphate; 3D-MPL; QS21; a CpG-containing oligodeoxynucleotide adjuvant; and an oil-in-water emulsion.
55. The combination immunogenic composition of paragraph 53 or 54, wherein the at least one adjuvant comprises aluminium hydroxide.
56. The combination immunogenic composition of any one of paragraphs 53 or 54, wherein the at least one adjuvant comprises an oil-in-water emulsion.
57. The combination immunogenic composition of paragraph 56, wherein the oil-in-water emulsion comprises less than 5mg squalene per human dose.
58. The combination immunogenic composition of paragraph 56 or 57, wherein the oil-in-water emulsion comprises a tocol.
59. The combination immunogenic composition of paragraph 53, wherein said adjuvant is suitable for administration to a neonate or pregnant human.
60. The combination immunogenic composition of any one of paragraphs 1 to 52, wherein the immunogenic composition does not comprise an adjuvant.
61. The combination immunogenic composition of any one of paragraphs 1 to 60, further comprising at least one antigen from a pathogenic organism other than RSV and *B. pertussis.*
62. The combination immunogenic composition of paragraph 61, comprising one or more antigens selected from the group consisting of: diphtheria toxoid (D); tetanus toxoid (T); Hepatitis B surface antigen (HBsAg); inactivated polio virus (IPV); capsular saccharide of *H. influenzae* type b (Hib) conjugated to a carrier protein; capsular saccharide of *N. meningitidis* type C conjugated to a carrier protein; capsular saccharide of *N. meningitidis* type Y conjugated to a carrier protein; capsular saccharide of *N. meningitidis* type A conjugated to a carrier protein; capsular saccharide of *N. meningitidis* type W conjugated to a carrier protein; and an antigen from *N. meningitidis* type B.
63. The combination immunogenic composition of paragraph 62, comprising D and T; D, T and IPV; D, T and HBsAg; D, T and Hib; D, T, IPV and HBsAg; D, T, IPV and Hib; D, T, HBsAg and Hib; or D, T, IPV, HBsAg and Hib.
64. The combination immunogenic composition of paragraph 62 or 63 comprising, in addition to the at least one RSV antigen:
   i. 20-30µg, for example exactly or approximately 25µg of PT;
   ii. 20-30µg, for example exactly or approximately 25µg of FHA;
   iii. 1-10µg, for example exactly or approximately 3 or 8µg of PRN;
   iv. 10-30Lf, for example exactly or approximately 15 or 25Lf of D; and
   v. 1-15Lf, for example exactly or approximately 5 or 10Lf of T.
65. The combination immunogenic composition of paragraph 62 or 63, comprising, in addition to the at least one RSV antigen:
   i. 2-10µg, for example exactly or approximately 2.5 or 8µg of PT;
   ii. 2-10µg, for example exactly or approximately 5 or 8µg of FHA;
   iii. 0.5-4µg, or example 2-3µg such as exactly or approximately 2.5 or 3µg of PRN;
   iv. 1-10Lf, for example exactly or approximately 2 or 2.5 or 9Lf of D; and
   v. 1-15Lf, for example exactly or approximately 5 or 10Lf of T.
66. The combination immunogenic composition of paragraphs 40 to 46, 64 or 65, wherein the at least one RSV antigen comprises a PreF antigen that comprises at least one modification that stabilizes the prefusion conformation of the F protein.
67. The combination immunogenic composition of paragraph 66, further comprising no adjuvant, or comprising a mineral salt adjuvant.
68. A method for eliciting an immune response against RSV and *B. pertussis,* comprising administering to a subject the combination immunogenic composition of any one of paragraphs 1 to 67.
69. The method of paragraph 68, wherein administering said composition elicits an immune response specific for RSV without enhancing viral disease following contact with RSV.
70. The method of paragraphs 68 or 69, wherein said elicited immune response is a booster response.
71. The method of any one of paragraphs 69 to 70, wherein the immune response against RSV and *B. pertussis* comprises a protective immune response that reduces or prevents incidence, or reduces severity, of infection with RSV and *B. pertussis* and/or reduces or prevents incidence, or reduces severity, of a pathological response following infection with RSV and *B. pertussis.*
72. The combination immunogenic composition of any one of paragraphs 1 to 67 for use in medicine.
73. The combination immunogenic composition of any one of paragraphs 1 to 67 for the prevention or treatment in a subject of infection by, or disease associated with, RSV and *B. pertussis.*
74. The method of any one of paragraphs 68-71 or the combination immunogenic composition of paragraphs 72 or 73, wherein the combination immunogenic composition is administered, or is for administration, to a subject as a single-dose regimen.
75. The method of any one of paragraphs 68-71 or the combination immunogenic composition of paragraphs 73 or 74, wherein the subject is a mammal, such as a human, selected from the group of: a neonate; an infant; a child; an adolescent; an adult; and an elderly adult.
76. The method or the combination immunogenic composition of paragraph 75 wherein the subject is an adolescent human, wherein said subject is between 10 and 18 years of age and wherein said combination immunogenic composition is administered, or is for administration, only once.
77. The method of any one of paragraphs 68-71 or the combination immunogenic composition of paragraphs 72 or 73, wherein the subject is not a pregnant female.
78. The method of any one of paragraphs 68-72 or the combination immunogenic composition of paragraphs 72 or 73, wherein the subject is a, optionally human, pregnant female with a gestational infant.
79. The method or the combination immunogenic composition of paragraph 78, wherein said combination immunogenic composition is administered, or is for administration, to said pregnant female only once per gestation.
80. A vaccination regimen for protecting an infant against infection or disease caused by RSV and *B. pertussis*, the vaccination regimen comprising:
   administering to a pregnant female with a gestational infant at least one immunogenic composition capable of boosting a humoral immune response specific for both RSV and *B. pertussis*, which at least one immunogenic composition comprises a recombinant RSV antigen comprising an F protein analog and at least one *B. pertussis* antigen,
   wherein at least one subset of RSV-specific antibodies and at least one subset of *B. pertussis-*specific antibodies elicited or increased in the pregnant female by the at least one immunogenic composition are transferred via the placenta to the gestational infant, thereby protecting the infant against infection or disease caused by RSV and *B. pertussis.*
81. A method for protecting an infant against infection or disease caused by RSV and *B. pertussis*, the method comprising:
   administering to a pregnant female with a gestational infant at least one immunogenic composition capable of boosting a humoral immune response specific for both RSV and *B. pertussis*, which at least one immunogenic composition comprises a recombinant RSV antigen comprising an F protein analog and at least one *B. pertussis* antigen,
   wherein at least one subset of RSV-specific antibodies and at least one subset of *B. pertussis-*specific antibodies elicited or increased in the pregnant female by the at least one immunogenic composition are transferred via the placenta to the gestational infant, thereby protecting the infant against infection or disease caused by RSV and *B. pertussis.*
82. An immunogenic composition or plurality of immunogenic compositions comprising a recombinant RSV antigen comprising an F protein analog and at least one pertussis antigen for use in protecting an infant against infection or disease caused by RSV and *B. pertussis*, wherein the immunogenic composition(s) is/are formulated for administration to a pregnant female and wherein the immunogenic composition(s) is/are capable of boosting a humoral immune response specific for both RSV and *B. pertussis*, and wherein at least one subset of RSV-specific antibodies and at least one subset of *B. pertussis-*specific antibodies boosted in the pregnant female by the immunogenic composition(s) are transferred via the placenta to the gestational infant, thereby protecting the infant against infection or disease caused by RSV and *B. pertussis.*
83. The vaccination regimen, method or use of any one of paragraphs 80 to 82, wherein the recombinant RSV antigen comprising an F protein analog and at least one *B. pertussis* antigen are coformulated in the same immunogenic composition, being a combination immunogenic composition as defined in any one of paragraphs 1 to 67.
84. The vaccination regimen, method or use of any one of paragraphs 80 to 83, wherein said immunogenic composition is administered, or is for administration, to said pregnant female only once per gestation.
85. The vaccination regimen, method or use of any one of paragraphs 80 to 82, wherein the recombinant RSV antigen comprising an F protein analog and at least one *B. pertussis* antigen are formulated in two different immunogenic compositions.
86. The vaccination regimen, method or use of paragraph 85, wherein the two different immunogenic compositions are administered on the same day (co-administered).
87. The vaccination regimen, method or use of paragraph 85, wherein the two different immunogenic compositions are administered on different days.
88. The vaccination regimen, method or use of any one of paragraphs 85 to 87, wherein the F protein analog is as defined in any one of paragraphs 1 to 36 and the at least one *B. pertussis* antigen is as defined in any one of paragraphs 37 to 50.
89. The vaccination regimen, method or use of any one of paragraphs 80 to 88, wherein said pregnant female is a human.
90. The vaccination regimen, method or use of any one of paragraphs 80 to 89, wherein the infant is immunologically immature.
91. The vaccination regimen, method or use of any one of paragraphs 80 to 90, wherein the infant is less than six months of age.
92. The vaccination regimen, method or use of any one of paragraphs 80 to 91, wherein the infant is less than two months of age, for example less than one month of age, for example a newborn.
93. The vaccination regimen, method or use of any one of paragraphs 80 to 92, wherein the at least one subset of RSV-specific antibodies and/or pertussis-specific antibodies transferred via the placenta comprises IgG antibodies, preferably IgG₁ antibodies.
94. The vaccination regimen, method or use of any one of paragraphs 80 to 93, wherein the at least one subset of RSV-specific antibodies transferred via the placenta are neutralizing antibodies.
95. The vaccination regimen, method or use of any one of paragraphs 80 to 94, wherein the at least one subset of RSV-specific antibodies is detectable at a level at or greater than 30µg/mL in the infant's serum at birth.
96. The vaccination regimen, method or use of any one of paragraphs 80 to 95, wherein the at least one subset of pertussis-specific antibodies is detectable at a level at or greater than 10 ELISA Units/ml (EU) in the infant's serum at birth.
97. The vaccination regimen, method or use of any one of paragraphs 80 to 96, further comprising administering to the infant at least one composition that primes or induces an active immune response against RSV in the infant.
98. The vaccination regimen, method or use of any one of paragraphs 80 to 97, further comprising administering to the infant at least one composition that primes or induces an active immune response against *B. pertussis* in the infant.
99. The vaccination regimen, method or use of paragraph 97 or 98, comprising administering to the infant at least one composition that primes or induces an active immune response against RSV and at least one composition that primes or induces an active immune response against *B. pertussis.*
100. The vaccination regimen, method or use of paragraph 99, wherein the at least one composition that primes or induces an active immune response against RSV and the at least one composition that primes or induces an active immune response against *B. pertussis* are the same composition.
101. The vaccination regimen, method or use of paragraph 99, wherein the at least one composition that primes or induces an active immune response against RSV and the at least one composition that primes or induces an active immune response against *B. pertussis* are different compositions.
102. The vaccination regimen, method or use of paragraph 101, wherein the different compositions are administered on the same or different days.
103. The vaccination regimen, method or use of paragraph 97 to 101, wherein the at least one composition administered to the infant comprises an RSV antigen comprising an F protein analog.
104. The vaccination regimen, method or use of any one of paragraphs 97 to 102, wherein the at least one composition administered to the infant comprises a nucleic acid, a recombinant viral vector or a viral replicon particle, which nucleic acid, recombinant viral vector or viral replicon particle encodes at least one RSV protein antigen or antigen analog.
105. The vaccination regimen, method or use of any one of paragraphs 80 to 104, wherein the at least one immunogenic composition is administered to a pregnant female at 26 weeks of gestation or later.
106. The vaccination regimen, method or use of any one of paragraphs 80 to 105, wherein the pregnant female is between 26 and 38 weeks of gestation, for example between 28 and 34 weeks of gestation.
107. A kit comprising a plurality of immunogenic compositions formulated for administration to a pregnant female, wherein the kit comprises:
   (a) a first immunogenic composition comprising an F protein analog capable of inducing, eliciting or boosting a humoral immune response specific for RSV; and
   (b) a second immunogenic composition comprising at least one *B. pertussis* antigen capable of inducing, eliciting or boosting a humoral response specific for *B. pertussis*,
   wherein upon administration to a pregnant female, the first and second immunogenic compositions induce, elicit or boost at least one subset of RSV-specific antibodies and at least one subset of *B. pertussis-*specific antibodies, which antibodies are transferred via the placenta to a gestating infant of the pregnant female, thereby protecting the infant against infection or disease caused by RSV and *B. pertussis.*
108. The kit of paragraph 107, wherein the F protein analog of the first immunogenic composition is as defined in any one of paragraphs 1 to 36.
109. The kit of paragraph 107 or 108, wherein the at least one *B. pertussis* antigen of the second immunogenic composition is as defined in any one of paragraphs 37 to 50.
110. The kit of any one of paragraphs 107 to 109, wherein the relevant features of the kit are as defined for the vaccine regimen, method or use of any one of paragraphs 83 to 101.
111. The kit of any one of paragraphs 107 to 110, wherein the first immunogenic composition and/or the second immunogenic composition are in at least one pre-filled syringe.
112. The kit of paragraph 111, wherein the pre-filled syringe is dual-chamber syringe.
113. The kit of any one of paragraphs 107 to 112, wherein the respective compositions of the kit are for administration to said pregnant female only once per gestation.

The following examples are provided to illustrate certain particular features and/or embodiments. These examples should not be construed to limit the invention to the particular features or embodiments described.

### EXAMPLES

Throughout the Examples the RSV vaccine (Pre-F) used comprised a glycosylation-modified PreF antigen of the type corresponding to SEQ ID NO: 22, i.e. containing the modification L112Q and a modification of the amino acids corresponding to positions 500-502 of SEQ ID NO:2 selected from: NGS; NKS; NGT; and NKT.

### Example 1 - Proof of concept: RSV maternal immunization in a guinea pig model

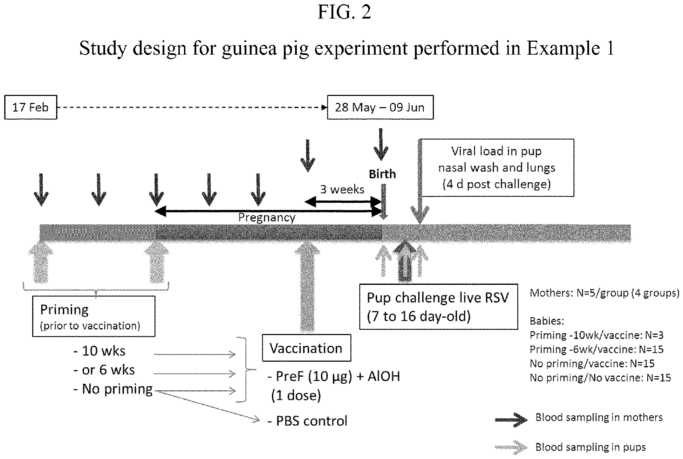

The guinea pig model was selected as placental structure and IgG transfer is closer to that of humans than is the case for typical rodent models (reviewed in Pentsuk and van der Laan (2009) Birth Defects Research (part B) 86:328-344). The relatively long gestational period of the guinea pig (68 days) allows for immunization and immune response development during pregnancy. In order to mimic the RSV immune status of pregnant women who have been exposed to RSV throughout their lives and have a pre-existing immune response to RSV, female guinea pigs were primed with live RSV at either 6 weeks or 10 weeks prior to vaccination (FIG.2).

Female guinea pigs (N=5/group) were primed intranasally with live RSV virus (2.5 x 10⁵ pfu), 6 or 10 weeks prior to vaccination (approximately at the time of mating or 4 weeks prior to mating). Two groups were left unprimed. Pregnant females were immunized approximately 6 weeks after the start of gestation with 10 µg of PreF antigen combined with aluminum hydroxide. One unprimed group of females was injected with PBS. Serum samples were collected throughout the priming and gestation period to monitor levels of anti-RSV binding and neutralizing antibodies.

Offspring (7- to 16-day old) were challenged intranasally with live RSV at 1 x 10⁷ pfu. Four days after challenge, lungs were collected and separated into 7 lobes. Virus was titrated in 6 of the 7 lobes and total virus particles per gram of lung were calculated.

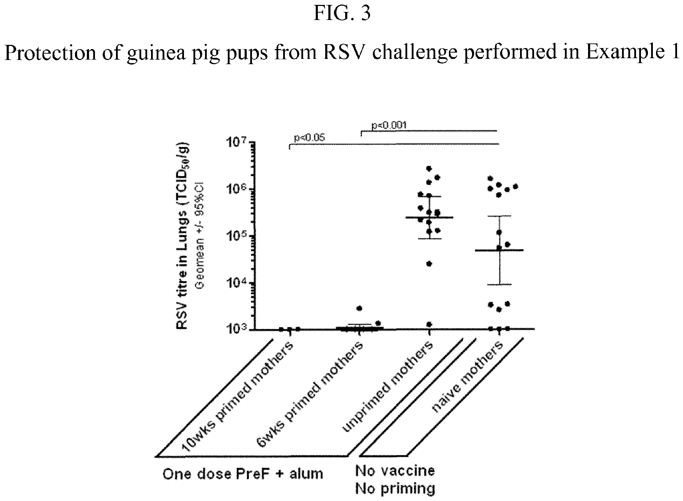

Results are shown in the graph in FIGS. 3 and 4.

Similar levels of antibodies were observed on the day of vaccination (D70-75 - before vaccination) whether guinea pigs had been primed 6 or 10 weeks earlier. Plateau titres were reached as of 14 days post priming. Neutralising antibody titres do not decline after reaching a plateau for at least about 60 days. Thus priming at both time points was equivalent in this model and suitable to mimic maternal infection in humans.

Results from lung viral load in the guinea pig offspring (FIG. 3) indicate that offspring born to primed and vaccinated mothers were protected from RSV challenge, as compared to offspring born to unprimed/unvaccinated mothers. In contrast, offspring born to unprimed/vaccinated mothers were not protected from RSV challenge. Steff et al (Proof of concept of the efficacy of a maternal RSV, recombinant F protein, vaccine for protection of offspring in the guinea pig model - poster 114, RSV Vaccines for the World conference, Porto, Portugal, 14-16 October 2013), provides further evidence of the efficacy of PreF antigen in inducing protective antibody levels in guinea pig pups after maternal immunization.

### Example 2 - Combination Vaccine protects against challenge by RSV

This example demonstrates protection against RSV elicited by a combination vaccine containing RSV (Pre-F) and *B. pertussis* antigens (PT, FHA and PRN). Immunogenicity (neutralizing antibody titers) of two doses of the combined Pa-RSV vaccine was evaluated in the Balb/c mouse model, followed by an intranasal RSV challenge to measure efficacy of the combination vaccine.

Groups of BALB/c mice (n=14/group) were immunized intra-muscularly twice at a 3-week interval with the formulations displayed in Table 1.

**Table 1: Vaccine formulations administered prior to RSV challenge**

| Vaccine | PT (µg) | FHA (µg) | PRN (µg) | PreF (µg) | Al(OH)₃(µg) | Vol (µL) | N |
|---|---|---|---|---|---|---|---|
| Pa-RSV w/ Al(OH)₃ | 6.25 | 6.25 | 2 | 2 | 50 | 50 | 14 |
| Pa-RSV | 6.25 | 6.25 | 2 | 2 | - | 50 | 14 |
| Standalone Pa | 6.25 | 6.25 | 2 | - | 50 | 50 | 14 |
| Standalone RSV | - | - | - | 2 | 50 | 50 | 14 |

Sera from all mice were individually collected on Day 0 (prior to first immunization), on Day 21 (prior to second immunization) and on Day 35 (2 weeks after second immunization) and tested for the presence of RSV neutralizing antibodies using a plaque reduction assay. Briefly, serial dilutions of each serum were incubated with RSV A Long (targeting 100 pfu/well) for 20 min at 33°C. After incubation, the virus-serum mixture was transferred to plates previously seeded with Vero cells and emptied of growth medium. On each plate, cells in one column were incubated with virus only (100% infectivity) and 2 wells received no virus or serum (cell controls). Plates were incubated for 2hrs at 33°C, medium was removed and RSV medium containing 0.5% CMC (low viscosity carboxymethylcellulose) was added to all wells. The plates were incubated for 3 days at 33°C before immunofluorescent staining.

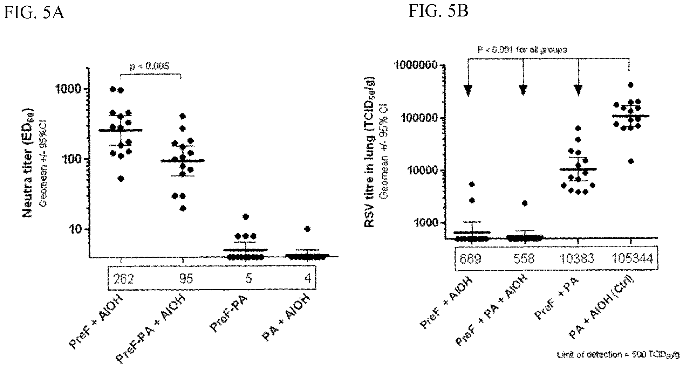

For staining, cell monolayers were washed with PBS and fixed with 1% paraformaldehyde. RSV-positive cells were detected using a commercial goat anti-RSV antiserum followed by a rabbit anti-goat IgG conjugated to FITC. The number of stained plaques per well was counted using an automated imaging system. Neutralizing antibody titer of each serum was determined as the inverse of the serum dilution causing 60% reduction in the number of plaques as compared to the control without serum (ED₆₀). Results are illustrated in FIG. 5A.

The PreF-based vaccine adjuvanted with Al(OH)₃ protects mice against an intranasal RSV challenge and this animal model is therefore useful for studying the capability of RSV vaccines to mediate viral clearance in the lungs. The combination of *B. pertussis* (PT, FHA and PRN) and RSV (PreF) antigens in a single vaccine was then tested for protective efficacy in the intranasal RSV challenge mouse model. Two weeks after the second vaccine dose, mice were challenged by instillation of 50 µl (25 µL per nostril) of live RSV A Long strain (about 1.45x10⁶ pfu/50 µl). Lungs were collected four days post challenge for evaluation of lung viral load. Four days after challenge, mice were euthanized, the lungs were aseptically harvested and individually weighed and homogenized. Serial dilutions (8 replicates each) of each lung homogenate were incubated with Vero cells and wells containing plaques were identified by immunofluorescence, 6 days after seeding. The viral titer was determined using the Spearman-Kärber method for TCID₅₀ calculation and was expressed per gram of lung. The statistical method employed is an Analysis of Variance (ANOVA 1) on the log 10 values.

Results are illustrated in FIG. 5B. As expected, 2 µg of PreF combined with Al(OH)₃ efficiently promoted viral clearance in the lungs compared to mice vaccinated with standalone Pa (control group where no protection from RSV challenge is expected). Only two animals out of 14 in the PreF group had detectable levels of RSV in the lungs, with no RSV detectable in the 12 other animals. Pa-RSV combination vaccine was equally capable of protecting mice against RSV challenge as shown by only one out of 14 animals with detectable levels of RSV in the lungs, RSV being undetectable in the remaining 13 animals. Overall, animals vaccinated with PreF + Al(OH)₃ vaccine or with Pa antigens + PreF + Al(OH)₃ had significantly lower lung viral titers than control animals vaccinated with standalone Pa (P<0.001). In the group vaccinated with Pa antigens + PreF in the absence of adjuvant, there was a significant reduction (P<0.001) in lung viral titers, however no animal in this group appeared fully protected from RSV challenge since virus was quantifiable in lungs from all animals.

Using a challenge animal model, we observed that the Pa-RSV combination vaccine elicited a protective immune response against RSV comparable to that of RSV vaccine. This immune response was associated with the production of RSV neutralizing antibodies.

### Example 3 - Combination Vaccine protects against challenge by B. pertussis

This example demonstrates protection against *Bordatella pertussis* elicited by a combination vaccine containing RSV (Pre-F) and *B. pertussis* antigens (PT, FHA and PRN). Immunogenicity (neutralizing antibody titers) of two doses of the combined Pa-RSV vaccine was evaluated in the Balb/c model, followed by an intranasal challenge with infectious *B*. *pertussis* to measure efficacy of the combination vaccine.

Groups of BALB/c mice (n=20/group) were immunized subcutaneously twice with a 3-week interval with the formulations displayed in Table 2.

**Table 2: Vaccine formulations administered prior to B. pertussis challenge**

| Vaccine | PT (µg) | FHA (µg) | PRN (µg) | PreF (µg) | Al(OH)₃(µg) | Vol (µL) | N |
|---|---|---|---|---|---|---|---|
| DTPa (1/4 HD) | 6.25 | 6.25 | 2 | | 125 | 125 | 20 |
| Standalone Pa | 6.25 | 6.25 | 2 | - | 50 | 50 | 20 |
| Pa-RSV | 6.25 | 6.25 | 2 | 2 | 50 | 50 | 20 |
| Standalone RSV | - | - | - | 2 | 50 | 50 | 20 |

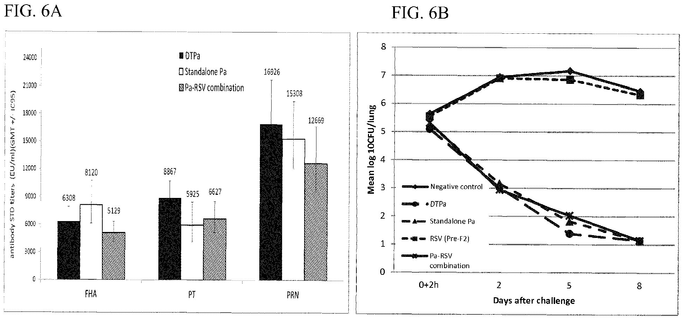

Sera from all mice were individually collected seven days after the second immunization (d28 - the day before challenge) and tested for the presence of anti-PT, -FHA and -PRN IgG antibodies. In brief, 96-well plates were coated with FHA (2 µg/ml), PT (2 µg/ml) or PRN (6 µg/ml) in a carbonate-bicarbonate buffer (50mM) and incubated overnight at 4°C. After the saturation step with the PBS-BSA 1% buffer, mouse sera were diluted at 1/100 in PBS-BSA 0.2% Tween 0.05% and serially diluted in the wells from the plates (12 dilutions, step ½). An anti-mouse IgG coupled to the peroxidase was added (1/5000 dilution). Colorimetric reaction was observed after the addition of the peroxidase substrate (OPDA), and stopped with HCL 1M before reading by spectrophotometry (wavelengths: 490-620 nm). For each serum tested and standard added on each plate, a 4-parameter logistic curve was fit to the relationship between the OD and the dilution (Softmaxpro). This allowed the derivation of each sample titer expressed in STD titers. Serological antibody responses specific to Pa antigens (PT, FHA and PRN) induced by the vaccines is considered indicative (but not dispositive) of the vaccine ability to elicit an antibody responses against the individual antigens found in the Pa vaccine. FIG. 6A shows that the DTPa, standalone Pa and Pa-RSV combination promoted PT, FHA and PRN-specific IgG responses after two immunizations. No antigen-specific antibodies were detected in sera from unvaccinated or RSV-vaccinated mice (data not presented). Statistical analysis demonstrated equivalence between the anti-PT and anti-FHA antibody responses induced by DTPa (Infanrix™) and the Pa-RSV combination. The amount of anti-PRN specific antibodies induced by the standalone Pa and Pa-RSV combination vaccines was also statistically equivalent, demonstrating that the presence of RSV antigen did not interfere with the production of anti-pertussis antibody responses.

To demonstrate protection, one week after the booster, the mice were challenged by instillation of 50 µl of bacterial suspension (about 5x10⁶ CFU/50 µl) into the left nostril. Five mice of each group were euthanized 2 hours, 2 days, 5 days and 8 days after the bacterial challenge. The lungs were aseptically harvested and individually homogenized. The lung bacterial clearance was measured by counting the colony growth on Bordet-Gengou agar plates. Data were plotted according to the mean of number of colony-forming unit (CFU - log10) per lung in each treatment group for each collection time. The statistical method employed is an Analysis of Variance (ANOVA) on the log10 values with 2 factors (treatment and day) using a heterogeneous variance model.

In this model, the acellular *B. pertussis* vaccine (Pa) protects mice against an intranasal challenge with the bacteria. This animal model is therefore useful for studying the capability of a *B. pertussis*-based vaccine to mediate bacterial clearance in the lungs. The combination of *B. pertussis* (PT, FHA and PRN) and RSV (Pre-F) antigens in a single vaccine was then tested for protective efficacy in the intranasal challenge mouse model. Representative results are illustrated in FIG. 6B. As expected, the adjusted human dose (one fourth dose of the commercial DTPa vaccine Infanrix™) efficiently promoted bacterial clearance compared to the unvaccinated mice. Both Pa standalone and Pa-RSV combination vaccines were also capable of eliciting a protective immune response leading to bacterial elimination. As expected, the standalone Pre-F RSV vaccine was unable to protect in this animal model against *B. pertussis.*

These results demonstrate in an animal model that the Pa-RSV combination vaccine elicited a protective immune response against *B. pertussis* as well as against RSV as demonstrated in Example 2 above. This immune response was associated with the production of specific antibodies against the three subunit antigens found in the acellular Pa vaccine (PT, FHA and PRN).

### Example 4 - Administration of combined Pa-RSV vaccine to pregnant dams does not interfere with protection of pups from RSV challenge in a guinea pig model

Female guinea pigs (N=5/group) were primed intranasally with live RSV virus (800 pfu). One group was left unprimed. Mating was started the day after priming. Pregnant females were immunized at 4 and 7 weeks post priming (two-dose regimen) or 7 weeks post priming (single dose regimen) with one of the following vaccines: 10 µg of PreF antigen combined with aluminum hydroxide (100 µg), 10 µg of PreF antigen + DTaP antigens (5 Lf diphtheria toxoid, 2 Lf tetanus toxoid, 5 µg FHA, 5 µg inactivated pertussis toxin, 1.6 µg PRN) combined with aluminium hydroxide (130 µg) or only with DTaP antigens (same quantities as above) combined with aluminum hydroxide (100 µg). Serum samples were collected 14 days post first or second immunization (day 63 post-priming) for females immunized once or twice, respectively. Levels of anti-RSV neutralizing antibodies were determined at this time point (1 to 6 weeks before pup birth).

Serum samples were collected from the offspring within 24 to 72 hours post birth. Pups between 5 and 18 days were challenged intranasally with live RSV at 2 x 10⁶ pfu. Four days after challenge, lungs were collected and homogenized. Virus was titrated in lung homogenates and total virus particles per gram of lung were calculated.

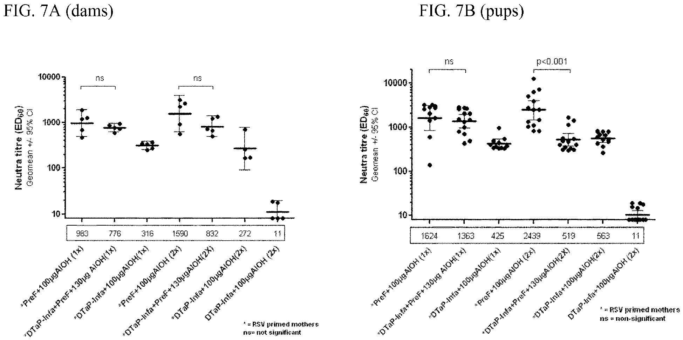

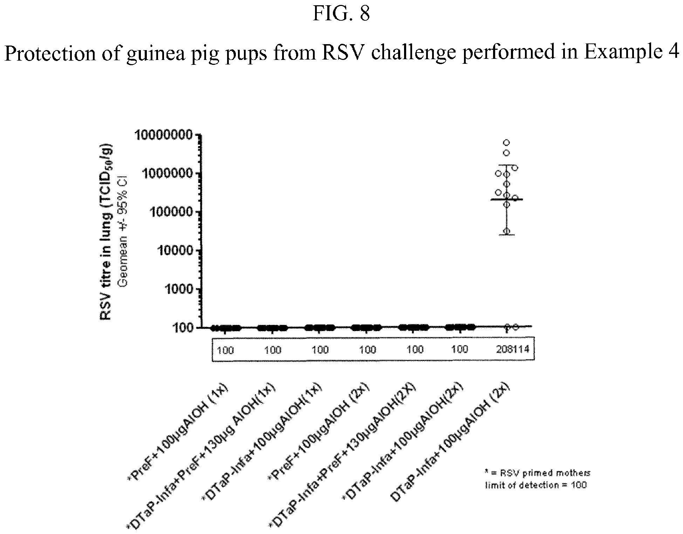

Results are shown in the graphs in FIGS. 7 and 8.

RSV neutralizing antibody titers in dams vaccinated once or twice with DTaP antigens only (groups 3 and 6 in FIG. 7A) were 316 and 272, respectively. This represents the titers induced by RSV priming since there was practically no RSV neutralizing response in unprimed dams vaccinated with DTaP antigens (group 7 in FIG. 7A). Pups from dams primed with RSV and vaccinated once or twice with DTaP antigens only had RSV neutralizing titers of 425 and 563 (groups 3 and 6 in Fig. 7B), respectively, representing the levels of neutralizing antibodies transferred to the offspring due to live RSV priming. These neutralizing antibody titers were sufficient to induce full protection from RSV challenge in the pups (FIG. 8).

When comparing levels of neutralizing antibodies induced in dams after live RSV priming and either a single dose of PreF vaccine alone (group 1 in FIG. 7A) or combined dose of PreF and DTaP antigens (group 2 in FIG. 7B), no significant difference in neutralizing antibody titers is observed, indicating no interference of the DTaP vaccine on RSV neutralizing antibody response. A similar observation can be made for pup neutralizing antibody titers (compare groups 1 and 2 in FIG. 7B). However when primed dams received two doses of combined PreF and DTaP antigens, RSV neutralizing titers in the dams were lower than the ones obtained after PreF vaccination only, although the difference did not reach statistical significance (titers of 832 vs 1590 after combined PreF-DTaP versus PreF-only vaccine, respectively; groups 4 and 5, FIG. 7A). The levels of neutralizing antibodies transferred to pups when dams were vaccinated with two doses of combined PreF-DTaP vaccines were significantly lower than those observed when dams were vaccinated twice with PreF-only vaccine (titers of 519 vs 2439 after combined PreF-DTaP versus PreF-only vaccine, respectively; groups 4 and 5, FIG. 7B). These results indicate that maternal vaccination with a single dose of the combined DTaP-RSV vaccine does not cause interference in the levels of RSV neutralizing antibodies transferred to the pups, whereas some degree of interference was apparent in the levels of RSV neutralizing antibodies observed in pups 24 to 72h hours post-birth after two-dose maternal vaccination with combined DTaP-RSV.

Results from lung viral load in the guinea pig offspring (FIG. 8) indicate that offspring born to primed and vaccinated mothers were fully protected from RSV challenge, whatever the vaccine regimen used in dams. The fact that animals primed and vaccinated with DTaP antigens only (no RSV antigen) are fully protected from RSV challenge whereas animals unprimed and vaccinated with DTaP antigens only are not protected suggests that priming with live RSV was sufficient to induce protective levels of antibodies that were transferred to the offspring, irrespective of the vaccine regimen used after priming. The apparent interference on observed levels of elicited neutralizing antibodies after two doses of combined PreF and DTaP antigens (FIG. 7) did not have any detectable impact on protection of the pups from RSV challenge.

### SEQUENCE LISTING

SEQ ID NO:1
   Nucleotide sequence encoding RSV reference Fusion protein
   Strain A2 GenBank Accession No. U50362 
atggagttgctaatcctcaaagcaaatgcaattaccacaatcctcactgcagtcacatttgttttgcttctggtcaaaacatcactgaagaat
tttatcaatcaacatgcagtgcagtagcaaaggctatcttagtgctctgagaactggttggtataccagtgttataactatagattaagtaat
atcaaggaaaataagtgtaatggaacagatgctaaggtaaaattgataaacaagaattagataaatataaaaatgctgtaacagaattgc
agttgctcatgcaaagcacccagcaacaaacaatcgagccagaagagaactaccaaggtttatgaattatacactcaaaatgccaaaa
aaaccaatgtaacattaagcaagaaaaggaaaagaagatttcttggtttttgttaggtgttggatctgcaatcgccagtggcgttgctgtat
ctaaggtcctgcacctgaagggggaagtgaacaagatcaaaagtgctctactatccacaaacaaggctgtagtcagttatcaaatggagt
tagtgtcttaaccagcaaagtgttagacctcaaaaactatatagaaaacaattgttacctattgtgaacaagcaaagctgcagcatatcaa
atatagcaactgtatagagttccaacaaaagaacaacagactactagagattaccagggaatttagtgttaagcaggtgtaactacacct
gtaagcacttacatgttaactaatagtgaattattgtcattatcaatgatatgcctataacaaatgatcagaaaaagttaatgtccaacaatgt
tcaaatgttagacagcaaagttactctatcatgtccataataaaagaggaagtcttagcatatgtgtacaattaccactatatggtgttatag
atacaccctgttggaaactacacacatcccccctatgtacaaccaacacaaaagaagggtccaacatctgtttaacaagaactgacagag
gtggtactgtgacaatgcaggatcagtatctttcttcccacaagctgaaacatgtaaagtcaatcaaatcgagtattttgtgacacaatgaa
cagtttaacattaccaagtgaagtaaactctgcaatgttgacatattcaaccccaaatatgattgtaaaattatgacttcaaaaacgatgtaa
gcagctccgttatcacatctctaggagccattgtgtcatgctatggcaaaacaaatgtacagcatccaataaaaatcgtggaatcataaa
gacattttctaacgggtgcgatatgtatcaaataaagggggtggacactgtgtctgtaggtaacacattatattatgtaaaaagcaagaagg
taaaagtctctatgtaaaaggtgaaccaataataaatttctatgacccttagtattcccctctgatgaatttgatgcatcaatatctcaagtca
acgagaagattaacagagcctagcatttattcgtaaatccgatgaattattacataatgtaaatgctggtaatccaccataaatatcatgata
actactataattatagtgattatagtaatattgttatcttaattgctgttggactgctcttatactgtaaggccagaagcacaccagtcacacta
agaaaagatcaactgagtggtataaataatattgcatttagtaactaa
SEQ ID NO:2
   Amino acid sequence of RSV reference F protein precursor F₀
   Strain A2 GenBank Accession No. AAB86664 
Mellilkanaittiltavtfcfasgqniteefyqstcsavskgylsalrtgwytsvitielsnikenkcngtdakvklikqeldkyknavt
elqllmqstpatnnrarrelprfmnytlnnakktnvtlskkrkrrflgfllgvgsaiasgvavskvlhlegevnkiksallstnkavvsl
sngvsvltskvldlknyidkqllpivnkqscsisniatviefqqknnrlleitrefsvnagvttpvstymltnsellslindmpitndqk
klmsnnvqivrqqsysimsiikeevlayvvqlplygvidtpcwklhtsplcttntkegsnicltrtdrgwycdnagsvsffpqaetc
kvqsnrvfcdtmnsltlpsevnlcnvdifnpkydckimtsktdvsssvitslgaivscygktkctasnknrgiiktfsngcdyvsnk
 
gvdtvsvgntlyyvnkqegkslyvkgepiinfydplvfpsdefdasisqvnekinqslafirksdellhnvnagkstinimittiiivi
ivillsliavglllyckarstpvtlskdqlsginniafsn
SEQ ID NO:3
   Nucleotide sequence encoding RSV reference G protein
   Strain Long 
Atgtccaaaaacaaggaccaacgcaccgctaagacactagaaaagacctgggacactctcaatcatttattattcatatcatcgggctt
atataagttaaatcttaaatctatagcacaaatcacattatccattctggcaatgataatctcaacttcacttataattacagccatcatattcat
agcctcggcaaaccacaaagtcacactaacaactgcaatcatacaagatgcaacaagccagatcaagaacacaaccccaacatacct
cactcaggatcctcagcttggaatcagcttctccaatctgtctgaaattacatcacaaaccaccaccatactagcttcaacaacaccagg
agtcaagtcaaacctgcaacccacaacagtcaagactaaaaacacaacaacaacccaaacacaacccagcaagcccactacaaaac
aacgccaaaacaaaccaccaaacaaacccaataatgattttcacttcgaagtgtttaactttgtaccctgcagcatatgcagcaacaatc
caacctgctgggctatctgcaaaagaataccaaacaaaaaaccaggaaagaaaaccaccaccaagcctacaaaaaaaccaaccttc
aagacaaccaaaaaagatctcaaacctcaaaccactaaaccaaaggaagtacccaccaccaagcccacagaagagccaaccatca
acaccaccaaaacaaacatcacaactacactgctcaccaacaacaccacaggaaatccaaaactcacaagtcaaatggaaaccttcc
actcaacctcctccgaaggcaatctaagcccttctcaagtctccacaacatccgagcacccatcacaaccctcatctccacccaacaca
acacgccagtag
SEQ ID NO:4
   Amino acid sequence of RSV reference G protein 
MSKNKDQRTAKTLEKTWDTLNHLLFISSGLYKLNLKSIAQITLSILAMIISTSLIITAIIFI
ASANHKVTLTTAIIQDATSQIKNTTPTYLTQDPQLGISFSNLSEITSQTTTILASTTPGV
KSNLQPTTVKTKNTTTTQTQPSKPTTKQRQNKPPNKPNNDFHFEVFNFVPCSICSNNP
TCWAICKRIPNKKPGKKTTTKPTKKPTFKTTKKDLKPQTTKPKEVPTTKPTEEPTINT
TKTNITTTLLTNNTTGNPKLTSQMETFHSTSSEGNLSPSQVSTTSEHPSQPSSPPNTTR
Q
Seq ID NO:5
   Nucleotide sequence of PreF analog optimized for CHO 
aagcttgccaccatggagctgctgatcctgaaaaccaacgccatcaccgccatcctggccgccgtgaccctgtgcttcgcctcctccc
agaacatcaccgaggagttctaccagtccacctgctccgccgtgtccaagggctacctgtccgccctgcggaccggctggtacacct
ccgtgatcaccatcgagctgtccaacatcaaggaaaacaagtgcaacggcaccgacgccaaggtgaagctgatcaagcaggagct
 
ggacaagtacaagagcgccgtgaccgaactccagctgctgatgcagtccacccctgccaccaacaacaagtttctgggcttcctgctg
ggcgtgggctccgccatcgcctccggcatcgccgtgagcaaggtgctgcacctggagggcgaggtgaacaagatcaagagcgcc
ctgctgtccaccaacaaggccgtggtgtccctgtccaacggcgtgtccgtgctgacctccaaggtgctggatctgaagaactacatcg
acaagcagctgctgcctatcgtgaacaagcagtcctgctccatctccaacatcgagaccgtgatcgagttccagcagaagaacaacc
ggctgctggagatcacccgcgagttctccgtgaacgccggcgtgaccacccctgtgtccacctacatgctgaccaactccgagctgct
gtccctgatcaacgacatgcctatcaccaacgaccagaaaaaactgatgtccaacaacgtgcagatcgtgcggcagcagtcctacag
catcatgagcatcatcaaggaagaggtgctggcctacgtggtgcagctgcctctgtacggcgtgatcgacacccccttgctggaagctg
cacacctcccccctgtgcaccaccaacaccaaggagggctccaacatctgcctgacccggaccgaccggggctggtactgcgaca
acgccggctccgtgtccttcttccctctggccgagacctgcaaggtgcagtccaaccgggtgttctgcgacaccatgaactccctgacc
ctgccttccgaggtgaacctgtgcaacatcgacatcttcaaccccaagtacgactgcaagatcatgaccagcaagaccgacgtgtcct
ccagcgtgatcacctccctgggcgccatcgtgtcctgctacggcaagaccaagtgcaccgcctccaacaagaaccggggaatcatc
aagaccttctccaacggctgcgactacgtgtccaataagggcgtggacaccgtgtccgtgggcaacacactgtactacgtgaataagc
aggagggcaagagcctgtacgtgaagggcgagcctatcatcaacttctacgaccctctggtgttcccttccgacgagttcgacgcctc
catcagccaggtgaacgagaagatcaaccagtccctggccttcatccggaagtccgacgagaagctgcataacgtggaggacaaga
tcgaggagatcctgtccaaaatctaccacatcgagaacgagatcgcccggatcaagaagctgatcggcgaggcctgataatctaga
SEQ ID NO:6
   Amino acid sequence of PreF analog 
MELLILKTNAITAILAAVTLCFASSQNITEEFYQSTCSAVSKGYLSALRTGWYTSVITI
ELSNIKENKCNGTDAKVKLIKQELDKYKSAVTELQLLMQSTPATNNKFLGFLLGVGS
AIASGIAVSKVLHLEGEVNKIKSALLSTNKAVVSLSNGVSVLTSKVLDLKNYIDKQLL
PIVNKQSCSISNIETVIEFQQKNNRLLEITREFSVNAGVTTPVSTYMLTNSELLSLINDM
PITNDQKKLMSNNVQIVRQQSYSIMSIIKEEVLAYVVQLPLYGVIDTPCWKLHTSPLC
TTNTKEGSNICLTRTDRGWYCDNAGSVSFFPLAETCKVQSNRVFCDTMNSLTLPSEV
NLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQV
NEKINQSLAFIRKSDEKLHNVEDKIEEILSKIYHIENEIARIKKLIGEA
Seq ID NO:7
   Nucleotide sequence encoding PreFG_V1 optimized for CHO 
aagcttgccaccatggagctgctgatcctcaagaccaacgccatcaccgccatcctggccgccgtgaccctgtgcttcgcctcctccc
agaacatcaccgaagagttctaccagtccacctgctccgccgtgtccaagggctacctgtccgccctgcggaccggctggtacacctc
cgtgatcaccatcgagctgtccaacatcaaagaaaacaagtgcaacggcaccgacgccaaggtcaagctgatcaagcaggaactgg
acaagtacaagagcgccgtgaccgaactccagctgctgatgcagtccacccctgccaccaacaacaagaagtttctgggcttcctgct
 
gggcgtgggctccgccatcgcctccggcatcgccgtgagcaaggtgctgcacctggagggcgaggtgaacaagatcaagagcgc
cctgctgtccaccaacaaggccgtggtgtccctgtccaacggcgtgtccgtgctgacctccaaggtgctggatctgaagaactacatc
gacaagcagctgctgcctatcgtgaacaagcagtcctgctccatctccaacatcgagaccgtgatcgagttccagcagaagaacaac
cggctgctggagatcacccgcgagttctccgtgaacgccggcgtgaccacccctgtgtccacctacatgctgacaaactccgagctg
ctctccctgatcaacgacatgcctatcaccaacgaccaaaaaaagctgatgtccaacaacgtgcagatcgtgcggcagcagtcctaca
gcatcatgagcatcatcaaggaagaagtcctggcctacgtcgtgcagctgcctctgtacggcgtgatcgacaccccttgctggaagct
gcacacctccccctgtgcaccaccaacaccaaagagggctccaacatctgcctgacccggaccgaccggggctggtactgcgac
aacgccggctccgtgtccttcttccctctggccgagacctgcaaggtgcagtccaaccgggtgttctgcgacaccatgaactccctgac
cctgccttccgaggtgaacctgtgcaacatcgacatcttcaaccccaagtacgactgcaagatcatgaccagcaagaccgacgtgtcc
tccagcgtgatcacctccctgggcgccatcgtgtcctgctacggcaagaccaagtgcaccgcctccaacaagaaccggggaatcatc
aagaccttctccaacggctgcgactacgtgtccaataagggcgtggacaccgtgtccgtgggcaacacactgtactacgtgaataagc
aggaaggcaagagcctgtacgtgaagggcgagcctatcatcaacttctacgaccctctggtgttcccttccgacgagttcgacgcctc
catcagccaggtcaacgagaagatcaaccagtccctggccttcatccggaagtccgacgagaagctgcataacgtggaggacaaga
tcgaagagatcctgtccaaaatctaccacatcgagaacgagatcgcccggatcaagaagctgatcggcgaggctggcggctctggc
ggcagcggcggctccaagcagcggcagaacaagcctcctaacaagcccaacaacgacttccacttcgaggtgttcaacttcgtgcct
tgctccatctgctccaacaaccctacctgctgggccatctgcaagagaatccccaacaagaagcctggcaagaaaaccaccaccaag
cctaccaagaagcctaccttcaagaccaccaagaaggaccacaagcctcagaccacaaagcctaaggaagtgccaaccaccaagc
accaccaccatcaccactgataatcta
Seq ID NO:8
   PreFG_V1 peptide for CHO 
MELLILKTNAITAILAAVTLCFASSQNITEEFYQSTCSAVSKGYLSALRTGWYTSVITI
ELSNIKENKCNGTDAKVKLIKQELDKYKSAVTELQLLMQSTPATNNKFLGFLLGV
GSAIASGIAVSKVLHLEGEVNKIKSALLSTNKAVVSLSNGVSVLTSKVLDLKNYIDKQ
LLPIVNKQSCSISNIETVIEFQQKNNRLLEITREFSVNAGVTTPVSTYMLTNSELLSLIN
DMPITNDQKKLMSNNVQIVRQQSYSIMSIIKEEVLAYVVQLPLYGVIDTPCWKLHTSP
LCTTNTKEGSNICLTRTDRGWYCDNAGSVSFFPLAETCKVQSNRVFCDTMNSLTLPS
EVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSDEFDASIS
QVNEKINQSLAFIRKSDEKLHNVEDKIEEILSKIYHIENEIARIKKLIGEAGGSGGSGGS
KQRQNKPPNKPNNDFHFEVFNFVPCSICSNNPTCWAICKRIPNKKPGKKTTTKPTKKP
TFKTTKKDHKPQTTKPKEVPTTK
Seq ID NO:9
   Nucleotide Sequence encoding PreFG_V2 for CHO 
aagcttgccaccatggagctgctgatcctcaagaccaacgccatcaccgccatcctggccgccgtgaccctgtgcttcgcctcctccc
agaacatcaccgaagagttctaccagtccacctgctccgccgtgtccaagggctacctgtccgccctgcggaccggctggtacacctc
cgtgatcaccatcgagctgtccaacatcaaagaaaacaagtgcaacggcaccgacgccaaggtcaagctgatcaagcaggaactgg
acaagtacaagagcgccgtgaccgaactccagctgctgatgcagtccacccctgccaccaacaacaagaagtttctgggcttcctgct
gggcgtgggctccgccatcgcctccggcatcgccgtgagcaaggtgctgcacctggagggcgaggtgaacaagatcaagagcgc
cctgctgtccaccaacaaggccgtggtgtccctgtccaacggcgtgtccgtgctgacctccaaggtgctggatctgaagaactacatc
gacaagcagctgctgcctatcgtgaacaagcagtcctgctccatctccaacatcgagaccgtgatcgagttccagcagaagaacaac
cggctgctggagatcacccgcgagttctccgtgaacgccggcgtgaccacccctgtgtccacctacatgctgacaaactccgagctg
ctctccctgatcaacgacatgcctatcaccaacgaccaaaaaaagctgatgtccaacaacgtgcagatcgtgcggcagcagtcctaca
gcatcatgagcatcatcaaggaagaagtcctggcctacgtcgtgcagctgcctctgtacggcgtgatcgacaccccttgctggaagct
gcacacctccccccctgtgcaccaccaacaccaaagagggctccaacatctgcctgacccggaccgaccggggctggtactgcgac
aacgccggctccgtgtccttcttccctctggccgagacctgcaaggtgcagtccaaccgggtgttctgcgacaccatgaactccctgac
cctgccttccgaggtgaacctgtgcaacatcgacatcttcaaccccaagtacgactgcaagatcatgaccagcaagaccgacgtgtcc
tccagcgtgatcacctccctgggcgccatcgtgtcctgctacggcaagaccaagtgcaccgcctccaacaagaaccggggaatcatc
aagaccttctccaacggctgcgactacgtgtccaataagggcgtggacaccgtgtccgtgggcaacacactgtactacgtgaataagc
aggaaggcaagagcctgtacgtgaagggcgagcctatcatcaacttctacgaccctctggtgttcccttccgacgagttcgacgcctc
catcagccaggtcaacgagaagatcaaccagtccctggccttcatccggaagtccgacgagaagctgcataacgtggaggacaaga
tcgaagagatcctgtccaaaatctaccacatcgagaacgagatcgcccggatcaagaagctgatcggcgaggctggcggcaagcag
cggcagaacaagcctcctaacaagcccaacaacgacttccacttcgaggtgttcaacttcgtgccttgctccatctgctccaacaaccct
acctgctgggccatctgcaagagaatccccaacaagaagcctggcaagaaaaccaccaccaagcctaccaagaagcctaccttcaa
gaccaccaagaaggaccacaagcctcagaccacaaagcctaaggaagtgccaaccaccaagcaccaccaccatcaccactgataa
tcta
Seq ID NO:10
   PreFG_V2 peptide for CHO 
MELLILKTNAITAILAAVTLCFASSQNITEEFYQSTCSAVSKGYLSALRTGWYTSVITI
ELSNIKENKCNGTDAKVKLIKQELDKYKSAVTELQLLMQSTPATNNKKFLGFLLGV
GSAIASGIAVSKVLHLEGEVNKIKSALLSTNKAVVSLSNGVSVLTSKVLDLKNYIDKQ
LLPIVNKQSCSISNIETVIEFQQKNNRLLEITREFSVNAGVTTPVSTYMLTNSELLSLIN
DMPITNDQKKLMSNNVQIVRQQSYSIMSIIKEEVLAYVVQLPLYGVIDTPCWKLHTSP
LCTTNTKEGSNICLTRTDRGWYCDNAGSVSFFPLAETCKVQSNRVFCDTMNSLTLPS
EVNLCNIDIFNPKYDCKIMTSKTDVSSSVITSLGAIVSCYGKTKCTASNKNRGIIKTFS
NGCDYVSNKGVDTVSVGNTLYYVNKQEGKSLYVKGEPIINFYDPLVFPSDEFDASIS
 
QVNEKINQSLAFIRKSDEKLHNVEDKIEEILSKIYHIENEIARIKKLIGEAGGKQRQNKP
PNKPNNDFHFEVFNFVPCSICSNNPTCWAICKRIPNKKPGKKTTTKPTKKPTFKTTKK
DHKPQTTKPKEVPTTK
SEQ ID NO:11
   Exemplary coiled-coil (isoleucine zipper)
   EDKIEEILSKIYHIENEIARIKKLIGEA
SEQ ID NO:12
   PreF antigen polynucleotide CHO2 
atggagctgcccatcctgaagaccaacgccatcaccatcctcgccgccgtgaccctgtgcttcgccagcagccagaacatcacg
gaggagttctaccagagcacgtgcagcgccgtgagcaagggctacctgagcgcgctgcgcacgggctggtacacgagcgtgatca
cgatcgagctgagcaacatcaaggagaacaagtgcaacggcacggacgcgaaggtgaagctgatcaagcaggagctggacaagt
acaagagcgcggtgacggagctgcagctgctgatgcagagcacgccggcgacgaacaacaagttcctcggcttcctgctgggcgt
gggcagcgcgatcgcgagcggcatcgccgtgagcaaggtgctgcacctggagggcgaggtgaacaagatcaagtccgcgctgct
gagcacgaacaaggcggtcgtgagcctgagcaacggcgtgagcgtgctgacgagcaaggtgctcgacctgaagaactacatcgac
aagcagctgctgccgatcgtgaacaagcagagctgcagcatcagcaacatcgagaccgtgatcgagttccagcagaagaacaacc
gcctgctggagatcacgcgggagttctccgtgaacgcaggcgtgacgacgcccgtgtctacgtacatgctgacgaacagcgagctg
ctcagcctgatcaacgacatgccgatcacgaacgaccagaagaagctgatgagcaacaacgtgcagatcgtgcgccagcagagcta
cagcatcatgagcatcatcaaggaggaggtgctggcatacgtggtgcagctgccgctgtacggcgtcatcgacacgccctgctggaa
gctgcacacgagcccgctgtgcacgaccaacacgaaggagggcagcaacatctgcctgacgcggacggaccggggctggtactg
cgacaacgcgggcagcgtgagcttcttcccgctcgcggagacgtgcaaggtgcagagcaaccgcgtcttctgcgacacgatgaaca
gcctgacgctgccgagcgaggtgaacctgtgcaacatcgacatcttcaacccgaagtacgactgcaagatcatgacgagcaagacc
gatgtcagcagcagcgtgatcacgagcctcggcgcgatcgtgagctgctacggcaagacgaagtgcacggcgagcaacaagaac
cgcggcatcatcaagacgttcagcaacggctgcgactatgtgagcaacaagggcgtggacactgtgagcgtcggcaacacgctgta
ctacgtgaacaagcaggagggcaagagcctgtacgtgaagggcgagccgatcatcaacttctacgacccgctcgtgttcccgagcg
acgagttcgacgcgagcatcagccaagtgaacgagaagatcaaccagagcctggcgttcatccgcaagagcgacgagaagctgca
caacgtggaggacaagatcgaggagatcctgagcaagatctaccacatcgagaacgagatcgcgcgcatcaagaagctgatcggc
gaggcgcatcatcaccatcaccattga
SEQ ID NO:13
   PreF antigen polynucleotide with intron 
atggagctgctgatcctgaaaaccaacgccatcaccgccatcctggccgccgtgaccctgtgcttcgcctcctcccagaacatcaccg
aggagttctaccagtccacctgctccgccgtgtccaagggctacctgtccgccctgcggaccggctggtacacctccgtgatcaccat
 
cgagctgtccaacatcaaggaaaacaagtgcaacggcaccgacgccaaggtgaagctgatcaagcaggagctggacaagtacaag
agcgccgtgaccgaactccagctgctgatgcagtccacccctgccaccaacaacaagtttctgggcttcctgctgggcgtgggctccg
ccatcgcctccggcatcgccgtgagcaaggtacgtgtcgggacttgtgttcccctttttttaataaaaagttatatctttaatgttatatacat
atttcctgtatgtgatccatgtgcttatgactttgtttatcatgtgtttaggtgctgcacctggagggcgaggtgaacaagatcaagagcgc
cctgctgtccaccaacaaggccgtggtgtccctgtccaacggcgtgtccgtgctgacctccaaggtgctggatctgaagaactacatc
gacaagcagctgctgcctatcgtgaacaagcagtcctgctccatctccaacatcgagaccgtgatcgagttccagcagaagaacaac
cggctgctggagatcacccgcgagttctccgtgaacgccggcgtgaccacccctgtgtccacctacatgctgaccaactccgagctg
ctgtccctgatcaacgacatgcctatcaccaacgaccagaaaaaactgatgtccaacaacgtgcagatcgtgcggcagcagtcctaca
gcatcatgagcatcatcaaggaagaggtgctggcctacgtggtgcagctgcctctgtacggcgtgatcgacaccccttgctggaagct
gcacacctccccctgtgcaccaccaacaccaaggagggctccaacatctgcctgacccggaccgaccggggctggtactgcgac
aacgccggctccgtgtccttcttccctctggccgagacctgcaaggtgcagtccaaccgggtgttctgcgacaccatgaactccctgac
cctgccttccgaggtgaacctgtgcaacatcgacatcttcaaccccaagtacgactgcaagatcatgaccagcaagaccgacgtgtcc
tccagcgtgatcacctccctgggcgccatcgtgtcctgctacggcaagaccaagtgcaccgcctccaacaagaaccggggaatcatc
aagaccttctccaacggctgcgactacgtgtccaataagggcgtggacaccgtgtccgtgggcaacacactgtactacgtgaataagc
aggagggcaagagcctgtacgtgaagggcgagcctatcatcaacttctacgaccctctggtgttcccttccgacgagttcgacgcctc
catcagccaggtgaacgagaagatcaaccagtccctggccttcatccggaagtccgacgagaagctgcataacgtggaggacaaga
tcgaggagatcctgtccaaaatctaccacatcgagaacgagatcgcccggatcaagaagctgatcggcgaggccggaggtcaccac
caccatcaccactga
SEQ ID NO:14
   Synthetic linker sequence
   GGSGGSGGS
SEQ ID NO:15
   Furin cleavage site
   RARR
SEQ ID NO:16
   Furin cleavage site
   RKRR
SEQ ID NO:17
   Nucleotide Sequence encoding PreF_NGTL 
atggagctgctgatcctgaaaaccaacgccatcaccgccatcctggccgccgtgaccctgtgcttcgcctcctcccagaacatcaccg
aggagttctaccagtccacctgctccgccgtgtccaagggctacctgtccgccctgcggaccggctggtacacctccgtgatcaccat
cgagctgtccaacatcaaggaaaacaagtgcaacggcaccgacgccaaggtgaagctgatcaagcaggagctggacaagtacaag
agcgccgtgaccgaactccagctgctgatgcagtccacccctgccaccaacaacaagtttctgggcttcctgctgggcgtgggctccg
ccatcgcctccggcatcgccgtgagcaaggtgctgcacctggagggcgaggtgaacaagatcaagagcgccctgctgtccaccaa
caaggccgtggtgtccctgtccaacggcgtgtccgtgctgacctccaaggtgctggatctgaagaactacatcgacaagcagctgctg
cctatcgtgaacaagcagtcctgctccatctccaacatcgagaccgtgatcgagttccagcagaagaacaaccggctgctggagatca
cccgcgagttctccgtgaacgccggcgtgaccacccctgtgtccacctacatgctgaccaactccgagctgctgtccctgatcaacga
catgcctatcaccaacgaccagaaaaaactgatgtccaacaacgtgcagatcgtgcggcagcagtcctacagcatcatgagcatcatc
aaggaagaggtgctggcctacgtggtgcagctgcctctgtacggcgtgatcgacaccccttgctggaagctgcacacctcccccctgt
gcaccaccaacaccaaggagggctccaacatctgcctgacccggaccgaccggggctggtactgcgacaacgccggctccgtgtc
cttcttccctctggccgagacctgcaaggtgcagtccaaccgggtgttctgcgacaccatgaactccctgaccctgccttccgaggtga
acctgtgcaacatcgacatcttcaaccccaagtacgactgcaagatcatgaccagcaagaccgacgtgtcctccagcgtgatcacctc
cctgggcgccatcgtgtcctgctacggcaagaccaagtgcaccgcctccaacaagaaccggggaatcatcaagaccttctccaacg
gctgcgactacgtgtccaataagggcgtggacaccgtgtccgtgggcaacacactgtactacgtgaataagcaggagggcaagagc
ctgtacgtgaagggcgagcctatcatcaacttctacgaccctctggtgttcccttccgacgagttcgacgcctccatcagccaggtgaa
cgagaagatcaacgggaccctggccttcatccggaagtccgacgagaagctgcataacgtggaggacaagatcgaggagatcctgt
ccaaaatctaccacatcgagaacgagatcgcccggatcaagaagctgatcggcgaggcc
SEQ ID NO:18
   Amino Acid Sequence of PreF_NGTL 
MELLILKTNAITAILAAVTLCFASSQNITEEFYQSTCSAVSKGYLSALRTGWYTSVITI
ELSNIKENKCNGTDAKVKLIKQELDKYKSAVTELQLLMQSTPATNNKFLGFLLGVGS
AIASGIAVSKVLHLEGEVNKIKSALLSTNKAVVSLSNGVSVLTSKVLDLKNYIDKQLL
PIVNKQSCSISNIETVIEFQQKNNRLLEITREFSVNAGVTTPVSTYMLTNSELLSLINDM
PITNDQKKLMSNNVQIVRQQSYSIMSIIKEEVLAYVVQLPLYGVIDTPCWKLHTSPLC
TTNTKEGSNICLTRTDRGWYCDNAGSVSFFPLAETCKVQSNRVFCDTMNSLTLPSEV
NLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQV
NEKINGTLAFIRKSDEKLHNVEDKIEEILSKIYHIENEIARIKKLIGEA
SEQ ID NO:19
   Nucleotide Sequence encoding PreF_L112Q 
atggagctgctgatcctgaaaaccaacgccatcaccgccatcctggccgccgtgaccctgtgcttcgcctcctcccagaacatcaccg
aggagttctaccagtccacctgctccgccgtgtccaagggctacctgtccgccctgcggaccggctggtacacctccgtgatcaccat
cgagctgtccaacatcaaggaaaacaagtgcaacggcaccgacgccaaggtgaagctgatcaagcaggagctggacaagtacaag
agcgccgtgaccgaactccagctgctgatgcagtccacccctgccaccaacaacaagtttctgggcttcctgcagggcgtgggctcc
gccatcgcctccggcatcgccgtgagcaaggtgctgcacctggagggcgaggtgaacaagatcaagagcgccctgctgtccacca
acaaggccgtggtgtccctgtccaacggcgtgtccgtgctgacctccaaggtgctggatctgaagaactacatcgacaagcagctgct
gcctatcgtgaacaagcagtcctgctccatctccaacatcgagaccgtgatcgagttccagcagaagaacaaccggctgctggagatc
acccgcgagttctccgtgaacgccggcgtgaccacccctgtgtccacctacatgctgaccaactccgagctgctgtccctgatcaacg
acatgcctatcaccaacgaccagaaaaaactgatgtccaacaacgtgcagatcgtgcggcagcagtcctacagcatcatgagcatcat
caaggaagaggtgctggcctacgtggtgcagctgcctctgtacggcgtgatcgacacccccttgctggaagctgcacacctccccct
gtgcaccaccaacaccaaggagggctccaacatctgcctgacccggaccgaccggggctggtactgcgacaacgccggctccgtg
tccttcttccctctggccgagacctgcaaggtgcagtccaaccgggtgttctgcgacaccatgaactccctgaccctgccttccgaggt
gaacctgtgcaacatcgacatcttcaaccccaagtacgactgcaagatcatgaccagcaagaccgacgtgtcctccagcgtgatcacc
tccctgggcgccatcgtgtcctgctacggcaagaccaagtgcaccgcctccaacaagaaccggggaatcatcaagaccttctccaac
ggctgcgactacgtgtccaataagggcgtggacaccgtgtccgtgggcaacacactgtactacgtgaataagcaggagggcaagag
cctgtacgtgaagggcgagcctatcatcaacttctacgaccctctggtgttcccttccgacgagttcgacgcctccatcagccaggtga
acgagaagatcaaccagtccctggccttcatccggaagtccgacgagaagctgcataacgtggaggacaagatcgaggagatcctg
tccaaaatctaccacatcgagaacgagatcgcccggatcaagaagctgatcggcgaggcc
SEQ ID NO:20
   Amino Acid Sequence of PreF_L112Q 
MELLILKTNAITAILAAVTLCFASSQNITEEFYQSTCSAVSKGYLSALRTGWYTSVITI
ELSNIKENKCNGTDAKVKLIKQELDKYKSAVTELQLLMQSTPATNNKFLGFLQGVGS
AIASGIAVSKVLHLEGEVNKIKSALLSTNKAVVSLSNGVSVLTSKVLDLKNYIDKQLL
PIVNKQSCSISNIETVIEFQQKNNRLLEITREFSVNAGVTTPVSTYMLTNSELLSLINDM
PITNDQKKLMSNNVQIVRQQSYSIMSIIKEEVLAYVVQLPLYGVIDTPCWKLHTSPLC
TTNTKEGSNICLTRTDRGWYCDNAGSVSFFPLAETCKVQSNRVFCDTMNSLTLPSEV
NLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQV
NEKINQSLAFIRKSDEKLHNVEDKIEEILSKIYHIENEIARIKKLIGEA
SEQ ID NO:21
   Nucleotide Sequence encoding PreF_NGTL_L112Q 
atggagctgctgatcctgaaaaccaacgccatcaccgccatcctggccgccgtgaccctgtgcttcgcctcctcccagaacatcaccg
aggagttctaccagtccacctgctccgccgtgtccaagggctacctgtccgccctgcggaccggctggtacacctccgtgatcaccat
cgagctgtccaacatcaaggaaaacaagtgcaacggcaccgacgccaaggtgaagctgatcaagcaggagctggacaagtacaag
agcgccgtgaccgaactccagctgctgatgcagtccacccctgccaccaacaacaagtttctgggcttcctgcagggcgtgggctcc
gccatcgcctccggcatcgccgtgagcaaggtgctgcacctggagggcgaggtgaacaagatcaagagcgccctgctgtccacca
acaaggccgtggtgtccctgtccaacggcgtgtccgtgctgacctccaaggtgctggatctgaagaactacatcgacaagcagctgct
gcctatcgtgaacaagcagtcctgctccatctccaacatcgagaccgtgatcgagttccagcagaagaacaaccggctgctggagatc
acccgcgagttctccgtgaacgccggcgtgaccacccctgtgtccacctacatgctgaccaactccgagctgctgtccctgatcaacg
acatgcctatcaccaacgaccagaaaaaactgatgtccaacaacgtgcagatcgtgcggcagcagtcctacagcatcatgagcatcat
caaggaagaggtgctggcctacgtggtgcagctgcctctgtacggcgtgatcgacaccccttgctggaagctgcacacctccccccct
gtgcaccaccaacaccaaggagggctccaacatctgcctgacccggaccgaccggggctggtactgcgacaacgccggctccgtg
tccttcttccctctggccgagacctgcaaggtgcagtccaaccgggtgttctgcgacaccatgaactccctgaccctgccttccgaggt
gaacctgtgcaacatcgacatcttcaaccccaagtacgactgcaagatcatgaccagcaagaccgacgtgtcctccagcgtgatcacc
tccctgggcgccatcgtgtcctgctacggcaagaccaagtgcaccgcctccaacaagaaccggggaatcatcaagaccttctccaac
ggctgcgactacgtgtccaataagggcgtggacaccgtgtccgtgggcaacacactgtactacgtgaataagcaggagggcaagag
cctgtacgtgaagggcgagcctatcatcaacttctacgaccctctggtgttcccttccgacgagttcgacgcctccatcagccaggtga
acgagaagatcaacgggaccctggccttcatccggaagtccgacgagaagctgcataacgtggaggacaagatcgaggagatcct
gtccaaaatctaccacatcgagaacgagatcgcccggatcaagaagctgatcggcgaggcc
SEQ ID NO:22
   Amino Acid Sequence of PreF_NGTL_L112Q

MELLILKTNAITAILAAVTLCFASSQNITEEFYQSTCSAVSKGYLSALRTGWYTSVITI
ELSNIKENKCNGTDAKVKLIKQELDKYKSAVTELQLLMQSTPATNNKFLGFLQGVGS
AIASGIAVSKVLHLEGEVNKIKSALLSTNKAVVSLSNGVSVLTSKVLDLKNYIDKQLL
PIVNKQSCSISNIETVIEFQQKNNRLLEITREFSVNAGVTTPVSTYMLTNSELLSLINDM
PITNDQKKLMSNNVQIVRQQSYSIMSIIKEEVLAYVVQLPLYGVIDTPCWKLHTSPLC
TTNTKEGSNICLTRTDRGWYCDNAGSVSFFPLAETCKVQSNRVFCDTMNSLTLPSEV
NLCNIDIFNPKYDCKIMTSKTDVSSSVITSLGAIVSCYGKTKCTASNKNRGIIKTFSNG
CDYVSNKGVDTVSVGNTLYYVNKQEGKSLYVKGEPIINFYDPLVFPSDEFDASISQV
NEKINGTLAFIRKSDEKLHNVEDKIEEILSKIYHIENEIARIKKLIGEA

## Claims

1. A combination immunogenic composition comprising at least one Respiratory Syncytial Virus (RSV) antigen and at least one *Bordetella pertussis* antigen, wherein said at least one RSV antigen is a recombinant soluble PreF antigen that comprises at least one modification that stabilizes the prefusion conformation of the F protein and the at least one *B. pertussis* antigen comprises at least one acellular pertussis (Pa) antigen selected from the group consisting of: pertussis toxoid (PT), filamentous haemagglutinin (FHA), pertactin (PRN), fimbrae type 2 (FIM2), fimbrae type 3 (FIM3) and BrkA.

2. The combination immunogenic composition of claim 1 wherein the recombinant soluble PreF antigen comprises in an N-terminal to C-terminal direction: an F₂ domain and an F₁ domain of an RSV F protein polypeptide, and a heterologous trimerization domain, wherein there is no furin cleavage site between the F₂ domain and the F₁ domain.

3. The combination immunogenic composition of claim 1, wherein the recombinant soluble PreF antigen is a polypeptide comprising a polypeptide selected from the group of SEQ ID NO:6, SEQ ID NO:8, SEQ ID NO:10, SEQ ID NO:18, SEQ ID NO:20 and SEQ ID NO:22.

4. The combination immunogenic composition of any one of claims 1-3, wherein the recombinant soluble PreF antigen is selected from the group of:
i. a polypeptide comprising SEQ ID NO:22;
ii. a polypeptide encoded by SEQ ID NO:21.

5. The combination immunogenic composition of claim 2, wherein the heterologous trimerization domain comprises a coiled-coil domain or comprises an isoleucine zipper.

6. The combination immunogenic composition of claim 5, wherein the isoleucine zipper domain comprises the amino acid sequence of SEQ ID NO:11.

7. The combination immunogenic composition of claim 2 wherein the F₁ and F₂ polypeptide domains correspond in sequence to the RSV A Long strain.

8. The combination immunogenic composition of any preceding claim wherein the at least one RSV antigen comprises a multimer, such as a trimer, of polypeptides.

9. The combination immunogenic composition of any preceding claim wherein the PT is chemically toxoided, or is genetically toxoided for example by one or both of the mutations: R9K and E129G.

10. The combination immunogenic composition of any preceding claim wherein said at least one Pa antigen comprises: PT and FHA; PT, FHA and PRN; or PT, FHA, PRN and either or both of FIM2 and FIM3.

11. The combination immunogenic composition of any preceding claim further comprising a pharmaceutically acceptable carrier or excipient.

12. The combination immunogenic composition of any preceding claim further comprising at least one adjuvant selected from the group of: an aluminium salt such as aluminium hydroxide or aluminium phosphate; calcium phosphate; 3D-MPL; QS21; a CpG-containing oligodeoxynucleotide adjuvant; and an oil-in-water emulsion.

13. The combination immunogenic composition of claim 12 wherein the at least one adjuvant comprises aluminium hydroxide.

14. The combination immunogenic composition of claim 12 wherein the at least one adjuvant comprises an oil-in-water emulsion.

15. The combination immunogenic composition of any preceding claim further comprising at least one antigen from a pathogenic organism other than RSV and *B. pertussis.*

16. The combination immunogenic composition of claim 15, comprising one or more antigens selected from the group consisting of: diphtheria toxoid (D); tetanus toxoid (T); Hepatitis B surface antigen (HBsAg); inactivated polio virus (IPV); capsular saccharide of *H. influenzae* type b (Hib) conjugated to a carrier protein; capsular saccharide *of N. meningitidis* type C conjugated to a carrier protein; capsular saccharide *of N. meningitidis* type Y conjugated to a carrier protein; capsular saccharide *of N. meningitidis* type A conjugated to a carrier protein; capsular saccharide *of N. meningitidis* type W conjugated to a carrier protein; and an antigen from *N. meningitidis* type B.

17. The combination immunogenic composition of claim 16, comprising D and T; D, T and IPV; D, T and HBsAg; D, T and Hib; D, T, IPV and HBsAg; D, T, IPV and Hib; D, T, HBsAg and Hib; or D, T, IPV, HBsAg and Hib.

18. A method for eliciting an immune response against RSV and *B. pertussis,* comprising administering to a subject the combination immunogenic composition of any one of claims 1 to 17, wherein the subject is a mammal, such as a human, selected from the group of: a neonate; an infant; a child; an adolescent; an adult; and an elderly adult, wherein the subject is not a pregnant female.


SEQUENCE LISTING

<110>  GlaxoSmithKline Biologicals S.A.

<120>  COMBINATION IMMUNOGENIC COMPOSITIONS

<130>  VB65526

<160>  22

<170>  PatentIn version 3.5

<210>  1
<211>  1697
<212>  DNA
<213>  respiratory syncytial virus

<400>  1
atggagttgc taatcctcaa agcaaatgca attaccacaa tcctcactgc agtcacattt      60

gttttgcttc tggtcaaaac atcactgaag aattttatca atcaacatgc agtgcagtag     120

caaaggctat cttagtgctc tgagaactgg ttggtatacc agtgttataa ctatagatta     180

agtaatatca aggaaaataa gtgtaatgga acagatgcta aggtaaaatt gataaacaag     240

aattagataa atataaaaat gctgtaacag aattgcagtt gctcatgcaa agcacccagc     300

aacaaacaat cgagccagaa gagaactacc aaggtttatg aattatacac tcaaaatgcc     360

aaaaaaacca atgtaacatt aagcaagaaa aggaaaagaa gatttcttgg tttttgttag     420

gtgttggatc tgcaatcgcc agtggcgttg ctgtatctaa ggtcctgcac ctgaagggga     480

agtgaacaag atcaaaagtg ctctactatc cacaaacaag gctgtagtca gttatcaaat     540

ggagttagtg tcttaaccag caaagtgtta gacctcaaaa actatataga aaacaattgt     600

tacctattgt gaacaagcaa agctgcagca tatcaaatat agcaactgta tagagttcca     660

acaaaagaac aacagactac tagagattac cagggaattt agtgttaagc aggtgtaact     720

acacctgtaa gcacttacat gttaactaat agtgaattat tgtcattatc aatgatatgc     780

ctataacaaa tgatcagaaa aagttaatgt ccaacaatgt tcaaatgtta gacagcaaag     840

ttactctatc atgtccataa taaaagagga agtcttagca tatgtgtaca attaccacta     900

tatggtgtta tagatacacc ctgttggaaa ctacacacat ccccctatgt acaaccaaca     960

caaaagaagg gtccaacatc tgtttaacaa gaactgacag aggtggtact gtgacaatgc    1020

aggatcagta tctttcttcc cacaagctga aacatgtaaa gtcaatcaaa tcgagtattt    1080

tgtgacacaa tgaacagttt aacattacca agtgaagtaa actctgcaat gttgacatat    1140

tcaaccccaa atatgattgt aaaattatga cttcaaaaac gatgtaagca gctccgttat    1200

cacatctcta ggagccattg tgtcatgcta tggcaaaaca aatgtacagc atccaataaa    1260

aatcgtggaa tcataaagac attttctaac gggtgcgata tgtatcaaat aaaggggtgg    1320

acactgtgtc tgtaggtaac acattatatt atgtaaaaag caagaaggta aaagtctcta    1380

tgtaaaaggt gaaccaataa taaatttcta tgacccttag tattcccctc tgatgaattt 1440

gatgcatcaa tatctcaagt caacgagaag attaacagag cctagcattt attcgtaaat 1500

ccgatgaatt attacataat gtaaatgctg gtaatccacc ataaatatca tgataactac 1560

tataattata gtgattatag taatattgtt atcttaattg ctgttggact gctcttatac 1620

tgtaaggcca gaagcacacc agtcacacta agaaagatca actgagtggt ataaataata 1680

ttgcatttag taactaa 1697

<210> 2
<211> 574
<212> PRT
<213> respiratory syncytial virus

<400> 2

Met Glu Leu Leu Ile Leu Lys Ala Asn Ala Ile Thr Thr Ile Leu Thr
1               5                   10                  15

Ala Val Thr Phe Cys Phe Ala Ser Gly Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Asn Ala Val Thr Glu Leu Gln Leu Leu
                85                  90                  95

Met Gln Ser Thr Pro Ala Thr Asn Asn Arg Ala Arg Arg Glu Leu Pro
            100                 105                 110

Arg Phe Met Asn Tyr Thr Leu Asn Asn Ala Lys Lys Thr Asn Val Thr
        115                 120                 125

Leu Ser Lys Lys Arg Lys Arg Arg Phe Leu Gly Phe Leu Leu Gly Val
    130                 135                 140

Gly Ser Ala Ile Ala Ser Gly Val Ala Val Ser Lys Val Leu His Leu
145                 150                 155                 160

Glu Gly Glu Val Asn Lys Ile Lys Ser Ala Leu Leu Ser Thr Asn Lys
                165                 170                 175

Ala Val Val Ser Leu Ser Asn Gly Val Ser Val Leu Thr Ser Lys Val
            180                 185                 190

Leu Asp Leu Lys Asn Tyr Ile Asp Lys Gln Leu Leu Pro Ile Val Asn
        195                 200                 205

Lys Gln Ser Cys Ser Ile Ser Asn Ile Ala Thr Val Ile Glu Phe Gln
    210                 215                 220

Gln Lys Asn Asn Arg Leu Leu Glu Ile Thr Arg Glu Phe Ser Val Asn
225                 230                 235                 240

Ala Gly Val Thr Thr Pro Val Ser Thr Tyr Met Leu Thr Asn Ser Glu
                245                 250                 255

Leu Leu Ser Leu Ile Asn Asp Met Pro Ile Thr Asn Asp Gln Lys Lys
            260                 265                 270

Leu Met Ser Asn Asn Val Gln Ile Val Arg Gln Gln Ser Tyr Ser Ile
        275                 280                 285

Met Ser Ile Ile Lys Glu Glu Val Leu Ala Tyr Val Val Gln Leu Pro
    290                 295                 300

Leu Tyr Gly Val Ile Asp Thr Pro Cys Trp Lys Leu His Thr Ser Pro
305                 310                 315                 320

Leu Cys Thr Thr Asn Thr Lys Glu Gly Ser Asn Ile Cys Leu Thr Arg
                325                 330                 335

Thr Asp Arg Gly Trp Tyr Cys Asp Asn Ala Gly Ser Val Ser Phe Phe
            340                 345                 350

Pro Gln Ala Glu Thr Cys Lys Val Gln Ser Asn Arg Val Phe Cys Asp
        355                 360                 365

Thr Met Asn Ser Leu Thr Leu Pro Ser Glu Val Asn Leu Cys Asn Val
    370                 375                 380

Asp Ile Phe Asn Pro Lys Tyr Asp Cys Lys Ile Met Thr Ser Lys Thr
385                 390                 395                 400

Asp Val Ser Ser Ser Val Ile Thr Ser Leu Gly Ala Ile Val Ser Cys
                405                 410                 415

Tyr Gly Lys Thr Lys Cys Thr Ala Ser Asn Lys Asn Arg Gly Ile Ile

420                 425                 430

Lys Thr Phe Ser Asn Gly Cys Asp Tyr Val Ser Asn Lys Gly Val Asp
        435                 440                 445

Thr Val Ser Val Gly Asn Thr Leu Tyr Tyr Val Asn Lys Gln Glu Gly
    450                 455                 460

Lys Ser Leu Tyr Val Lys Gly Glu Pro Ile Ile Asn Phe Tyr Asp Pro
465                 470                 475                 480

Leu Val Phe Pro Ser Asp Glu Phe Asp Ala Ser Ile Ser Gln Val Asn
                485                 490                 495

Glu Lys Ile Asn Gln Ser Leu Ala Phe Ile Arg Lys Ser Asp Glu Leu
            500                 505                 510

Leu His Asn Val Asn Ala Gly Lys Ser Thr Ile Asn Ile Met Ile Thr
        515                 520                 525

Thr Ile Ile Ile Val Ile Ile Val Ile Leu Leu Ser Leu Ile Ala Val
    530                 535                 540

Gly Leu Leu Leu Tyr Cys Lys Ala Arg Ser Thr Pro Val Thr Leu Ser
545                 550                 555                 560

Lys Asp Gln Leu Ser Gly Ile Asn Asn Ile Ala Phe Ser Asn
                565                 570

<210>  3
<211>  897
<212>  DNA
<213>  respiratory syncytial virus

<400>  3
atgtccaaaa acaaggacca acgcaccgct aagacactag aaaagacctg ggacactctc      60

aatcatttat tattcatatc atcgggctta tataagttaa atcttaaatc tatagcacaa     120

atcacattat ccattctggc aatgataatc tcaacttcac ttataattac agccatcata     180

ttcatagcct cggcaaacca caaagtcaca ctaacaactg caatcataca agatgcaaca     240

agccagatca agaacacaac cccaacatac ctcactcagg atcctcagct tggaatcagc     300

ttctccaatc tgtctgaaat tacatcacaa accaccacca tactagcttc aacaacacca     360

ggagtcaagt caaacctgca acccacaaca gtcaagacta aaaacacaac aacaacccaa     420

acacaaccca gcaagcccac tacaaaacaa cgccaaaaca aaccaccaaa caaacccaat     480

aatgattttc acttcgaagt gtttaacttt gtaccctgca gcatatgcag caacaatcca     540

acctgctggg ctatctgcaa aagaatacca aacaaaaaac caggaaagaa aaccaccacc 600

aagcctacaa aaaaaccaac cttcaagaca accaaaaaag atctcaaacc tcaaaccact 660

aaaccaaagg aagtacccac caccaagccc acagaagagc caaccatcaa caccaccaaa 720

acaaacatca caactacact gctcaccaac aacaccacag gaaatccaaa actcacaagt 780

caaatggaaa ccttccactc aacctcctcc gaaggcaatc taagcccttc tcaagtctcc 840

acaacatccg agcacccatc acaaccctca tctccaccca acacaacacg ccagtag 897

<210> 4
<211> 298
<212> PRT
<213> respiratory syncytial virus

<400> 4

Met Ser Lys Asn Lys Asp Gln Arg Thr Ala Lys Thr Leu Glu Lys Thr
1               5                   10                  15

Trp Asp Thr Leu Asn His Leu Leu Phe Ile Ser Ser Gly Leu Tyr Lys
            20                  25                  30

Leu Asn Leu Lys Ser Ile Ala Gln Ile Thr Leu Ser Ile Leu Ala Met
        35                  40                  45

Ile Ile Ser Thr Ser Leu Ile Ile Thr Ala Ile Ile Phe Ile Ala Ser
    50                  55                  60

Ala Asn His Lys Val Thr Leu Thr Thr Ala Ile Ile Gln Asp Ala Thr
65                  70                  75                  80

Ser Gln Ile Lys Asn Thr Thr Pro Thr Tyr Leu Thr Gln Asp Pro Gln
                85                  90                  95

Leu Gly Ile Ser Phe Ser Asn Leu Ser Glu Ile Thr Ser Gln Thr Thr
            100                 105                 110

Thr Ile Leu Ala Ser Thr Thr Pro Gly Val Lys Ser Asn Leu Gln Pro
        115                 120                 125

Thr Thr Val Lys Thr Lys Asn Thr Thr Thr Thr Gln Thr Gln Pro Ser
    130                 135                 140

Lys Pro Thr Thr Lys Gln Arg Gln Asn Lys Pro Pro Asn Lys Pro Asn
145                 150                 155                 160

Asn Asp Phe His Phe Glu Val Phe Asn Phe Val Pro Cys Ser Ile Cys
                165                 170                 175

Ser Asn Asn Pro Thr Cys Trp Ala Ile Cys Lys Arg Ile Pro Asn Lys
            180                 185                 190

Lys Pro Gly Lys Lys Thr Thr Thr Lys Pro Thr Lys Lys Pro Thr Phe
        195                 200                 205

Lys Thr Thr Lys Lys Asp Leu Lys Pro Gln Thr Thr Lys Pro Lys Glu
    210                 215                 220

Val Pro Thr Thr Lys Pro Thr Glu Glu Pro Thr Ile Asn Thr Thr Lys
225                 230                 235                 240

Thr Asn Ile Thr Thr Thr Leu Leu Thr Asn Asn Thr Thr Gly Asn Pro
                245                 250                 255

Lys Leu Thr Ser Gln Met Glu Thr Phe His Ser Thr Ser Ser Glu Gly
            260                 265                 270

Asn Leu Ser Pro Ser Gln Val Ser Thr Thr Ser Glu His Pro Ser Gln
        275                 280                 285

Pro Ser Ser Pro Pro Asn Thr Thr Arg Gln
    290                 295

<210>  5
<211>  1566
<212>  DNA
<213>  Artificial Sequence

<220>
<223>  Recombinant PreF polynucleotide

<400>  5
aagcttgcca ccatggagct gctgatcctg aaaaccaacg ccatcaccgc catcctggcc      60

gccgtgaccc tgtgcttcgc ctcctcccag aacatcaccg aggagttcta ccagtccacc     120

tgctccgccg tgtccaaggg ctacctgtcc gccctgcgga ccggctggta cacctccgtg     180

atcaccatcg agctgtccaa catcaaggaa aacaagtgca acggcaccga cgccaaggtg     240

aagctgatca agcaggagct ggacaagtac aagagcgccg tgaccgaact ccagctgctg     300

atgcagtcca cccctgccac caacaacaag tttctgggct tcctgctggg cgtgggctcc     360

gccatcgcct ccggcatcgc cgtgagcaag gtgctgcacc tggagggcga ggtgaacaag     420

atcaagagcg ccctgctgtc caccaacaag gccgtggtgt ccctgtccaa cggcgtgtcc     480

gtgctgacct ccaaggtgct ggatctgaag aactacatcg acaagcagct gctgcctatc     540

gtgaacaagc agtcctgctc catctccaac atcgagaccg tgatcgagtt ccagcagaag     600

aacaaccggc tgctggagat cacccgcgag ttctccgtga acgccggcgt gaccacccct     660

gtgtccacct acatgctgac caactccgag ctgctgtccc tgatcaacga catgcctatc 720

accaacgacc agaaaaaact gatgtccaac aacgtgcaga tcgtgcggca gcagtcctac 780

agcatcatga gcatcatcaa ggaagaggtg ctggcctacg tggtgcagct gcctctgtac 840

ggcgtgatcg acaccccttg ctggaagctg cacacctccc ccctgtgcac caccaacacc 900

aaggagggct ccaacatctg cctgacccgg accgaccggg gctggtactg cgacaacgcc 960

ggctccgtgt ccttcttccc tctggccgag acctgcaagg tgcagtccaa ccgggtgttc 1020

tgcgacacca tgaactccct gaccctgcct tccgaggtga acctgtgcaa catcgacatc 1080

ttcaacccca agtacgactg caagatcatg accagcaaga ccgacgtgtc ctccagcgtg 1140

atcacctccc tgggcgccat cgtgtcctgc tacggcaaga ccaagtgcac cgcctccaac 1200

aagaaccggg gaatcatcaa gaccttctcc aacggctgcg actacgtgtc caataagggc 1260

gtggacaccg tgtccgtggg caacacactg tactacgtga ataagcagga gggcaagagc 1320

ctgtacgtga agggcgagcc tatcatcaac ttctacgacc ctctggtgtt cccttccgac 1380

gagttcgacg cctccatcag ccaggtgaac gagaagatca accagtccct ggccttcatc 1440

cggaagtccg acgagaagct gcataacgtg gaggacaaga tcgaggagat cctgtccaaa 1500

atctaccaca tcgagaacga gatcgcccgg atcaagaagc tgatcggcga ggcctgataa 1560

tctaga 1566

<210> 6
<211> 514
<212> PRT
<213> Artificial Sequence

<220>
<223> Recombinant PreF Antigen

<400> 6

Met Glu Leu Leu Ile Leu Lys Thr Asn Ala Ile Thr Ala Ile Leu Ala
1               5                   10                  15

Ala Val Thr Leu Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Ser Ala Val Thr Glu Leu Gln Leu Leu
                85                  90                  95

Met Gln Ser Thr Pro Ala Thr Asn Asn Lys Phe Leu Gly Phe Leu Leu
            100                 105                 110

Gly Val Gly Ser Ala Ile Ala Ser Gly Ile Ala Val Ser Lys Val Leu
        115                 120                 125

His Leu Glu Gly Glu Val Asn Lys Ile Lys Ser Ala Leu Leu Ser Thr
    130                 135                 140

Asn Lys Ala Val Val Ser Leu Ser Asn Gly Val Ser Val Leu Thr Ser
145                 150                 155                 160

Lys Val Leu Asp Leu Lys Asn Tyr Ile Asp Lys Gln Leu Leu Pro Ile
                165                 170                 175

Val Asn Lys Gln Ser Cys Ser Ile Ser Asn Ile Glu Thr Val Ile Glu
            180                 185                 190

Phe Gln Gln Lys Asn Asn Arg Leu Leu Glu Ile Thr Arg Glu Phe Ser
        195                 200                 205

Val Asn Ala Gly Val Thr Thr Pro Val Ser Thr Tyr Met Leu Thr Asn
    210                 215                 220

Ser Glu Leu Leu Ser Leu Ile Asn Asp Met Pro Ile Thr Asn Asp Gln
225                 230                 235                 240

Lys Lys Leu Met Ser Asn Asn Val Gln Ile Val Arg Gln Gln Ser Tyr
                245                 250                 255

Ser Ile Met Ser Ile Ile Lys Glu Glu Val Leu Ala Tyr Val Val Gln
            260                 265                 270

Leu Pro Leu Tyr Gly Val Ile Asp Thr Pro Cys Trp Lys Leu His Thr
        275                 280                 285

Ser Pro Leu Cys Thr Thr Asn Thr Lys Glu Gly Ser Asn Ile Cys Leu
    290                 295                 300

Thr Arg Thr Asp Arg Gly Trp Tyr Cys Asp Asn Ala Gly Ser Val Ser
305                 310                 315                 320

Phe Phe Pro Leu Ala Glu Thr Cys Lys Val Gln Ser Asn Arg Val Phe
                325                 330                 335

Cys Asp Thr Met Asn Ser Leu Thr Leu Pro Ser Glu Val Asn Leu Cys
            340                 345                 350

Asn Ile Asp Ile Phe Asn Pro Lys Tyr Asp Cys Lys Ile Met Thr Ser
        355                 360                 365

Lys Thr Asp Val Ser Ser Ser Val Ile Thr Ser Leu Gly Ala Ile Val
    370                 375                 380

Ser Cys Tyr Gly Lys Thr Lys Cys Thr Ala Ser Asn Lys Asn Arg Gly
385                 390                 395                 400

Ile Ile Lys Thr Phe Ser Asn Gly Cys Asp Tyr Val Ser Asn Lys Gly
                405                 410                 415

Val Asp Thr Val Ser Val Gly Asn Thr Leu Tyr Tyr Val Asn Lys Gln
            420                 425                 430

Glu Gly Lys Ser Leu Tyr Val Lys Gly Glu Pro Ile Ile Asn Phe Tyr
        435                 440                 445

Asp Pro Leu Val Phe Pro Ser Asp Glu Phe Asp Ala Ser Ile Ser Gln
    450                 455                 460

Val Asn Glu Lys Ile Asn Gln Ser Leu Ala Phe Ile Arg Lys Ser Asp
465                 470                 475                 480

Glu Lys Leu His Asn Val Glu Asp Lys Ile Glu Glu Ile Leu Ser Lys
                485                 490                 495

Ile Tyr His Ile Glu Asn Glu Ile Ala Arg Ile Lys Lys Leu Ile Gly
            500                 505                 510

Glu Ala

<210>  7
<211>  1855
<212>  DNA
<213>  Artificial Sequence

<220>
<223>  Chimeric PreF-G Antigen polynucleotide seqeunce

<400>  7
aagcttgcca ccatggagct gctgatcctc aagaccaacg ccatcaccgc catcctggcc      60

gccgtgaccc tgtgcttcgc ctcctcccag aacatcaccg aagagttcta ccagtccacc     120

tgctccgccg tgtccaaggg ctacctgtcc gccctgcgga ccggctggta cacctccgtg 180

atcaccatcg agctgtccaa catcaaagaa aacaagtgca acggcaccga cgccaaggtc 240

aagctgatca agcaggaact ggacaagtac aagagcgccg tgaccgaact ccagctgctg 300

atgcagtcca cccctgccac caacaacaag aagtttctgg gcttcctgct gggcgtgggc 360

tccgccatcg cctccggcat cgccgtgagc aaggtgctgc acctggaggg cgaggtgaac 420

aagatcaaga gcgccctgct gtccaccaac aaggccgtgg tgtccctgtc caacggcgtg 480

tccgtgctga cctccaaggt gctggatctg aagaactaca tcgacaagca gctgctgcct 540

atcgtgaaca agcagtcctg ctccatctcc aacatcgaga ccgtgatcga gttccagcag 600

aagaacaacc ggctgctgga gatcacccgc gagttctccg tgaacgccgg cgtgaccacc 660

cctgtgtcca cctacatgct gacaaactcc gagctgctct ccctgatcaa cgacatgcct 720

atcaccaacg accaaaaaaa gctgatgtcc aacaacgtgc agatcgtgcg gcagcagtcc 780

tacagcatca tgagcatcat caaggaagaa gtcctggcct acgtcgtgca gctgcctctg 840

tacggcgtga tcgacacccc ttgctggaag ctgcacacct cccccctgtg caccaccaac 900

accaaagagg gctccaacat ctgcctgacc cggaccgacc ggggctggta ctgcgacaac 960

gccggctccg tgtccttctt ccctctggcc gagacctgca aggtgcagtc caaccgggtg 1020

ttctgcgaca ccatgaactc cctgaccctg ccttccgagg tgaacctgtg caacatcgac 1080

atcttcaacc ccaagtacga ctgcaagatc atgaccagca agaccgacgt gtcctccagc 1140

gtgatcacct ccctgggcgc catcgtgtcc tgctacggca agaccaagtg caccgcctcc 1200

aacaagaacc ggggaatcat caagaccttc tccaacggct gcgactacgt gtccaataag 1260

ggcgtggaca ccgtgtccgt gggcaacaca ctgtactacg tgaataagca ggaaggcaag 1320

agcctgtacg tgaagggcga gcctatcatc aacttctacg accctctggt gttcccttcc 1380

gacgagttcg acgcctccat cagccaggtc aacgagaaga tcaaccagtc cctggccttc 1440

atccggaagt ccgacgagaa gctgcataac gtggaggaca agatcgaaga gatcctgtcc 1500

aaaatctacc acatcgagaa cgagatcgcc cggatcaaga agctgatcgg cgaggctggc 1560

ggctctggcg gcagcggcgg ctccaagcag cggcagaaca agcctcctaa caagcccaac 1620

aacgacttcc acttcgaggt gttcaacttc gtgccttgct ccatctgctc caacaaccct 1680

acctgctggg ccatctgcaa gagaatcccc aacaagaagc ctggcaagaa aaccaccacc 1740

aagcctacca agaagcctac cttcaagacc accaagaagg accacaagcc tcagaccaca 1800

aagcctaagg aagtgccaac caccaagcac caccaccatc accactgata atcta 1855

<210> 8
<211> 605
<212> PRT
<213> Artificial Sequence

<220>
<223>  Chimeric PreF-G polypeptide

<400>  8

Met Glu Leu Leu Ile Leu Lys Thr Asn Ala Ile Thr Ala Ile Leu Ala
1               5                   10                  15

Ala Val Thr Leu Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Ser Ala Val Thr Glu Leu Gln Leu Leu
                85                  90                  95

Met Gln Ser Thr Pro Ala Thr Asn Asn Lys Lys Phe Leu Gly Phe Leu
            100                 105                 110

Leu Gly Val Gly Ser Ala Ile Ala Ser Gly Ile Ala Val Ser Lys Val
        115                 120                 125

Leu His Leu Glu Gly Glu Val Asn Lys Ile Lys Ser Ala Leu Leu Ser
    130                 135                 140

Thr Asn Lys Ala Val Val Ser Leu Ser Asn Gly Val Ser Val Leu Thr
145                 150                 155                 160

Ser Lys Val Leu Asp Leu Lys Asn Tyr Ile Asp Lys Gln Leu Leu Pro
                165                 170                 175

Ile Val Asn Lys Gln Ser Cys Ser Ile Ser Asn Ile Glu Thr Val Ile
            180                 185                 190

Glu Phe Gln Gln Lys Asn Asn Arg Leu Leu Glu Ile Thr Arg Glu Phe
        195                 200                 205

Ser Val Asn Ala Gly Val Thr Thr Pro Val Ser Thr Tyr Met Leu Thr
    210                 215                 220

Asn Ser Glu Leu Leu Ser Leu Ile Asn Asp Met Pro Ile Thr Asn Asp

225                 230                 235                 240

Gln Lys Lys Leu Met Ser Asn Asn Val Gln Ile Val Arg Gln Gln Ser
                245                 250                 255

Tyr Ser Ile Met Ser Ile Ile Lys Glu Glu Val Leu Ala Tyr Val Val
            260                 265                 270

Gln Leu Pro Leu Tyr Gly Val Ile Asp Thr Pro Cys Trp Lys Leu His
        275                 280                 285

Thr Ser Pro Leu Cys Thr Thr Asn Thr Lys Glu Gly Ser Asn Ile Cys
    290                 295                 300

Leu Thr Arg Thr Asp Arg Gly Trp Tyr Cys Asp Asn Ala Gly Ser Val
305                 310                 315                 320

Ser Phe Phe Pro Leu Ala Glu Thr Cys Lys Val Gln Ser Asn Arg Val
                325                 330                 335

Phe Cys Asp Thr Met Asn Ser Leu Thr Leu Pro Ser Glu Val Asn Leu
            340                 345                 350

Cys Asn Ile Asp Ile Phe Asn Pro Lys Tyr Asp Cys Lys Ile Met Thr
        355                 360                 365

Ser Lys Thr Asp Val Ser Ser Ser Val Ile Thr Ser Leu Gly Ala Ile
    370                 375                 380

Val Ser Cys Tyr Gly Lys Thr Lys Cys Thr Ala Ser Asn Lys Asn Arg
385                 390                 395                 400

Gly Ile Ile Lys Thr Phe Ser Asn Gly Cys Asp Tyr Val Ser Asn Lys
                405                 410                 415

Gly Val Asp Thr Val Ser Val Gly Asn Thr Leu Tyr Tyr Val Asn Lys
            420                 425                 430

Gln Glu Gly Lys Ser Leu Tyr Val Lys Gly Glu Pro Ile Ile Asn Phe
        435                 440                 445

Tyr Asp Pro Leu Val Phe Pro Ser Asp Glu Phe Asp Ala Ser Ile Ser
    450                 455                 460

Gln Val Asn Glu Lys Ile Asn Gln Ser Leu Ala Phe Ile Arg Lys Ser
465                 470                 475                 480

Asp Glu Lys Leu His Asn Val Glu Asp Lys Ile Glu Glu Ile Leu Ser
                485                 490                 495

Lys Ile Tyr His Ile Glu Asn Glu Ile Ala Arg Ile Lys Lys Leu Ile
            500                 505                 510

Gly Glu Ala Gly Gly Ser Gly Gly Ser Gly Gly Ser Lys Gln Arg Gln
        515                 520                 525

Asn Lys Pro Pro Asn Lys Pro Asn Asn Asp Phe His Phe Glu Val Phe
    530                 535                 540

Asn Phe Val Pro Cys Ser Ile Cys Ser Asn Asn Pro Thr Cys Trp Ala
545                 550                 555                 560

Ile Cys Lys Arg Ile Pro Asn Lys Lys Pro Gly Lys Lys Thr Thr Thr
                565                 570                 575

Lys Pro Thr Lys Lys Pro Thr Phe Lys Thr Thr Lys Lys Asp His Lys
            580                 585                 590

Pro Gln Thr Thr Lys Pro Lys Glu Val Pro Thr Thr Lys
        595                 600                 605

<210>  9
<211>  1834
<212>  DNA
<213>  Artificial Sequence

<220>
<223>  Chimeric PreF-G polynucleotide

<400>  9
aagcttgcca ccatggagct gctgatcctc aagaccaacg ccatcaccgc catcctggcc      60

gccgtgaccc tgtgcttcgc ctcctcccag aacatcaccg aagagttcta ccagtccacc     120

tgctccgccg tgtccaaggg ctacctgtcc gccctgcgga ccggctggta cacctccgtg     180

atcaccatcg agctgtccaa catcaaagaa aacaagtgca acggcaccga cgccaaggtc     240

aagctgatca agcaggaact ggacaagtac aagagcgccg tgaccgaact ccagctgctg     300

atgcagtcca cccctgccac caacaacaag aagtttctgg gcttcctgct gggcgtgggc     360

tccgccatcg cctccggcat cgccgtgagc aaggtgctgc acctggaggg cgaggtgaac     420

aagatcaaga gcgccctgct gtccaccaac aaggccgtgg tgtccctgtc caacggcgtg     480

tccgtgctga cctccaaggt gctggatctg aagaactaca tcgacaagca gctgctgcct     540

atcgtgaaca agcagtcctg ctccatctcc aacatcgaga ccgtgatcga gttccagcag     600

aagaacaacc ggctgctgga gatcacccgc gagttctccg tgaacgccgg cgtgaccacc     660

cctgtgtcca cctacatgct gacaaactcc gagctgctct ccctgatcaa cgacatgcct 720

atcaccaacg accaaaaaaa gctgatgtcc aacaacgtgc agatcgtgcg gcagcagtcc 780

tacagcatca tgagcatcat caaggaagaa gtcctggcct acgtcgtgca gctgcctctg 840

tacggcgtga tcgacacccc ttgctggaag ctgcacacct cccccctgtg caccaccaac 900

accaaagagg gctccaacat ctgcctgacc cggaccgacc ggggctggta ctgcgacaac 960

gccggctccg tgtccttctt ccctctggcc gagacctgca aggtgcagtc caaccgggtg 1020

ttctgcgaca ccatgaactc cctgaccctg ccttccgagg tgaacctgtg caacatcgac 1080

atcttcaacc ccaagtacga ctgcaagatc atgaccagca agaccgacgt gtcctccagc 1140

gtgatcacct ccctgggcgc catcgtgtcc tgctacggca agaccaagtg caccgcctcc 1200

aacaagaacc ggggaatcat caagaccttc tccaacggct gcgactacgt gtccaataag 1260

ggcgtggaca ccgtgtccgt gggcaacaca ctgtactacg tgaataagca ggaaggcaag 1320

agcctgtacg tgaagggcga gcctatcatc aacttctacg accctctggt gttcccttcc 1380

gacgagttcg acgcctccat cagccaggtc aacgagaaga tcaaccagtc cctggccttc 1440

atccggaagt ccgacgagaa gctgcataac gtggaggaca agatcgaaga gatcctgtcc 1500

aaaatctacc acatcgagaa cgagatcgcc cggatcaaga agctgatcgg cgaggctggc 1560

ggcaagcagc ggcagaacaa gcctcctaac aagcccaaca acgacttcca cttcgaggtg 1620

ttcaacttcg tgccttgctc catctgctcc aacaacccta cctgctgggc catctgcaag 1680

agaatcccca acaagaagcc tggcaagaaa accaccacca agcctaccaa gaagcctacc 1740

ttcaagacca ccaagaagga ccacaagcct cagaccacaa agcctaagga agtgccaacc 1800

accaagcacc accaccatca ccactgataa tcta 1834

<210> 10
<211> 598
<212> PRT
<213> Artificial Sequence

<220>
<223> Chimeric PreF-G polypeptide

<400> 10

Met Glu Leu Leu Ile Leu Lys Thr Asn Ala Ile Thr Ala Ile Leu Ala
1               5                   10                  15

Ala Val Thr Leu Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Ser Ala Val Thr Glu Leu Gln Leu Leu
                85                  90                  95

Met Gln Ser Thr Pro Ala Thr Asn Asn Lys Lys Phe Leu Gly Phe Leu
            100                 105                 110

Leu Gly Val Gly Ser Ala Ile Ala Ser Gly Ile Ala Val Ser Lys Val
        115                 120                 125

Leu His Leu Glu Gly Glu Val Asn Lys Ile Lys Ser Ala Leu Leu Ser
    130                 135                 140

Thr Asn Lys Ala Val Val Ser Leu Ser Asn Gly Val Ser Val Leu Thr
145                 150                 155                 160

Ser Lys Val Leu Asp Leu Lys Asn Tyr Ile Asp Lys Gln Leu Leu Pro
                165                 170                 175

Ile Val Asn Lys Gln Ser Cys Ser Ile Ser Asn Ile Glu Thr Val Ile
            180                 185                 190

Glu Phe Gln Gln Lys Asn Asn Arg Leu Leu Glu Ile Thr Arg Glu Phe
        195                 200                 205

Ser Val Asn Ala Gly Val Thr Thr Pro Val Ser Thr Tyr Met Leu Thr
    210                 215                 220

Asn Ser Glu Leu Leu Ser Leu Ile Asn Asp Met Pro Ile Thr Asn Asp
225                 230                 235                 240

Gln Lys Lys Leu Met Ser Asn Asn Val Gln Ile Val Arg Gln Gln Ser
                245                 250                 255

Tyr Ser Ile Met Ser Ile Ile Lys Glu Glu Val Leu Ala Tyr Val Val
            260                 265                 270

Gln Leu Pro Leu Tyr Gly Val Ile Asp Thr Pro Cys Trp Lys Leu His
        275                 280                 285

Thr Ser Pro Leu Cys Thr Thr Asn Thr Lys Glu Gly Ser Asn Ile Cys
    290                 295                 300

Leu Thr Arg Thr Asp Arg Gly Trp Tyr Cys Asp Asn Ala Gly Ser Val
305                 310                 315                 320

Ser Phe Phe Pro Leu Ala Glu Thr Cys Lys Val Gln Ser Asn Arg Val
                325                 330                 335

Phe Cys Asp Thr Met Asn Ser Leu Thr Leu Pro Ser Glu Val Asn Leu
            340                 345                 350

Cys Asn Ile Asp Ile Phe Asn Pro Lys Tyr Asp Cys Lys Ile Met Thr
        355                 360                 365

Ser Lys Thr Asp Val Ser Ser Ser Val Ile Thr Ser Leu Gly Ala Ile
    370                 375                 380

Val Ser Cys Tyr Gly Lys Thr Lys Cys Thr Ala Ser Asn Lys Asn Arg
385                 390                 395                 400

Gly Ile Ile Lys Thr Phe Ser Asn Gly Cys Asp Tyr Val Ser Asn Lys
                405                 410                 415

Gly Val Asp Thr Val Ser Val Gly Asn Thr Leu Tyr Tyr Val Asn Lys
            420                 425                 430

Gln Glu Gly Lys Ser Leu Tyr Val Lys Gly Glu Pro Ile Ile Asn Phe
        435                 440                 445

Tyr Asp Pro Leu Val Phe Pro Ser Asp Glu Phe Asp Ala Ser Ile Ser
    450                 455                 460

Gln Val Asn Glu Lys Ile Asn Gln Ser Leu Ala Phe Ile Arg Lys Ser
465                 470                 475                 480

Asp Glu Lys Leu His Asn Val Glu Asp Lys Ile Glu Glu Ile Leu Ser
                485                 490                 495

Lys Ile Tyr His Ile Glu Asn Glu Ile Ala Arg Ile Lys Lys Leu Ile
            500                 505                 510

Gly Glu Ala Gly Gly Lys Gln Arg Gln Asn Lys Pro Pro Asn Lys Pro
        515                 520                 525

Asn Asn Asp Phe His Phe Glu Val Phe Asn Phe Val Pro Cys Ser Ile
    530                 535                 540

Cys Ser Asn Asn Pro Thr Cys Trp Ala Ile Cys Lys Arg Ile Pro Asn
545                 550                 555                 560

Lys Lys Pro Gly Lys Lys Thr Thr Thr Lys Pro Thr Lys Lys Pro Thr
                565                 570                 575

Phe Lys Thr Thr Lys Lys Asp His Lys Pro Gln Thr Thr Lys Pro Lys
            580                 585                 590

Glu Val Pro Thr Thr Lys
        595

<210>  11
<211>  28
<212>  PRT
<213>  Artificial Sequence

<220>
<223>  Isoleucine substituted GCN4 leucine zipper

<400>  11

Glu Asp Lys Ile Glu Glu Ile Leu Ser Lys Ile Tyr His Ile Glu Asn
1               5                   10                  15

Glu Ile Ala Arg Ile Lys Lys Leu Ile Gly Glu Ala
            20                  25

<210>  12
<211>  1563
<212>  DNA
<213>  Artificial sequence

<220>
<223>  Codon optimized PreF nucleotide sequence

<400>  12
atggagctgc ccatcctgaa gaccaacgcc atcaccacca tcctcgccgc cgtgaccctg      60

tgcttcgcca gcagccagaa catcacggag gagttctacc agagcacgtg cagcgccgtg     120

agcaagggct acctgagcgc gctgcgcacg ggctggtaca cgagcgtgat cacgatcgag     180

ctgagcaaca tcaaggagaa caagtgcaac ggcacggacg cgaaggtgaa gctgatcaag     240

caggagctgg acaagtacaa gagcgcggtg acggagctgc agctgctgat gcagagcacg     300

ccggcgacga acaacaagtt cctcggcttc ctgctgggcg tgggcagcgc gatcgcgagc     360

ggcatcgccg tgagcaaggt gctgcacctg gagggcgagg tgaacaagat caagtccgcg     420

ctgctgagca cgaacaaggc ggtcgtgagc ctgagcaacg gcgtgagcgt gctgacgagc     480

aaggtgctcg acctgaagaa ctacatcgac aagcagctgc tgccgatcgt gaacaagcag     540

agctgcagca tcagcaacat cgagaccgtg atcgagttcc agcagaagaa caaccgcctg     600

ctggagatca cgcgggagtt ctccgtgaac gcaggcgtga cgacgcccgt gtctacgtac     660

atgctgacga acagcgagct gctcagcctg atcaacgaca tgccgatcac gaacgaccag 720

aagaagctga tgagcaacaa cgtgcagatc gtgcgccagc agagctacag catcatgagc 780

atcatcaagg aggaggtgct ggcatacgtg gtgcagctgc cgctgtacgg cgtcatcgac 840

acgccctgct ggaagctgca cacgagcccg ctgtgcacga ccaacacgaa ggagggcagc 900

aacatctgcc tgacgcggac ggaccggggc tggtactgcg acaacgcggg cagcgtgagc 960

ttcttcccgc tcgcggagac gtgcaaggtg cagagcaacc gcgtcttctg cgacacgatg 1020

aacagcctga cgctgccgag cgaggtgaac ctgtgcaaca tcgacatctt caacccgaag 1080

tacgactgca agatcatgac gagcaagacc gatgtcagca gcagcgtgat cacgagcctc 1140

ggcgcgatcg tgagctgcta cggcaagacg aagtgcacgg cgagcaacaa gaaccgcggc 1200

atcatcaaga cgttcagcaa cggctgcgac tatgtgagca acaagggcgt ggacactgtg 1260

agcgtcggca acacgctgta ctacgtgaac aagcaggagg gcaagagcct gtacgtgaag 1320

ggcgagccga tcatcaactt ctacgacccg ctcgtgttcc cgagcgacga gttcgacgcg 1380

agcatcagcc aagtgaacga gaagatcaac cagagcctgg cgttcatccg caagagcgac 1440

gagaagctgc acaacgtgga ggacaagatc gaggagatcc tgagcaagat ctaccacatc 1500

gagaacgaga tcgcgcgcat caagaagctg atcggcgagg cgcatcatca ccatcaccat 1560

tga 1563

<210> 13
<211> 1685
<212> DNA
<213> Artificial sequence

<220>
<223> PreF polynucleotide sequence with intron

<400> 13
   atggagctgc tgatcctgaa aaccaacgcc atcaccgcca tcctggccgc cgtgaccctg 60
   tgcttcgcct cctcccagaa catcaccgag gagttctacc agtccacctg ctccgccgtg 120
   tccaagggct acctgtccgc cctgcggacc ggctggtaca cctccgtgat caccatcgag 180
   ctgtccaaca tcaaggaaaa caagtgcaac ggcaccgacg ccaaggtgaa gctgatcaag 240
   caggagctgg acaagtacaa gagcgccgtg accgaactcc agctgctgat gcagtccacc 300
   cctgccacca acaacaagtt tctgggcttc ctgctgggcg tgggctccgc catcgcctcc 360
   ggcatcgccg tgagcaaggt acgtgtcggg acttgtgttc cccttttttt aataaaaagt 420
   tatatcttta atgttatata catatttcct gtatgtgatc catgtgctta tgactttgtt 480
   tatcatgtgt ttaggtgctg cacctggagg gcgaggtgaa caagatcaag agcgccctgc 540
   tgtccaccaa caaggccgtg gtgtccctgt ccaacggcgt gtccgtgctg acctccaagg 600
   tgctggatct gaagaactac atcgacaagc agctgctgcc tatcgtgaac aagcagtcct 660
   gctccatctc caacatcgag accgtgatcg agttccagca gaagaacaac cggctgctgg 720
   agatcacccg cgagttctcc gtgaacgccg gcgtgaccac ccctgtgtcc acctacatgc 780
   tgaccaactc cgagctgctg tccctgatca acgacatgcc tatcaccaac gaccagaaaa 840
   aactgatgtc caacaacgtg cagatcgtgc ggcagcagtc ctacagcatc atgagcatca 900
   tcaaggaaga ggtgctggcc tacgtggtgc agctgcctct gtacggcgtg atcgacaccc 960
   cttgctggaa gctgcacacc tcccccctgt gcaccaccaa caccaaggag ggctccaaca 1020
   tctgcctgac ccggaccgac cggggctggt actgcgacaa cgccggctcc gtgtccttct 1080
   tccctctggc cgagacctgc aaggtgcagt ccaaccgggt gttctgcgac accatgaact 1140
   ccctgaccct gccttccgag gtgaacctgt gcaacatcga catcttcaac cccaagtacg 1200
   actgcaagat catgaccagc aagaccgacg tgtcctccag cgtgatcacc tccctgggcg 1260
   ccatcgtgtc ctgctacggc aagaccaagt gcaccgcctc caacaagaac cggggaatca 1320
   tcaagacctt ctccaacggc tgcgactacg tgtccaataa gggcgtggac accgtgtccg 1380
   tgggcaacac actgtactac gtgaataagc aggagggcaa gagcctgtac gtgaagggcg 1440
   agcctatcat caacttctac gaccctctgg tgttcccttc cgacgagttc gacgcctcca 1500
   tcagccaggt gaacgagaag atcaaccagt ccctggcctt catccggaag tccgacgaga 1560
   agctgcataa cgtggaggac aagatcgagg agatcctgtc caaaatctac cacatcgaga 1620
   acgagatcgc ccggatcaag aagctgatcg gcgaggccgg aggtcaccac caccatcacc 1680
   actga 1685

<210> 14
<211> 9
<212> PRT
<213> Artificial sequence

<220>
<223> Synthetic linker peptide

<400> 14

Gly Gly Ser Gly Gly Ser Gly Gly Ser
1               5

<210> 15
<211> 4
<212> PRT
<213> Artificial sequence

<220>
<223> Furin cleavage consensus motif

<400> 15

Arg Ala Arg Arg

1


<210>  16
<211>  4
<212>  PRT
<213>  Artificial sequence

<220>
<223>  Furin cleavage consensus motif

<400>  16

Arg Lys Arg Arg
1


<210>  17
<211>  1542
<212>  DNA
<213>  Artificial Sequence

<220>
<223>  Synthetic PreF Polynucleotide

<400>  17
atggagctgc tgatcctgaa aaccaacgcc atcaccgcca tcctggccgc cgtgaccctg      60

tgcttcgcct cctcccagaa catcaccgag gagttctacc agtccacctg ctccgccgtg     120

tccaagggct acctgtccgc cctgcggacc ggctggtaca cctccgtgat caccatcgag     180

ctgtccaaca tcaaggaaaa caagtgcaac ggcaccgacg ccaaggtgaa gctgatcaag     240

caggagctgg acaagtacaa gagcgccgtg accgaactcc agctgctgat gcagtccacc     300

cctgccacca acaacaagtt tctgggcttc ctgctgggcg tgggctccgc catcgcctcc     360

ggcatcgccg tgagcaaggt gctgcacctg gagggcgagg tgaacaagat caagagcgcc     420

ctgctgtcca ccaacaaggc cgtggtgtcc ctgtccaacg gcgtgtccgt gctgacctcc     480

aaggtgctgg atctgaagaa ctacatcgac aagcagctgc tgcctatcgt gaacaagcag     540

tcctgctcca tctccaacat cgagaccgtg atcgagttcc agcagaagaa caaccggctg     600

ctggagatca cccgcgagtt ctccgtgaac gccggcgtga ccacccctgt gtccacctac     660

atgctgacca actccgagct gctgtccctg atcaacgaca tgcctatcac caacgaccag     720

aaaaaactga tgtccaacaa cgtgcagatc gtgcggcagc agtcctacag catcatgagc     780

atcatcaagg aagaggtgct ggcctacgtg gtgcagctgc ctctgtacgg cgtgatcgac     840

accccttgct ggaagctgca cacctccccc ctgtgcacca ccaacaccaa ggagggctcc     900

aacatctgcc tgacccggac cgaccggggc tggtactgcg acaacgccgg ctccgtgtcc     960

ttcttccctc tggccgagac ctgcaaggtg cagtccaacc gggtgttctg cgacaccatg    1020

aactccctga ccctgccttc cgaggtgaac ctgtgcaaca tcgacatctt caaccccaag    1080

tacgactgca agatcatgac cagcaagacc gacgtgtcct ccagcgtgat cacctccctg    1140

ggcgccatcg tgtcctgcta cggcaagacc aagtgcaccg cctccaacaa gaaccgggga     1200

atcatcaaga ccttctccaa cggctgcgac tacgtgtcca ataagggcgt ggacaccgtg     1260

tccgtgggca acacactgta ctacgtgaat aagcaggagg gcaagagcct gtacgtgaag     1320

ggcgagccta tcatcaactt ctacgaccct ctggtgttcc cttccgacga gttcgacgcc     1380

tccatcagcc aggtgaacga gaagatcaac gggaccctgg ccttcatccg gaagtccgac     1440

gagaagctgc ataacgtgga ggacaagatc gaggagatcc tgtccaaaat ctaccacatc     1500

gagaacgaga tcgcccggat caagaagctg atcggcgagg cc                        1542

<210>  18
<211>  514
<212>  PRT
<213>  Artificial Sequence

<220>
<223>  Synthetic PreF Polypeptide

<400>  18

Met Glu Leu Leu Ile Leu Lys Thr Asn Ala Ile Thr Ala Ile Leu Ala
1               5                   10                  15

Ala Val Thr Leu Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Ser Ala Val Thr Glu Leu Gln Leu Leu
                85                  90                  95

Met Gln Ser Thr Pro Ala Thr Asn Asn Lys Phe Leu Gly Phe Leu Leu
            100                 105                 110

Gly Val Gly Ser Ala Ile Ala Ser Gly Ile Ala Val Ser Lys Val Leu
        115                 120                 125

His Leu Glu Gly Glu Val Asn Lys Ile Lys Ser Ala Leu Leu Ser Thr
    130                 135                 140

Asn Lys Ala Val Val Ser Leu Ser Asn Gly Val Ser Val Leu Thr Ser

145                 150                 155                 160

Lys Val Leu Asp Leu Lys Asn Tyr Ile Asp Lys Gln Leu Leu Pro Ile
                165                 170                 175

Val Asn Lys Gln Ser Cys Ser Ile Ser Asn Ile Glu Thr Val Ile Glu
            180                 185                 190

Phe Gln Gln Lys Asn Asn Arg Leu Leu Glu Ile Thr Arg Glu Phe Ser
        195                 200                 205

Val Asn Ala Gly Val Thr Thr Pro Val Ser Thr Tyr Met Leu Thr Asn
    210                 215                 220

Ser Glu Leu Leu Ser Leu Ile Asn Asp Met Pro Ile Thr Asn Asp Gln
225                 230                 235                 240

Lys Lys Leu Met Ser Asn Asn Val Gln Ile Val Arg Gln Gln Ser Tyr
                245                 250                 255

Ser Ile Met Ser Ile Ile Lys Glu Glu Val Leu Ala Tyr Val Val Gln
            260                 265                 270

Leu Pro Leu Tyr Gly Val Ile Asp Thr Pro Cys Trp Lys Leu His Thr
        275                 280                 285

Ser Pro Leu Cys Thr Thr Asn Thr Lys Glu Gly Ser Asn Ile Cys Leu
    290                 295                 300

Thr Arg Thr Asp Arg Gly Trp Tyr Cys Asp Asn Ala Gly Ser Val Ser
305                 310                 315                 320

Phe Phe Pro Leu Ala Glu Thr Cys Lys Val Gln Ser Asn Arg Val Phe
                325                 330                 335

Cys Asp Thr Met Asn Ser Leu Thr Leu Pro Ser Glu Val Asn Leu Cys
            340                 345                 350

Asn Ile Asp Ile Phe Asn Pro Lys Tyr Asp Cys Lys Ile Met Thr Ser
        355                 360                 365

Lys Thr Asp Val Ser Ser Ser Val Ile Thr Ser Leu Gly Ala Ile Val
    370                 375                 380

Ser Cys Tyr Gly Lys Thr Lys Cys Thr Ala Ser Asn Lys Asn Arg Gly
385                 390                 395                 400

Ile Ile Lys Thr Phe Ser Asn Gly Cys Asp Tyr Val Ser Asn Lys Gly
                405                 410                 415

Val Asp Thr Val Ser Val Gly Asn Thr Leu Tyr Tyr Val Asn Lys Gln
            420                 425                 430

Glu Gly Lys Ser Leu Tyr Val Lys Gly Glu Pro Ile Ile Asn Phe Tyr
        435                 440                 445

Asp Pro Leu Val Phe Pro Ser Asp Glu Phe Asp Ala Ser Ile Ser Gln
    450                 455                 460

Val Asn Glu Lys Ile Asn Gly Thr Leu Ala Phe Ile Arg Lys Ser Asp
465                 470                 475                 480

Glu Lys Leu His Asn Val Glu Asp Lys Ile Glu Glu Ile Leu Ser Lys
                485                 490                 495

Ile Tyr His Ile Glu Asn Glu Ile Ala Arg Ile Lys Lys Leu Ile Gly
            500                 505                 510

Glu Ala

<210>  19
<211>  1542
<212>  DNA
<213>  Artificial Sequence

<220>
<223>  Synthetic PreF Polynucleotide

<400>  19
atggagctgc tgatcctgaa aaccaacgcc atcaccgcca tcctggccgc cgtgaccctg      60

tgcttcgcct cctcccagaa catcaccgag gagttctacc agtccacctg ctccgccgtg     120

tccaagggct acctgtccgc cctgcggacc ggctggtaca cctccgtgat caccatcgag     180

ctgtccaaca tcaaggaaaa caagtgcaac ggcaccgacg ccaaggtgaa gctgatcaag     240

caggagctgg acaagtacaa gagcgccgtg accgaactcc agctgctgat gcagtccacc     300

cctgccacca acaacaagtt tctgggcttc ctgcagggcg tgggctccgc catcgcctcc     360

ggcatcgccg tgagcaaggt gctgcacctg gagggcgagg tgaacaagat caagagcgcc     420

ctgctgtcca ccaacaaggc cgtggtgtcc ctgtccaacg gcgtgtccgt gctgacctcc     480

aaggtgctgg atctgaagaa ctacatcgac aagcagctgc tgcctatcgt gaacaagcag     540

tcctgctcca tctccaacat cgagaccgtg atcgagttcc agcagaagaa caaccggctg     600

ctggagatca cccgcgagtt ctccgtgaac gccggcgtga ccacccctgt gtccacctac     660

atgctgacca actccgagct gctgtccctg atcaacgaca tgcctatcac caacgaccag 720

aaaaaactga tgtccaacaa cgtgcagatc gtgcggcagc agtcctacag catcatgagc 780

atcatcaagg aagaggtgct ggcctacgtg gtgcagctgc ctctgtacgg cgtgatcgac 840

accccttgct ggaagctgca cacctccccc ctgtgcacca ccaacaccaa ggagggctcc 900

aacatctgcc tgacccggac cgaccggggc tggtactgcg acaacgccgg ctccgtgtcc 960

ttcttccctc tggccgagac ctgcaaggtg cagtccaacc gggtgttctg cgacaccatg 1020

aactccctga ccctgccttc cgaggtgaac ctgtgcaaca tcgacatctt caaccccaag 1080

tacgactgca agatcatgac cagcaagacc gacgtgtcct ccagcgtgat cacctccctg 1140

ggcgccatcg tgtcctgcta cggcaagacc aagtgcaccg cctccaacaa gaaccgggga 1200

atcatcaaga ccttctccaa cggctgcgac tacgtgtcca ataagggcgt ggacaccgtg 1260

tccgtgggca acacactgta ctacgtgaat aagcaggagg gcaagagcct gtacgtgaag 1320

ggcgagccta tcatcaactt ctacgaccct ctggtgttcc cttccgacga gttcgacgcc 1380

tccatcagcc aggtgaacga gaagatcaac cagtccctgg ccttcatccg gaagtccgac 1440

gagaagctgc ataacgtgga ggacaagatc gaggagatcc tgtccaaaat ctaccacatc 1500

gagaacgaga tcgcccggat caagaagctg atcggcgagg cc 1542

<210> 20
<211> 514
<212> PRT
<213> Artificial Sequence

<220>
<223> Synthetic PreF Polypeptide

<400> 20

Met Glu Leu Leu Ile Leu Lys Thr Asn Ala Ile Thr Ala Ile Leu Ala
1               5                   10                  15

Ala Val Thr Leu Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Ser Ala Val Thr Glu Leu Gln Leu Leu
                85                  90                  95

Met Gln Ser Thr Pro Ala Thr Asn Asn Lys Phe Leu Gly Phe Leu Gln
        100                 105                 110

Gly Val Gly Ser Ala Ile Ala Ser Gly Ile Ala Val Ser Lys Val Leu
    115                 120                 125

His Leu Glu Gly Glu Val Asn Lys Ile Lys Ser Ala Leu Leu Ser Thr
    130                 135                 140

Asn Lys Ala Val Val Ser Leu Ser Asn Gly Val Ser Val Leu Thr Ser
145                 150                 155                 160

Lys Val Leu Asp Leu Lys Asn Tyr Ile Asp Lys Gln Leu Leu Pro Ile
                165                 170                 175

Val Asn Lys Gln Ser Cys Ser Ile Ser Asn Ile Glu Thr Val Ile Glu
            180                 185                 190

Phe Gln Gln Lys Asn Asn Arg Leu Leu Glu Ile Thr Arg Glu Phe Ser
        195                 200                 205

Val Asn Ala Gly Val Thr Thr Pro Val Ser Thr Tyr Met Leu Thr Asn
    210                 215                 220

Ser Glu Leu Leu Ser Leu Ile Asn Asp Met Pro Ile Thr Asn Asp Gln
225                 230                 235                 240

Lys Lys Leu Met Ser Asn Asn Val Gln Ile Val Arg Gln Gln Ser Tyr
                245                 250                 255

Ser Ile Met Ser Ile Ile Lys Glu Glu Val Leu Ala Tyr Val Val Gln
            260                 265                 270

Leu Pro Leu Tyr Gly Val Ile Asp Thr Pro Cys Trp Lys Leu His Thr
        275                 280                 285

Ser Pro Leu Cys Thr Thr Asn Thr Lys Glu Gly Ser Asn Ile Cys Leu
    290                 295                 300

Thr Arg Thr Asp Arg Gly Trp Tyr Cys Asp Asn Ala Gly Ser Val Ser
305                 310                 315                 320

Phe Phe Pro Leu Ala Glu Thr Cys Lys Val Gln Ser Asn Arg Val Phe
                325                 330                 335

Cys Asp Thr Met Asn Ser Leu Thr Leu Pro Ser Glu Val Asn Leu Cys

340                 345                 350

Asn Ile Asp Ile Phe Asn Pro Lys Tyr Asp Cys Lys Ile Met Thr Ser
        355                 360                 365

Lys Thr Asp Val Ser Ser Ser Val Ile Thr Ser Leu Gly Ala Ile Val
    370                 375                 380

Ser Cys Tyr Gly Lys Thr Lys Cys Thr Ala Ser Asn Lys Asn Arg Gly
385                 390                 395                 400

Ile Ile Lys Thr Phe Ser Asn Gly Cys Asp Tyr Val Ser Asn Lys Gly
                405                 410                 415

Val Asp Thr Val Ser Val Gly Asn Thr Leu Tyr Tyr Val Asn Lys Gln
            420                 425                 430

Glu Gly Lys Ser Leu Tyr Val Lys Gly Glu Pro Ile Ile Asn Phe Tyr
        435                 440                 445

Asp Pro Leu Val Phe Pro Ser Asp Glu Phe Asp Ala Ser Ile Ser Gln
    450                 455                 460

Val Asn Glu Lys Ile Asn Gln Ser Leu Ala Phe Ile Arg Lys Ser Asp
465                 470                 475                 480

Glu Lys Leu His Asn Val Glu Asp Lys Ile Glu Glu Ile Leu Ser Lys
                485                 490                 495

Ile Tyr His Ile Glu Asn Glu Ile Ala Arg Ile Lys Lys Leu Ile Gly
            500                 505                 510

Glu Ala

<210>  21
<211>  1542
<212>  DNA
<213>  Artificial Sequence

<220>
<223>  Synthetic PreF Polynucleotide

<400>  21
atggagctgc tgatcctgaa aaccaacgcc atcaccgcca tcctggccgc cgtgaccctg      60

tgcttcgcct cctcccagaa catcaccgag gagttctacc agtccacctg ctccgccgtg     120

tccaagggct acctgtccgc cctgcggacc ggctggtaca cctccgtgat caccatcgag     180

ctgtccaaca tcaaggaaaa caagtgcaac ggcaccgacg ccaaggtgaa gctgatcaag     240

caggagctgg acaagtacaa gagcgccgtg accgaactcc agctgctgat gcagtccacc 300

cctgccacca acaacaagtt tctgggcttc ctgcagggcg tgggctccgc catcgcctcc 360

ggcatcgccg tgagcaaggt gctgcacctg gagggcgagg tgaacaagat caagagcgcc 420

ctgctgtcca ccaacaaggc cgtggtgtcc ctgtccaacg gcgtgtccgt gctgacctcc 480

aaggtgctgg atctgaagaa ctacatcgac aagcagctgc tgcctatcgt gaacaagcag 540

tcctgctcca tctccaacat cgagaccgtg atcgagttcc agcagaagaa caaccggctg 600

ctggagatca cccgcgagtt ctccgtgaac gccggcgtga ccacccctgt gtccacctac 660

atgctgacca actccgagct gctgtccctg atcaacgaca tgcctatcac caacgaccag 720

aaaaaactga tgtccaacaa cgtgcagatc gtgcggcagc agtcctacag catcatgagc 780

atcatcaagg aagaggtgct ggcctacgtg gtgcagctgc ctctgtacgg cgtgatcgac 840

accccttgct ggaagctgca cacctccccc ctgtgcacca ccaacaccaa ggagggctcc 900

aacatctgcc tgacccggac cgaccggggc tggtactgcg acaacgccgg ctccgtgtcc 960

ttcttccctc tggccgagac ctgcaaggtg cagtccaacc gggtgttctg cgacaccatg 1020

aactccctga ccctgccttc cgaggtgaac ctgtgcaaca tcgacatctt caaccccaag 1080

tacgactgca agatcatgac cagcaagacc gacgtgtcct ccagcgtgat cacctccctg 1140

ggcgccatcg tgtcctgcta cggcaagacc aagtgcaccg cctccaacaa gaaccgggga 1200

atcatcaaga ccttctccaa cggctgcgac tacgtgtcca ataagggcgt ggacaccgtg 1260

tccgtgggca acacactgta ctacgtgaat aagcaggagg gcaagagcct gtacgtgaag 1320

ggcgagccta tcatcaactt ctacgaccct ctggtgttcc cttccgacga gttcgacgcc 1380

tccatcagcc aggtgaacga gaagatcaac gggaccctgg ccttcatccg gaagtccgac 1440

gagaagctgc ataacgtgga ggacaagatc gaggagatcc tgtccaaaat ctaccacatc 1500

gagaacgaga tcgcccggat caagaagctg atcggcgagg cc 1542

<210> 22
<211> 514
<212> PRT
<213> Artificial Sequence

<220>
<223> Synthetic PreF Polypeptide

<400> 22

Met Glu Leu Leu Ile Leu Lys Thr Asn Ala Ile Thr Ala Ile Leu Ala
1               5                   10                  15

Ala Val Thr Leu Cys Phe Ala Ser Ser Gln Asn Ile Thr Glu Glu Phe
            20                  25                  30

Tyr Gln Ser Thr Cys Ser Ala Val Ser Lys Gly Tyr Leu Ser Ala Leu
        35                  40                  45

Arg Thr Gly Trp Tyr Thr Ser Val Ile Thr Ile Glu Leu Ser Asn Ile
    50                  55                  60

Lys Glu Asn Lys Cys Asn Gly Thr Asp Ala Lys Val Lys Leu Ile Lys
65                  70                  75                  80

Gln Glu Leu Asp Lys Tyr Lys Ser Ala Val Thr Glu Leu Gln Leu Leu
                85                  90                  95

Met Gln Ser Thr Pro Ala Thr Asn Asn Lys Phe Leu Gly Phe Leu Gln
            100                 105                 110

Gly Val Gly Ser Ala Ile Ala Ser Gly Ile Ala Val Ser Lys Val Leu
        115                 120                 125

His Leu Glu Gly Glu Val Asn Lys Ile Lys Ser Ala Leu Leu Ser Thr
    130                 135                 140

Asn Lys Ala Val Val Ser Leu Ser Asn Gly Val Ser Val Leu Thr Ser
145                 150                 155                 160

Lys Val Leu Asp Leu Lys Asn Tyr Ile Asp Lys Gln Leu Leu Pro Ile
                165                 170                 175

Val Asn Lys Gln Ser Cys Ser Ile Ser Asn Ile Glu Thr Val Ile Glu
            180                 185                 190

Phe Gln Gln Lys Asn Asn Arg Leu Leu Glu Ile Thr Arg Glu Phe Ser
        195                 200                 205

Val Asn Ala Gly Val Thr Thr Pro Val Ser Thr Tyr Met Leu Thr Asn
    210                 215                 220

Ser Glu Leu Leu Ser Leu Ile Asn Asp Met Pro Ile Thr Asn Asp Gln
225                 230                 235                 240

Lys Lys Leu Met Ser Asn Asn Val Gln Ile Val Arg Gln Gln Ser Tyr
                245                 250                 255

Ser Ile Met Ser Ile Ile Lys Glu Glu Val Leu Ala Tyr Val Val Gln
            260                 265                 270

Leu Pro Leu Tyr Gly Val Ile Asp Thr Pro Cys Trp Lys Leu His Thr
        275                 280                 285

Ser Pro Leu Cys Thr Thr Asn Thr Lys Glu Gly Ser Asn Ile Cys Leu
    290                 295                 300

Thr Arg Thr Asp Arg Gly Trp Tyr Cys Asp Asn Ala Gly Ser Val Ser
305                 310                 315                 320

Phe Phe Pro Leu Ala Glu Thr Cys Lys Val Gln Ser Asn Arg Val Phe
                325                 330                 335

Cys Asp Thr Met Asn Ser Leu Thr Leu Pro Ser Glu Val Asn Leu Cys
            340                 345                 350

Asn Ile Asp Ile Phe Asn Pro Lys Tyr Asp Cys Lys Ile Met Thr Ser
        355                 360                 365

Lys Thr Asp Val Ser Ser Ser Val Ile Thr Ser Leu Gly Ala Ile Val
    370                 375                 380

Ser Cys Tyr Gly Lys Thr Lys Cys Thr Ala Ser Asn Lys Asn Arg Gly
385                 390                 395                 400

Ile Ile Lys Thr Phe Ser Asn Gly Cys Asp Tyr Val Ser Asn Lys Gly
                405                 410                 415

Val Asp Thr Val Ser Val Gly Asn Thr Leu Tyr Tyr Val Asn Lys Gln
            420                 425                 430

Glu Gly Lys Ser Leu Tyr Val Lys Gly Glu Pro Ile Ile Asn Phe Tyr
        435                 440                 445

Asp Pro Leu Val Phe Pro Ser Asp Glu Phe Asp Ala Ser Ile Ser Gln
    450                 455                 460

Val Asn Glu Lys Ile Asn Gly Thr Leu Ala Phe Ile Arg Lys Ser Asp
465                 470                 475                 480

Glu Lys Leu His Asn Val Glu Asp Lys Ile Glu Glu Ile Leu Ser Lys
                485                 490                 495

Ile Tyr His Ile Glu Asn Glu Ile Ala Arg Ile Lys Lys Leu Ile Gly
            500                 505                 510

Glu Ala